# EUROPEAN PATENT APPLICATION

(11) **EP 1 167 375 A1**
(43) Date of publication of application: **02.01.2002**
(21) Application number: 00915372.7
(22) Date of filing: 06.04.2000
(51) Int. Cl.: C07H 17/08, A61K 31/7048, A61P 31/04

(54) **ERYTHROMYCIN DERIVATIVES**

(30) Priority: 08.04.1999 JP 10072699
(71) Applicant: HOKURIKU SEIYAKU CO., LTD., Katsuyama-shi, Fukui 911-0813 (JP)
(72) Inventor: KATO, Hideo, Katsuyama-shi, Fukui 911-0813 (JP); KADO, Noriyuki, Katsuyama-shi, Fukui 911-0813 (JP); YOSHIDA, Toshihiko, Katsuyama-shi, Fukui 911-0813 (JP); NISHIMOTO, Akemi, Katsuyama-shi, Fukui 911-0813 (JP); NARITA, Ken, Katsuyama-shi, Fukui 911-0813 (JP)
(74) Representative: Sternagel, Fleischer, Godemeyer & Partner Patentanwälte
(86) International application number: JP0002226
(87) International publication number: WO0061593

(57) **Abstract**

A novel erythromycin derivative represented by the following general formula or a salt thereof: 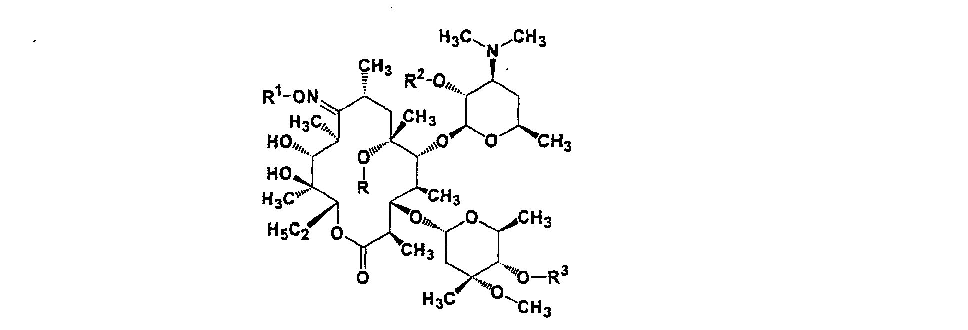 wherein R represents a hydrogen atom or a lower alkyl group; R¹ represents an alkyl group, a cycloalkyl group, a (cycloalkyl)alkyl group, an aralkyl group, or a group represented by the formula -(CH₂)ₙ-X-R⁴; R² represents a hydrogen atom or an acetyl group; R³ represents an acyl group or a group represented by the formula -C(=O)-Y-R⁵; R⁴ represents an alkyl group, an alkoxyalkyl group, an alkylthioalkyl group, an alkylaminoalkyl group, an aryl group, or an aralkyl group; R⁵ represents an alkyl group, an aryl group, or an aralkyl group; n represents an integer of from 1 to 6; X represents an oxygen atom, a sulfur atom, or a group represented by -NZ-; Y represents an oxygen atom or a group represented by -NH-; and Z represents a hydrogen atom or an alkyl group. The derivative has excellent antibacterial activity against atypical acid-fast mycobacteria including multiple drug-resistant bacteria and is extremely useful as an antibacterial agent.

## Description

### Field of the Invention

The present invention relates to novel erythromycin derivatives or salts thereof as antibacterial agents, which have excellent antibacterial activity especially against atypical acid-fast mycobacteria including multiple drug-resistant bacteria. The present invention also relates to medicaments comprising the same as an active ingredient.

### Related Art

Atypical acid-fast mycobacteria have low sensitivity to various antibacterial agents including antituberculosis agents, and for this reason, atypical acid-fast mycobacteriosis is extremely intractable diseases. Rifampicin (The Merck Index, 12th edition, 8382) and the like are known as compounds that can be applied to similar disease to those treated by the compounds of the present invention. Furthermore, as macrolide derivatives that have a similar chemical structure to that of the compounds of the present invention, clarithromycin (The Merck Index, 12th edition, 2400), roxithromycin (The Merck Index, 12th edition, 8433) as a 9-oxime type compound and the like are known as antibacterial agents. Moreover, 4"-O-acetylerythromycin A 9-(O-methyloxime) and the like are reported in Japanese Patent Unexamined Publication (KOKAI) No. 63-107921/1988 to have a viral replication inhibitory action. However, it has little been known that these macrolide derivatives have antibacterial activity against atypical acid-fast mycobacteria. Clinical application of clarithromycin has been approved in the United State and other countries, which is considered as the most promising agent for the treatment of atypical acid-fast mycobacteriosis among macrolide derivatives at present. However, antibacterial activity of clarithromycin is also not sufficient as an agent for treatment of atypical acid-fast mycobacteriosis. Therefore, development of more excellent antibacterial agents has been desired.

In recent years, the increase of opportunistic infections has become a big social problem. Causes of the increase of the opportunistic infections may include increase of compromised hosts with degraded biophylaxis mechanism such as patients infected by human immunodeficiency virus (HIV), patients of cancer and diabetes, and elderly persons, increase of multiple drug-resistant bacteria whose typical examples are Methicillin-resistant Staphylococcus aureus and the like, microbial substitution of patients by these bacteria and so forth. For these causes, chemotherapy of opportunistic infections become more difficult.

Atypical acid-fast mycobacteriosis is one of the opportunistic infections. Atypical acid-fast mycobacteria, the causal bacteria of the atypical acid-fast mycobacteriosis, proliferate slowly, and even when they are captured by phagocytes, they can survive in the cells for a long period of time. Therefore, prolonged chemotherapy is requires to treat infections by these bacteria. In particular, among the atypical acid-fast mycobacteria, few effective antibacterial agents are available against *Mycobacterium avium* complex (MAC), and accordingly, surgical treatment for the therapeutic treatment of this infection has also been studied at present. Moreover, even the aforementioned clarithromycin lacks selectivity to the atypical acid-fast mycobacteria, and clarithromycin resistant MACs have already been known. As explained above, various problems arise in chemotherapy of atypical acid-fast mycobacteriosis, for example, low sensitivity to known antibacterial agents, and conditions of high possibility of microbial substitution or emergence of resistant bacteria.

### Disclosure of the Invention

An object of the present invention is to provide a compound that has selective and excellent antibacterial activity against atypical acid-fast mycobacteria.

The inventors of the present invention eagerly conducted researches to achieve the aforementioned object. As a result, they found that the novel erythromycin derivatives or salts thereof according to the present invention were useful as antibacterial agents, and that they had excellent antibacterial activity particularly against atypical acid-fast mycobacteria. The present invention was achieved on the basis of the findings.

The present invention thus relates to novel erythromycin derivatives represented by the following general formula (I) or salts thereof: 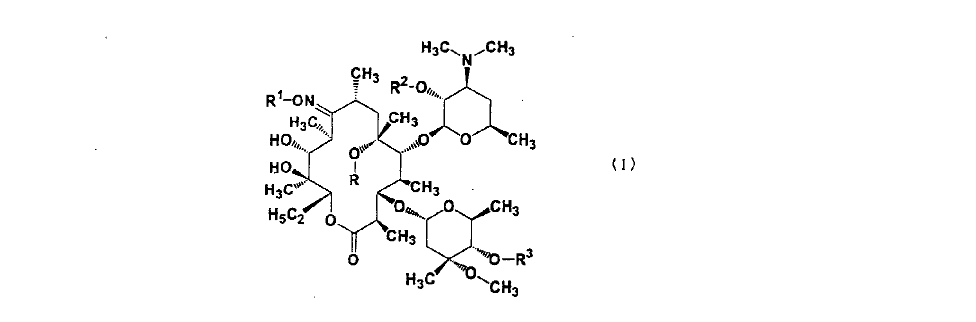 wherein R represents a hydrogen atom or a lower alkyl group; R¹ represents a C₅₋₁₂ alkyl group which may be substituted, a cycloalkyl group which may be substituted, a (cycloalkyl)alkyl group which may be substituted, an aralkyl group which may be substituted, or a group represented by the formula -(CH₂)ₙ-X-R⁴; R² represents a hydrogen atom or an acetyl group; R³ represents an acyl group which may be substituted, or a group represented by the formula -C(=O)-Y-R⁵; R⁴ represents an alkyl group which may be substituted, an alkoxyalkyl group which may be substituted, an alkylthioalkyl group which may be substituted, an alkylaminoalkyl group which may be substituted, an aryl group which may be substituted or an aralkyl group which may be substituted; R⁵ represents an alkyl group which may be substituted, an aryl group which may be substituted, or an aralkyl group which may be substituted; n represents an integer of from 1 to 6; X represents an oxygen atom, a sulfur atom, or a group represented by -NZ-; Y represents an oxygen atom or a group represented by -NH-; and Z represents a hydrogen atom or an alkyl group which may be substituted.

According to the second aspect of the present invention, there are provided compounds represented by the following general formula (II) or salts thereof: 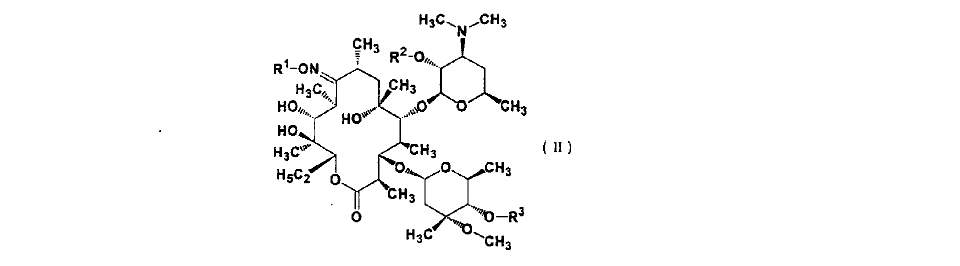 wherein R¹, R², and R³ have the same meanings as those defined above, which corresponds to the compounds represented by the aforementioned general formula (I) wherein R is a hydrogen atom.

According to the third aspect of the present invention, there are provided the compounds represented by the aforementioned general formulas (I) and (II) or salts thereof wherein R² is a hydrogen atom.

According to further aspect of the present invention, there are provided a medicament which comprises a compound represented by the aforementioned general formula (I) and (II) or a physiologically acceptable salt thereof as an active ingredient. The medicament provided by the present invention can suitably be used as, for example, an antibacterial agent, in particular, an agent for treatment of atypical acid-fast mycobacteriosis.

The present invention further provides a use of a compound represented by the aforementioned general formula (I) and (II) or a physiologically acceptable salt thereof for the manufacture of the aforementioned medicament; and a method for therapeutic treatment of infectious diseases, in particular a method for therapeutic treatment of atypical acid-fast mycobacteriosis which comprises the step of administering to a mammal including a human a therapeutically effective amount of a compound represented by the aforementioned general formula (I) and (II) or a physiologically acceptable salt thereof.

### Best Mode for Carrying out the Invention

The novel erythromycin derivatives represented by the aforementioned general formula (I) according to the present invention will be specifically explained below. It is apparent for a skilled person that the compounds represented by the aforementioned general formula (II) of the present invention fall within the compounds represented by the aforementioned general formula (I).

In the aforementioned general formula (I) of the present invention, the lower alkyl group represented by R can be, for example, methyl group, ethyl group, n-propyl group, n-butyl group or the like. The alkyl group of the optionally substituted C₅₋₁₂ alkyl group represented by R¹ can be a linear or branched alkyl group having 5 to 12 carbon atoms, for example, n-pentyl group, isopentyl group, neopentyl group, tert-pentyl group, n-hexyl group, 4-methylpentyl group, n-heptyl group, n-octyl group, n-nonyl group, n-decyl group, n-undecyl group, n-dodecyl group and the like. The cycloalkyl group of the optionally substituted cycloalkyl group represented by R¹ can be a cycloalkyl group having 3 to 6 carbon atoms, for example, cyclopropyl group, cyclobutyl group, cyclopentyl group, cyclohexyl group. The (cycloalkyl)alkyl group of the optionally substituted (cycloalkyl)alkyl group represented by R¹ is a group consisting of a linear or branched alkyl group having 1 to 12 carbon atoms substituted with the aforementioned cycloalkyl group at any position. Examples include, for example, cyclopropylmethyl group, cyclobutylmethyl group, cyclopentylmethyl group, cyclohexylmethyl group, cyclopropylethyl group, cyclobutylethyl group, cyclopentylethyl group, cyclohexylethyl group, cyclohexylpropyl group, cyclohexylbutyl group, cyclohexylpentyl group, cyclohexylhexyl group, cyclohexylheptyl group, cyclohexyloctyl group, cyclohexylnonyl group, cyclohexyldecyl group, cyclohexylundecyl group, cyclohexyldodecyl group and the like.

In the aforementioned general formula (I) of the present invention, the aralkyl group of the optionally substituted aralkyl group represented by R¹, R⁴ or R⁵ is a group consisting of a linear or branched alkyl group having 1 to 12 carbon atoms substituted with an aryl group at any position. Examples include, for example, benzyl group, naphthylmethyl group, naphthylethyl group, naphthylpropyl group, pyridylmethyl group, pyridylethyl group, pyridylpropyl group, pyrimidylmethyl group, pyrimidylethyl group, pyrimidylpropyl group, pyrazinylmethyl group, pyrazinylethyl group, pyrazinylpropyl group, furylmethyl group, furylethyl group, furylpropyl group, benzofuranylmethyl group, benzofuranylethyl group, benzofuranylpropyl group, thenyl group, thienylethyl group, thienylpropyl group, benzo[b]thienylmethyl group, benzo[b]thienylethyl group, benzo[b]thienylpropyl group, pyrrolylmethyl group, pyrrolylethyl group, pyrrolylpropyl group, indolylmethyl group, indolylethyl group, indolylpropyl group, imidazolylmethyl group, imidazolylethyl group, imidazolylpropyl group, benzimidazolylmethyl group, benzimidazolylethyl group, benzimidazolylpropyl group, quinolylmethyl group, quinolylethyl group, quinolylpropyl group, isoquinolylmethyl group, isoquinolylethyl group, isoquinolylpropyl group, phenethyl group, phenylpropyl group, phenylbutyl group, phenylpentyl group, phenylhexyl group, phenylheptyl group, phenyloctyl group, phenylnonyl group, phenyldecyl group, phenylundecyl group, phenyldodecyl group and the like.

The acyl group of the optionally substituted acyl group represented by R³ can be a formyl group, or a group consisting of an alkyl group, cycloalkyl group, (cycloalkyl)alkyl group, aryl group or aralkyl group which is substituted with a carbonyl group at any position. Examples include, for example, acetyl group, propionyl group, butyryl group, isobutyryl group, n-valeryl group, isovaleryl group, pivaloyl group, n-hexanoyl group, n-heptanoyl group, n-octanoyl group, n-nonanoyl group, n-decanoyl group, n-undecanoyl group, n-dodecanoyl group, n-tridecanoyl group, cyclopropylcarbonyl group, cyclobutylcarbonyl group, cyclopentylcarbonyl group, cyclohexylcarbonyl group, cyclopropylacetyl group, cyclobutylacetyl group, cyclopentylacetyl group, cyclohexylacetyl group, cyclohexylpropionyl group, cyclohexylbutyryl group, cyclohexylpentanoyl group, cyclohexylhexanoyl group, cyclohexylheptanoyl group, cyclohexyloctanoyl group, cyclohexylnonanoyl group, cyclohexyldecanoyl group, cyclohexylundecanoyl group, cyclohexyldodecanoyl group, cyclohexyltridecanoyl group, benzoyl group, naphthoyl group, nicotinoyl group, isonicotinoyl group, pyridazolylcarbonyl group, pyrazinylcarbonyl group, furoyl group, benzofuranylcarbonyl group, thenoyl group, benzo[b]thienylcarbonyl group, pyrrolylcarbonyl group, indolylcarbonyl group, imidazolylcarbonyl group, benzimidazolylcarbonyl group, quinolylcarbonyl group, isoquinolylcarbonyl group, phenylacetyl group, naphthylacetyl group, pyridylacetyl group, furylacetyl group, thienylacetyl group, pyrrolylacetyl group, phenylpropionyl group, phenylbutyryl group, phenylpentanoyl group, phenylhexanoyl group, phenylheptanoyl group, phenyloctanoyl group, phenylnonanoyl group, phenyldecanoyl group, phenylundecanoyl group, phenyldodecanoyl group, phenyltridecanoyl group, a-methylphenylacetyl group and the like.

The alkyl group of the optionally substituted alkyl group represented by R⁴, R⁵ or Z can be a linear or branched alkyl group having 1 to 12 carbon atoms. Examples include, for example, methyl group, ethyl group, n-propyl group, isopropyl group, n-butyl group, isobutyl group, sec-butyl group, tert-butyl group, n-pentyl group, isopentyl group, neopentyl group, tert-pentyl group, n-hexyl group, n-heptyl group, n-octyl group, n-nonyl group, n-decyl group, n-undecyl group, n-dodecyl group and the like. The alkoxyalkyl group of the optionally substituted alkoxyalkyl group represented by R⁴ is a group consisting of a linear or branched alkyl group having 1 to 12 carbon atoms substituted with a linear or branched alkoxyl group having 1 to 12 carbon atoms at any position. Examples include, for example, methoxymethyl group, ethoxymethyl group, n-propoxymethyl group, isopropoxymethyl group, n-butoxymethyl group, isobutoxymethyl group, sec-butoxymethyl group, tert-butoxymethyl group, n-pentyloxymethyl group, isopentyloxymethyl group, neopentyloxymethyl group, tert-pentyloxymethyl group, n-hexyloxymethyl group, n-heptyloxymethyl group, n-octyloxymethyl group, n-nonyloxymethyl group, n-decyloxymethyl group, n-undecyloxymethyl group, n-dodecyloxymethyl group, methoxyethyl group, methoxypropyl group, methoxybutyl group, methoxypentyl group, methoxyhexyl group, methoxyheptyl group, methoxyoctyl group, methoxynonyl group, methoxydecyl group, methoxyundecyl group, methoxydodecyl group, ethoxyethyl group, n-hexyloxyethyl group, n-dodecyloxyethyl group, n-propoxypropyl group and the like. The alkylthioalkyl group of the optionally substituted alkylthioalkyl group represented by R⁴ is a group consisting of a linear or branched alkyl group having 1 to 12 carbon atoms substituted with a linear or branched alkylthio group having 1 to 12 carbon atoms at any position. Examples include, for example, methylthiomethyl group, ethylthiomethyl group, n-propylthiomethyl group, isopropylthiomethyl group, n-butylthiomethyl group, isobutylthiomethyl group, sec-butylthiomethyl group, tert-butylthiomethyl group, n-pentylthiomethyl group, isopentylthiomethyl group, neopentylthiomethyl group, tert-pentylthiomethyl group, n-hexylthiomethyl group, n-heptylthiomethyl group, n-octylthiomethyl group, n-nonylthiomethyl group, n-decylthiomethyl group, n-undecylthiomethyl group, n-dodecylthiomethyl group, methylthioethyl group, methylthiopropyl group, methylthiobutyl group, methylthiopentyl group, methylthiohexyl group, methylthioheptyl group, methylthiooctyl group, methylthiononyl group, methylthiodecyl group, methylthioundecyl group, methylthiododecyl group and the like. The alkylaminoalkyl group of the optionally substituted alkylaminoalkyl group represented by R⁴ is a group consisting of a linear or branched alkyl group having 1 to 12 carbon atoms substituted with an amino group furthermore substituted with one or two linear or branched alkyl groups having 1 to 12 carbon atoms at any position. Examples include, for example, methylaminomethyl group, ethylaminomethyl group, n-propylaminomethyl group, isopropylaminomethyl group, n-butylaminomethyl group, isobutylaminomethyl group, sec-butylaminomethyl group, tert-butylaminomethyl group, n-pentylaminomethyl group, isopentylaminomethyl group, neopentylaminomethyl group, tert-pentylaminomethyl group, n-hexylaminomethyl group, n-heptylaminomethyl group, n-octylaminomethyl group, n-nonylaminomethyl group, n-decylaminomethyl group, n-undecylaminomethyl group, n-dodecylaminomethyl group, methylaminoethyl group, methylaminopropyl group, methylaminobutyl group, methylaminopentyl group, methylaminohexyl group, methylaminoheptyl group, methylaminooctyl group, methylaminononyl group, methylaminodecyl group, methylaminoundecyl group, methylaminododecyl group, dimethylaminomethyl group, dimethylaminoethyl group and the like.

In the aforementioned general formula (I) of the present invention, the aryl group of the optionally substituted aryl group represented by R⁴ or R⁵ can be, for example, phenyl group, naphthyl group, pyridyl group, pyrimidyl group, pyrazinyl group, furyl group, benzofuranyl group, thienyl group, benzo[b]thienyl group, pyrrolyl group, indolyl group, imidazolyl group, benzimidazolyl group, quinolyl group, isoquinolyl group and the like.

In the aforementioned general formula (I) of the present invention, when a functional group is defined as "a group which may be substituted", the substituent is not limited so long as it can exist on the functional group. Number and kind of the substituent are not particularly limited. When two or more substituents are present, they may be the same or different. The position of the substituent is not also limited. Examples of substitutable groups include, for example, a hydroxyl group which may be protected, an alkoxyl group, an amino group which may be substituted, a carbamoyl group which may be substituted, a halogen atom, an alkyl group, trifluoromethyl group, an acyl group, a cycloalkyl group, an aryl group, an aryloxy group, cyano group, nitro group, guanidino group, amidino group, carboxyl group, an alkoxycarbonyl group, an aryloxycarbonyl group, aralkyloxycarbonyl group and the like. The protective group of the hydroxyl group may be any protective group so long as a protected group is substantially inactive in a reaction system in which the hydroxyl group should not be involved in the reaction, and the protected group is readily cleavable under a certain condition for deprotection. Examples of the protective group include, for example, an acyl group, trialkylsilyl group such as trimethylsilyl group and triethylsilyl group, benzyl group and the like. The alkoxyl group can be a linear or branched alkoxyl group, and examples include, for example, methoxy group, ethoxy group, n-propoxy group, isopropoxy group, n-butoxy group, isobutoxy group, sec-butoxy group, tert-butoxy group, n-pentyloxy group, isopentyloxy group, neopentyloxy group, tert-pentyloxy group, n-hexyloxy group and the like. Examples of the amino group which may be substituted include, for example; amino group, methylamino group, ethylamino group, n-propylamino group, isopropylamino group, n-butylamino group, isobutylamino group, sec-butylamino group, tert-butylamino group, n-pentylamino group, isopentylamino group, neopentylamino group, tert-pentylamino group, n-hexylamino group, dimethylamino group, diethylamino group and the like. Examples of the carbamoyl group which may be substituted include, for example, carbamoyl group, N-methylcarbamoyl group, N-ethylcarbamoyl group, N-n-propylcarbamoyl group, N-isopropylcarbamoyl group, N-n-butylcarbamoyl group, N-isobutylcarbamoyl group, N-sec-butylcarbamoyl group, N-tert-butylcarbamoyl group, N-n-pentylcarbamoyl group, N-isopentylcarbamoyl group, N-neopentylcarbamoyl group, N-tert-pentylcarbamoyl group, N-n-hexylcarbamoyl group, N,N-dimethylcarbamoyl group, N,N-diethylcarbamoyl group and the like. Examples of the halogen atom include, for example, fluorine atom, chlorine atom, bromine atom or iodine atom. Examples of the aryloxy group include, for example, phenoxy group, naphthyloxy group, pyridyloxy group, pyrimidyloxy group, pyrazinyloxy group, benzofuranyloxy group, thienyloxy group, indolyloxy group, benzimidazolyloxy group, quinolyloxy group, isoquinolyloxy group and the like. Examples of the alkoxycarbonyl group include, for example, methoxycarbonyl group, ethoxycarbonyl group, n-propoxycarbonyl group, isopropoxycarbonyl group, n-butoxycarbonyl group, isobutoxycarbonyl group, sec-butoxycarbonyl group, tert-butoxycarbonyl group, n-pentyloxycarbonyl group, isopentyloxycarbonyl group, neopentyloxycarbonyl group, tert-pentyloxycarbonyl group, n-hexyloxycarbonyl group and the like. Examples of the aryloxycarbonyl group include, for example, phenoxycarbonyl group, naphthyloxycarbonyl group, pyridyloxycarbonyl group, pyrimidyloxycarbonyl group, benzofuranyloxycarbonyl group, thienyloxycarbonyl group, indolyloxycarbonyl group, benzimidazolyloxycarbonyl group, quinolyloxycarbonyl group, isoquinolyloxycarbonyl group and the like. Examples of the aralkyloxycarbonyl group include, for example, benzyloxycarbonyl group, naphthylmethyloxycarbonyl group, pyridylmethyloxycarbonyl group, furylmethyloxycarbonyl group, benzofuranylmethyloxycarbonyl group, thenyloxycarbonyl group, indolylmethyloxycarbonyl group, imidazolylmethyloxycarbonyl group, benzimidazolylmethyloxycarbonyl group, quinolylmethyloxycarbonyl group, isoquinolylmethyloxycarbonyl group and the like.

As examples of the aforementioned alkyl group, acyl group, cycloalkyl group and aryl group which may be substituted, those groups exemplified above may be used.

In the specification, the substitution or bonding position on the "aryl group" of the "aralkyl group", the "acyl group", the "aryl group", the "aryloxy group", the "aryloxycarbonyl group" and the "aralkyloxycarbonyl group" may be any positions so far that ring constituting elements at the position are substitutable or bond-formable.

The compounds represented by the aforementioned general formula (I) of the present invention have asymmetric carbons, and therefore they can exist as stereoisomers such as optical isomers, diastereoisomers, and geometrical isomers. Any of these isomers and any mixtures thereof or racemates thereof, and salts thereof also fall within the scope of the present invention.

The compounds represented by the aforementioned general formula (I) of the present invention can be converted into salts, preferably physiologically acceptable salts, if desired, and the resulting salts can also be converted into compounds in free forms. Examples of salts of the compounds represented by the aforementioned general formula (I) of the present invention include, for example, acid addition salts or alkali addition salts. Examples of the acid addition salts include, for example, mineral acid salts such as hydrochlorides, hydrobromide, nitrates, sulfates, hydroiodides and phosphates, and organic acid salts such as acetates, propionates, butyrates, isobutyrates, formates, valerates, isovalerates, pivalates, trifluoroacetates, acrylates, maleates, tartrates, citrates, oleates, laurates, stearates, myristates, succinates, lactobionates, glutarates, sebacates, gluconates, benzoates, methanesulfonates, ethanesulfonates, 2-hydroxyethanesulfonates, benzenesulfonates, phthalates, terephthalates, cinnamates, p-toluenesulfonates, laurylsulfates, gluceptates, malates, malonates, aspartates, glutamates, adipates, oxalates, nicotinates, picrates, thiocyanates, undecanoates, mandelates, fumarates, 10-camphorsulfonates, lactates, 5-oxotetrahydrofuran-2-carboxylates, 2-hydroxyglutarates. Examples of the alkali addition salts include, for example, inorganic alkali salts such as sodium salts, potassium salts, calcium salts, magnesium salts and ammonium salts, and organic base salts such as ethanolamine salts and N,N-dialkylethanolamine salts, and salts of the optically active substances thereof.

The compounds represented by the aforementioned general formula (I) or salts thereof according to the present invention can exist in the forms of any crystals or any solvates with water or organic solvents depending on the manufacturing conditions. These crystals, hydrates, solvates, and mixtures thereof also fall within the scope of the present invention.

Preferred compounds of the present invention include the compounds listed below. However, the present invention is not limited to these examples. In the tables, Me represents methyl group, Et represents ethyl group, n-Pr represents n-propyl group, i-Pr represents isopropyl group, n-Bu represents n-butyl group, i-Bu represents isobutyl group, s-Bu represents sec-butyl group, t-Bu represents tert-butyl group, n-Pent represents n-pentyl group, i-Pent represents isopentyl group, neo-Pent represents neopentyl group, t-Pent represents tert-pentyl group, n-Hex represents n-hexyl group, n-Hept represents n-heptyl group, n-Oct represents n-octyl group, n-Non represents n-nonyl group, n-Dec represents n-decyl group, n-Undec represents n-undecyl group, n-Dodec represents n-dodecyl group, and Ac represents acetyl group.

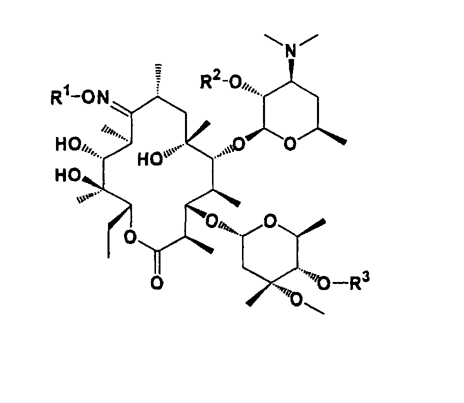

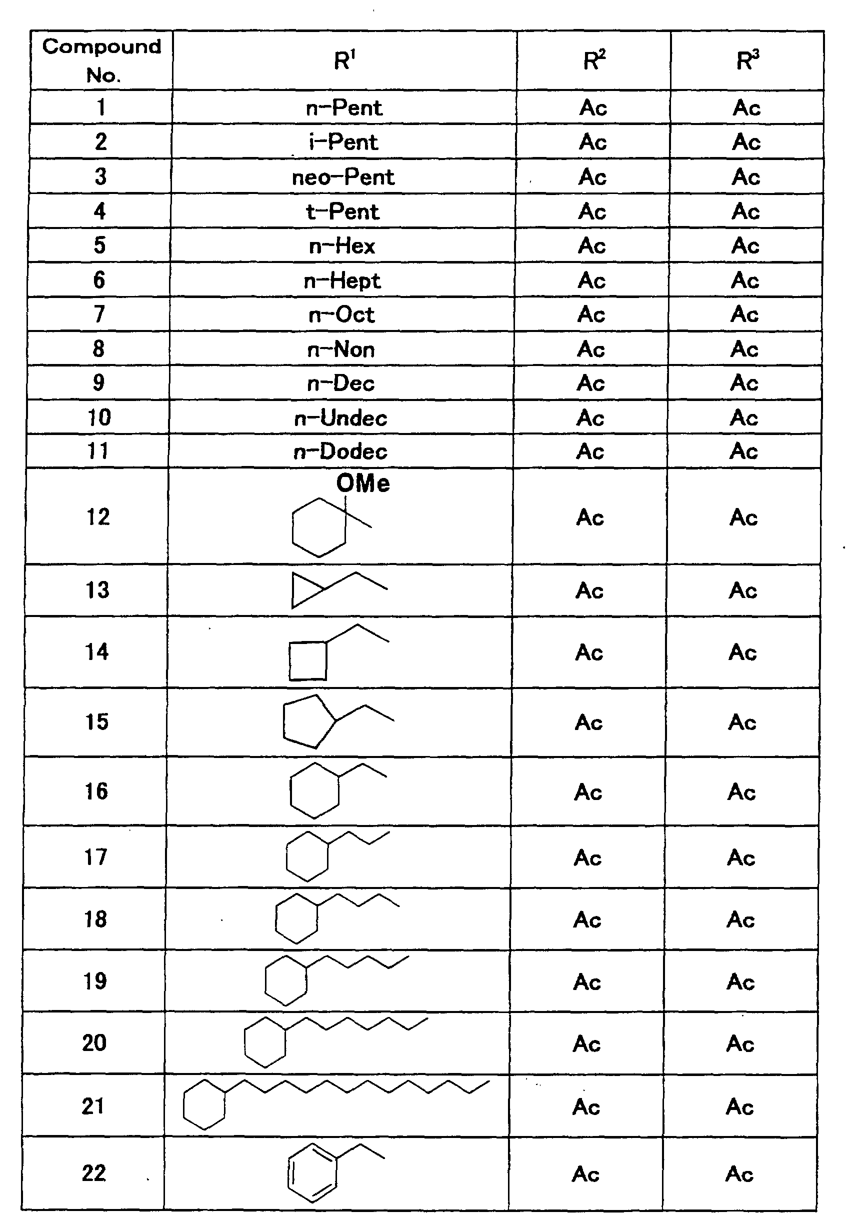

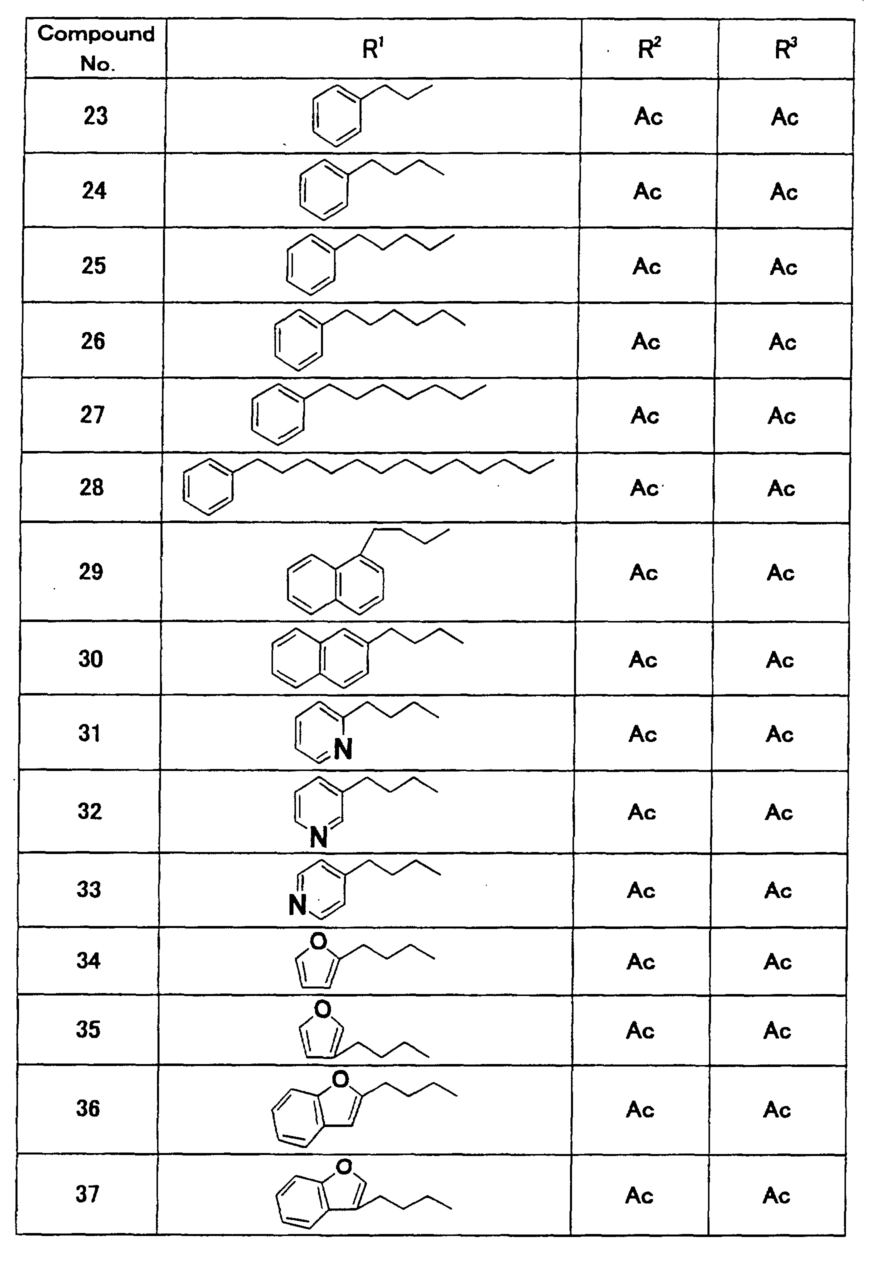

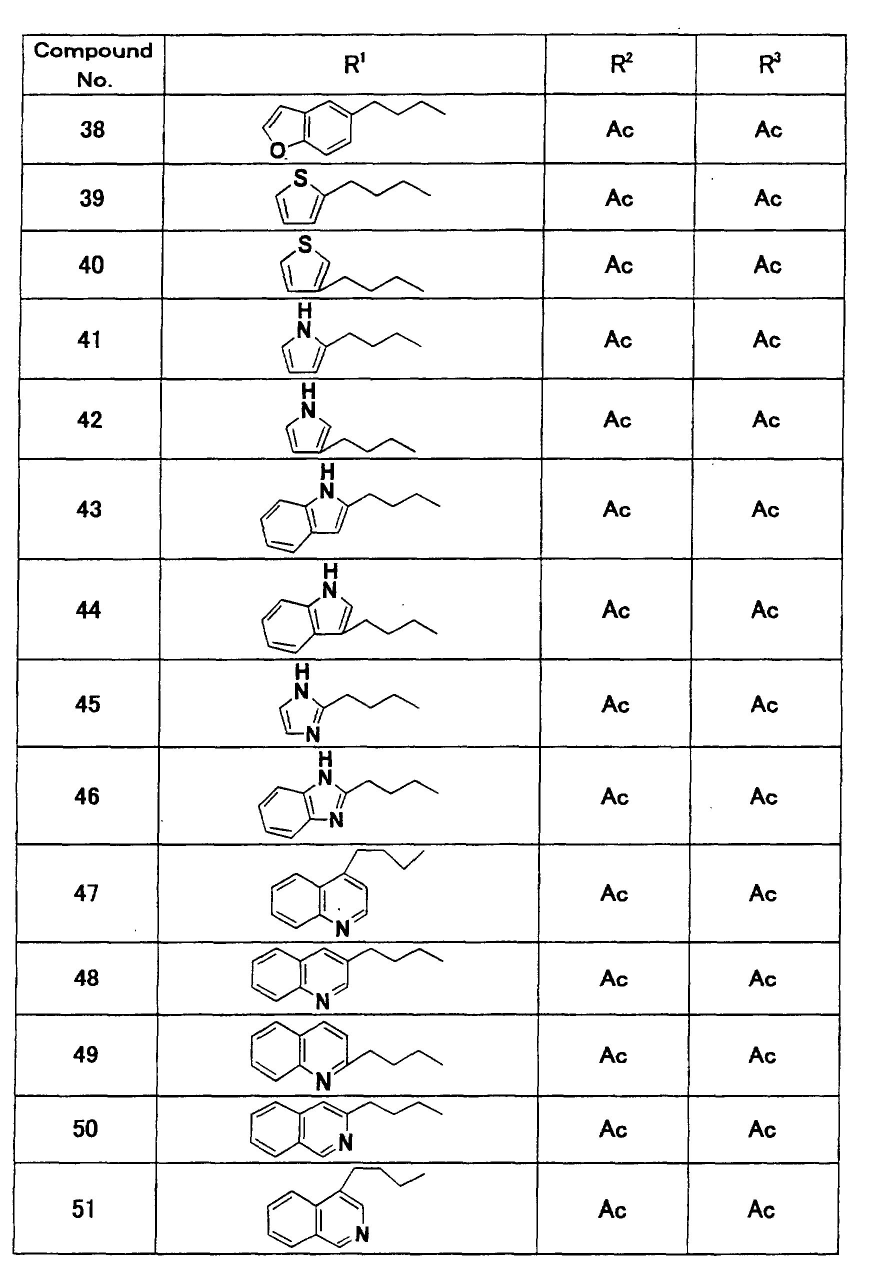

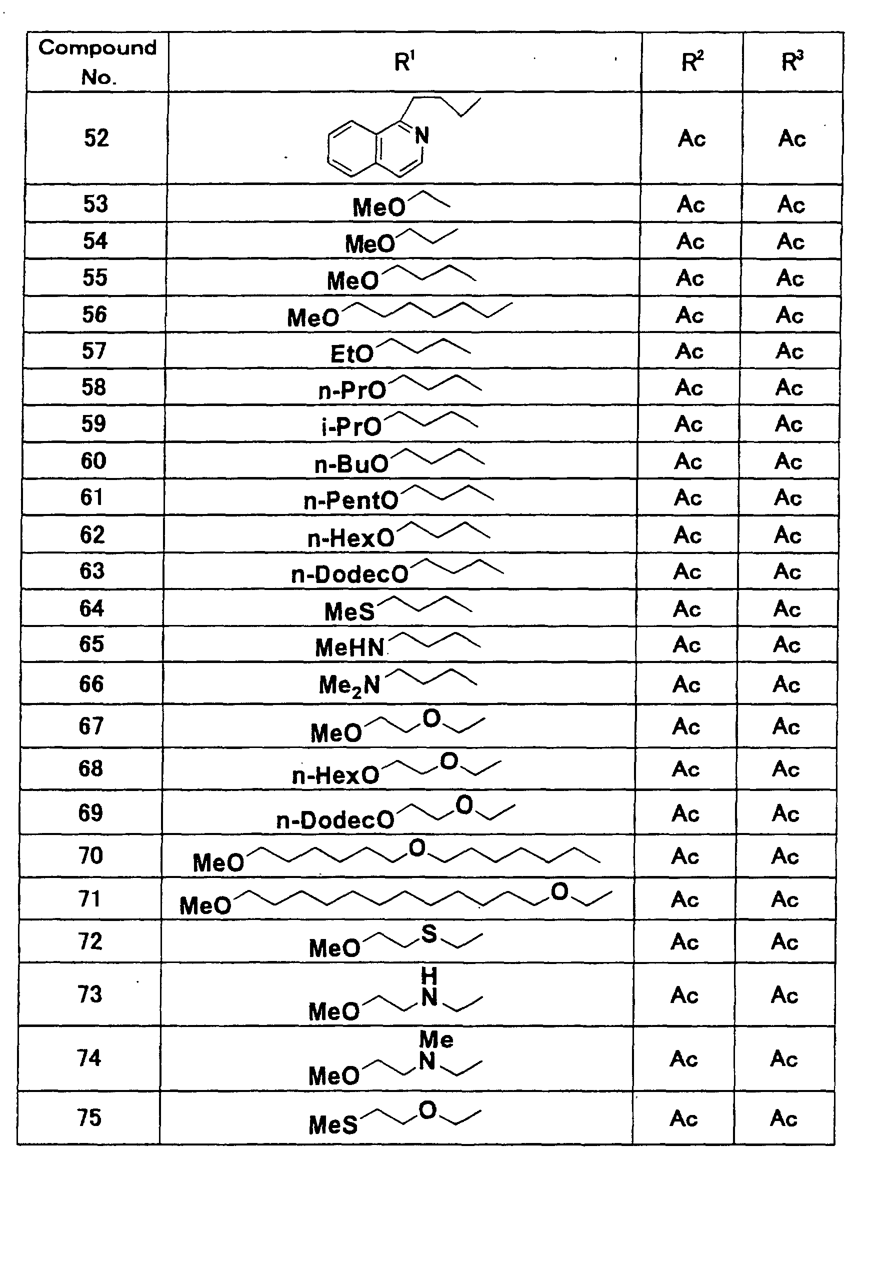

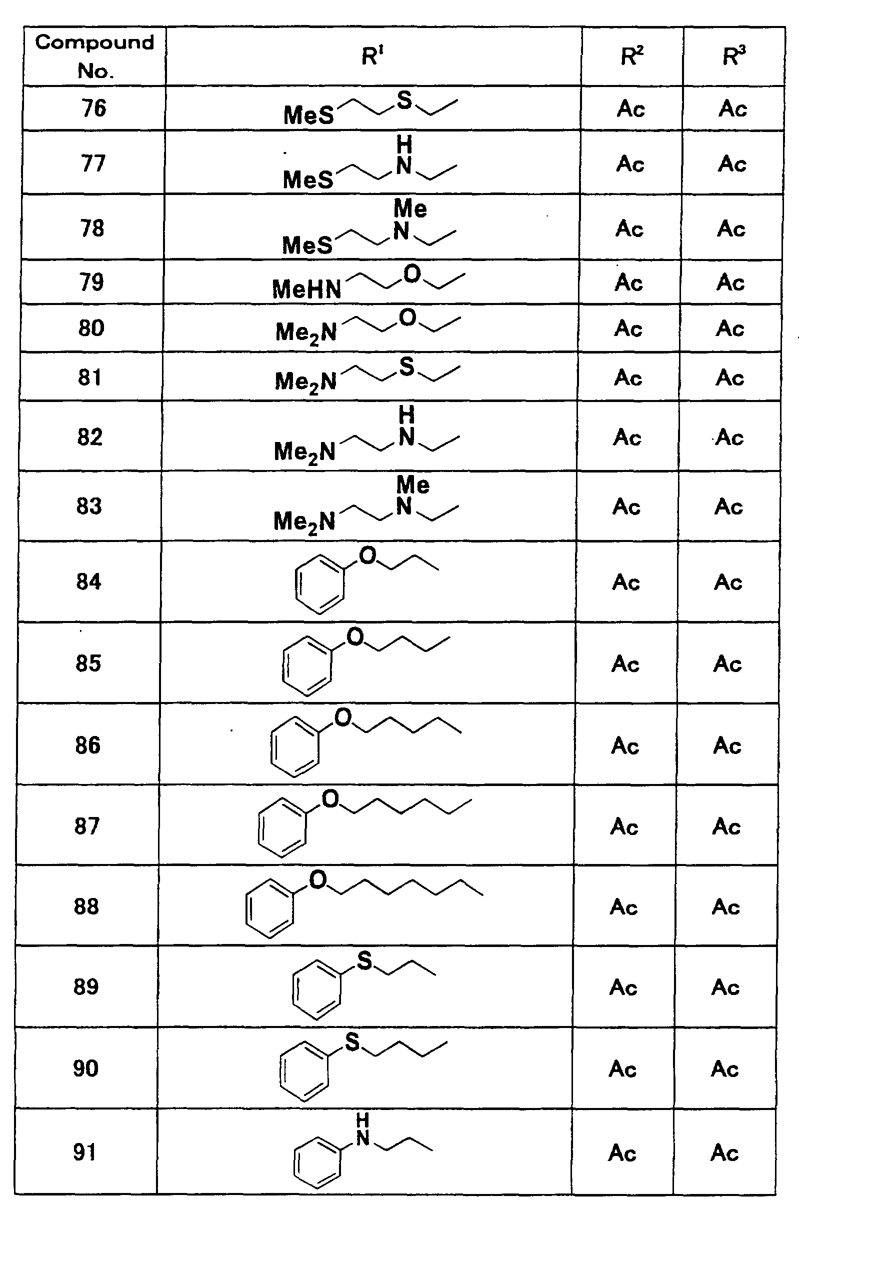

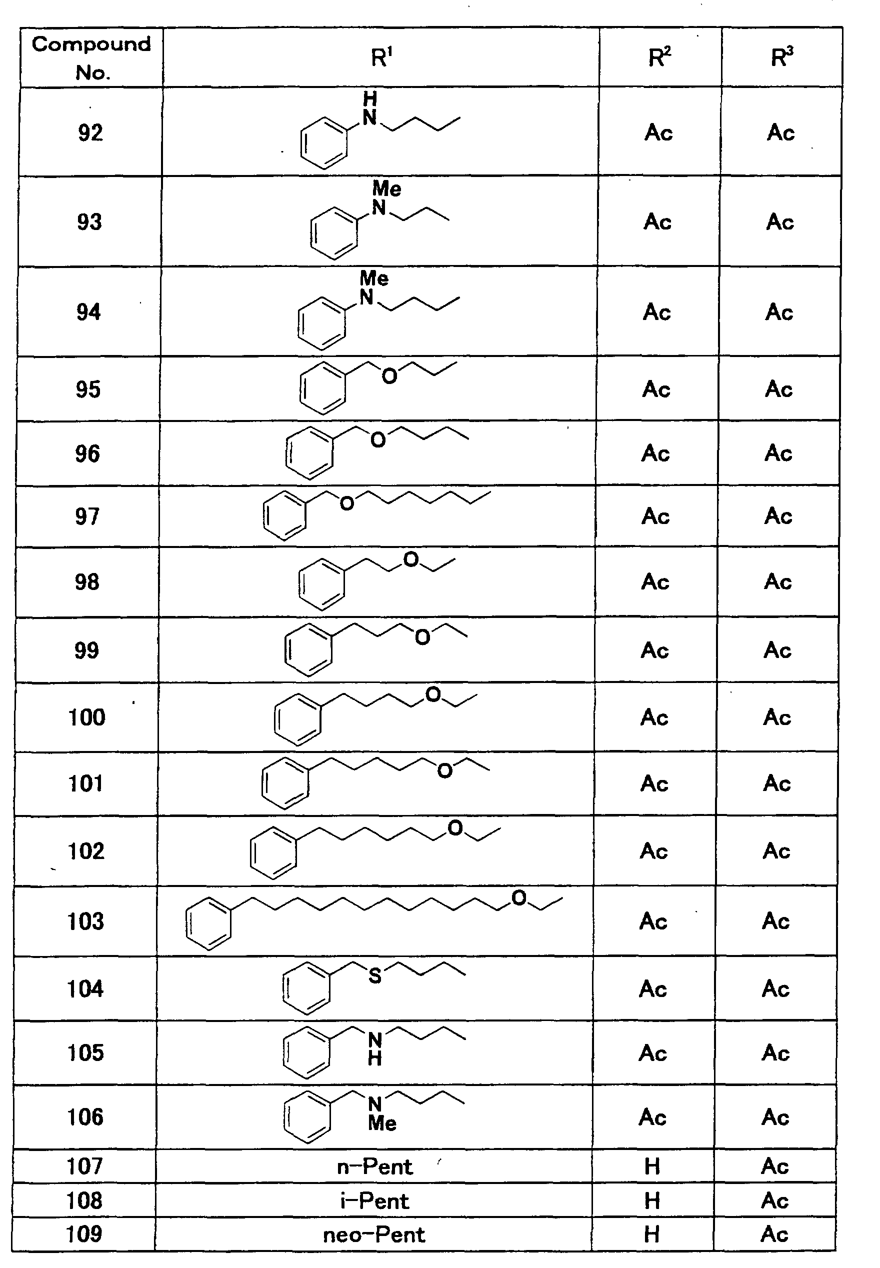

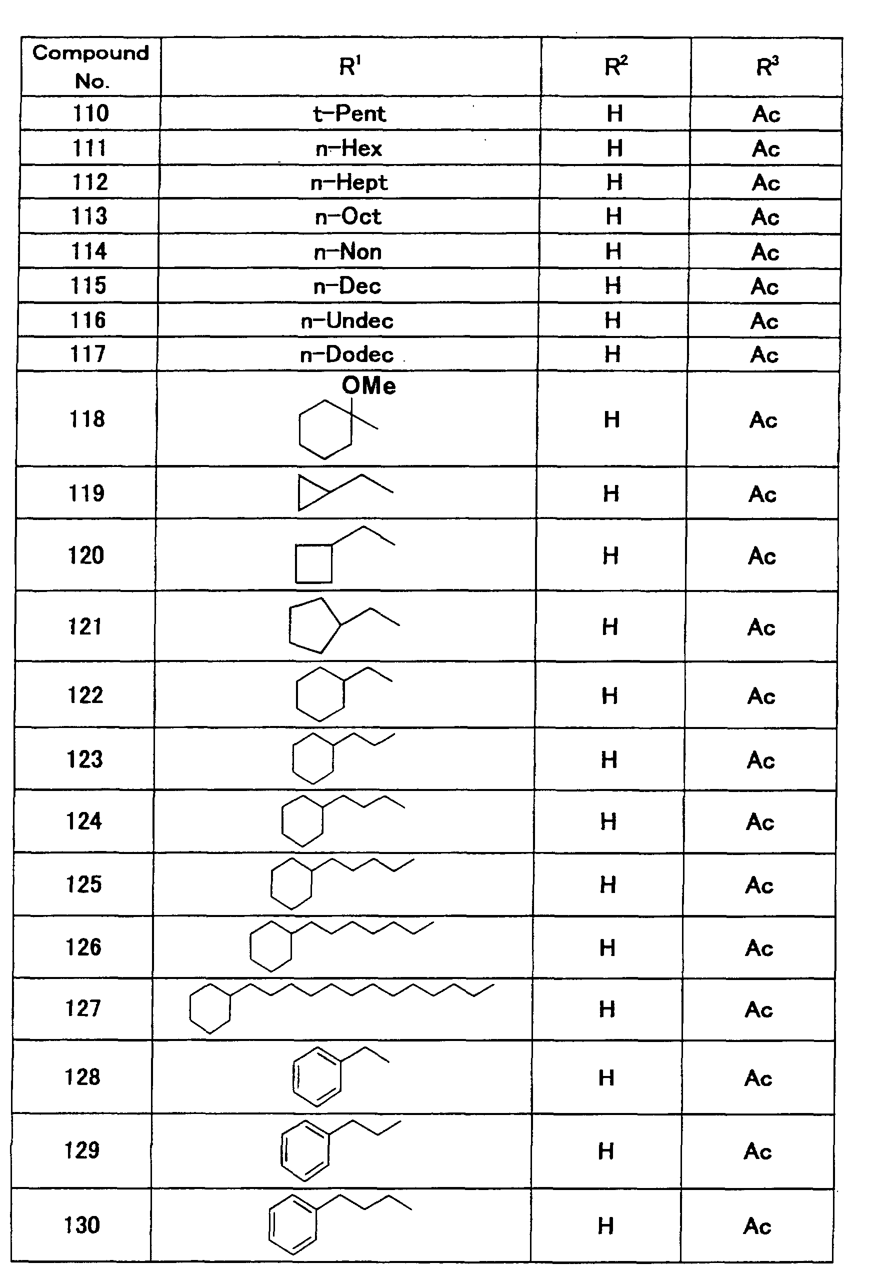

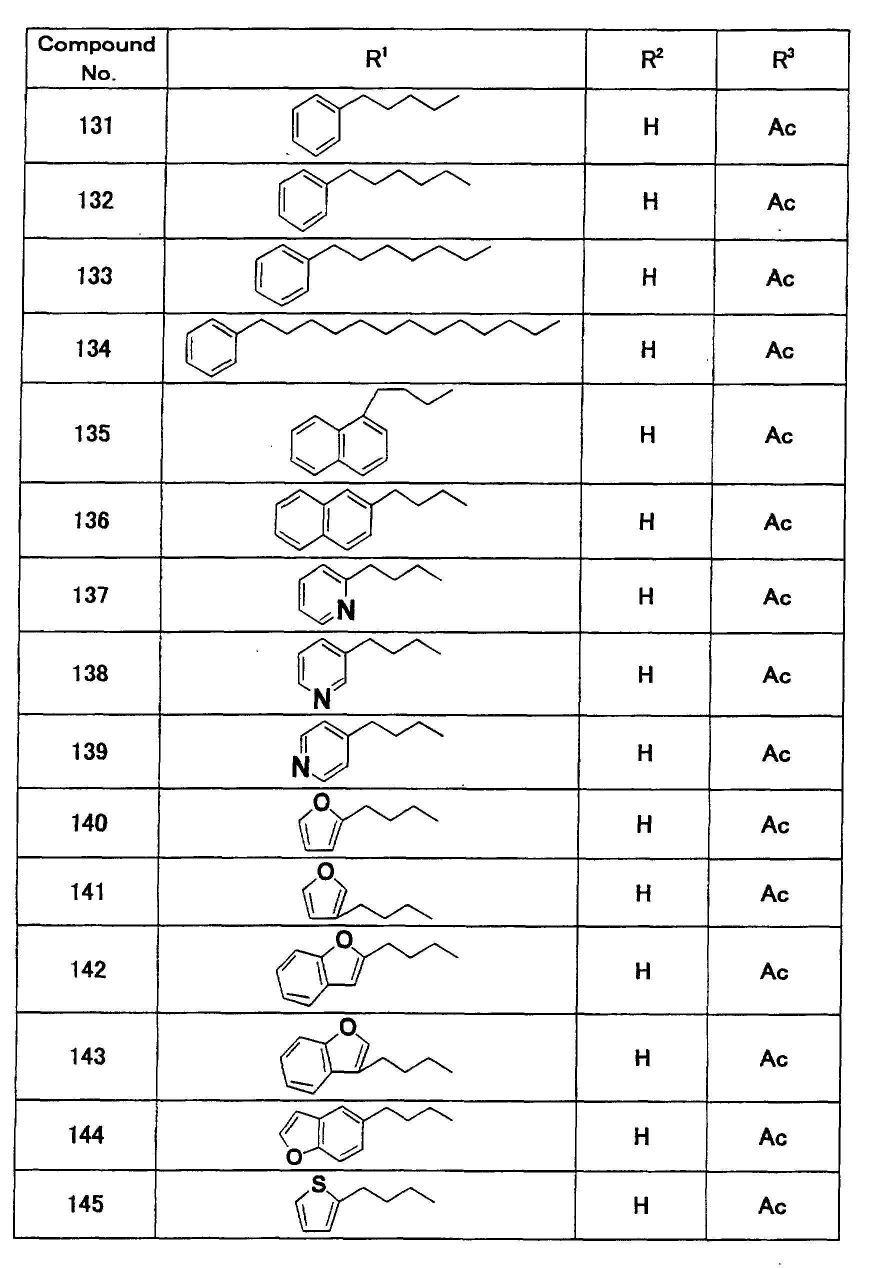

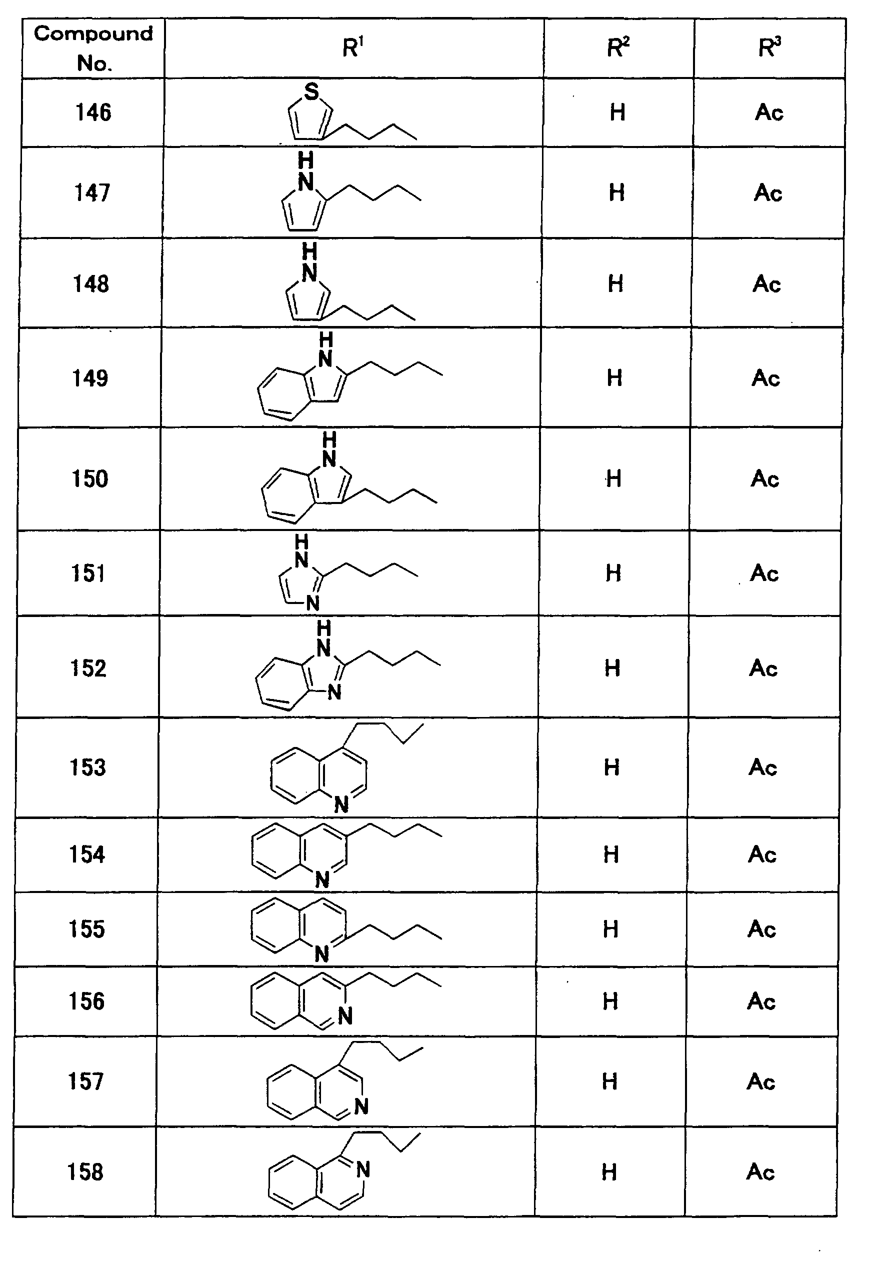

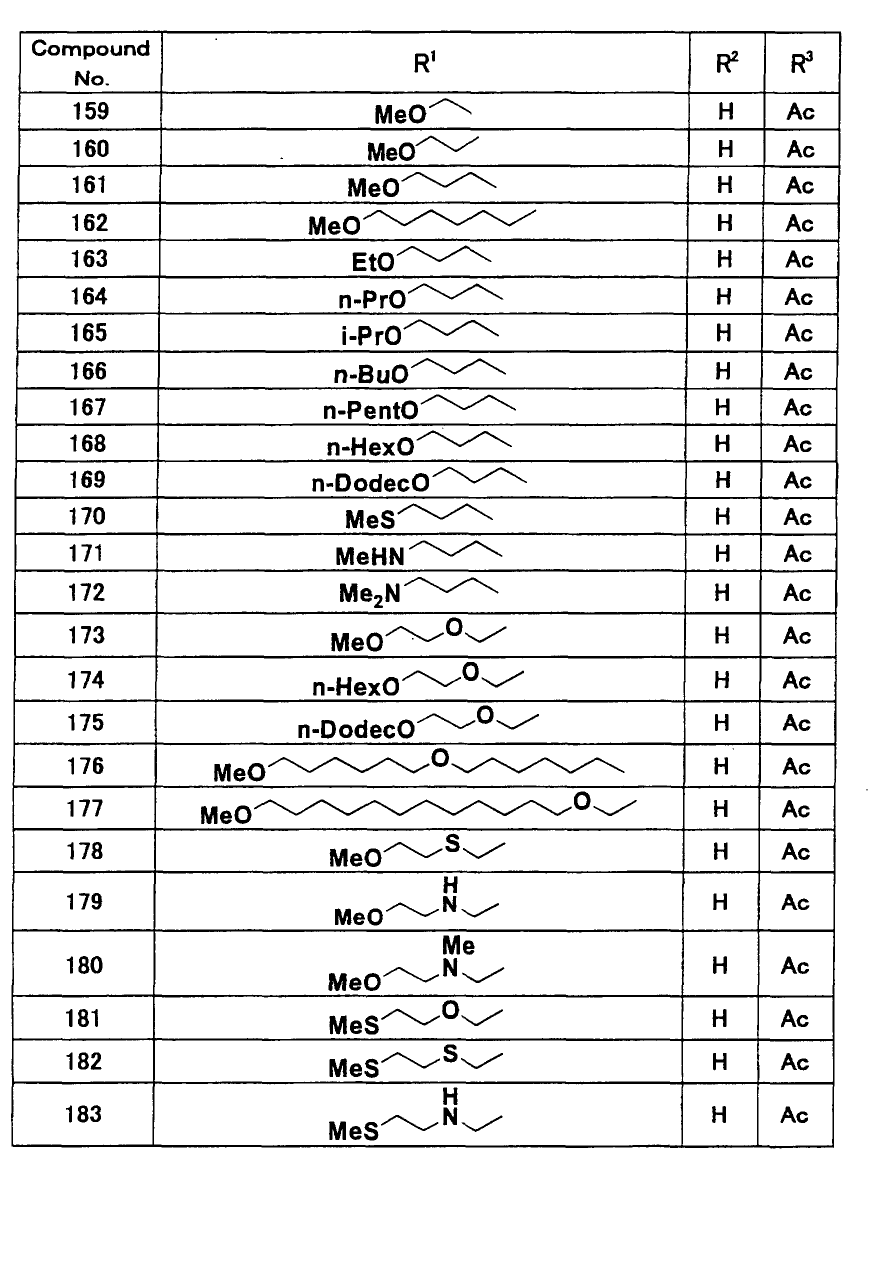

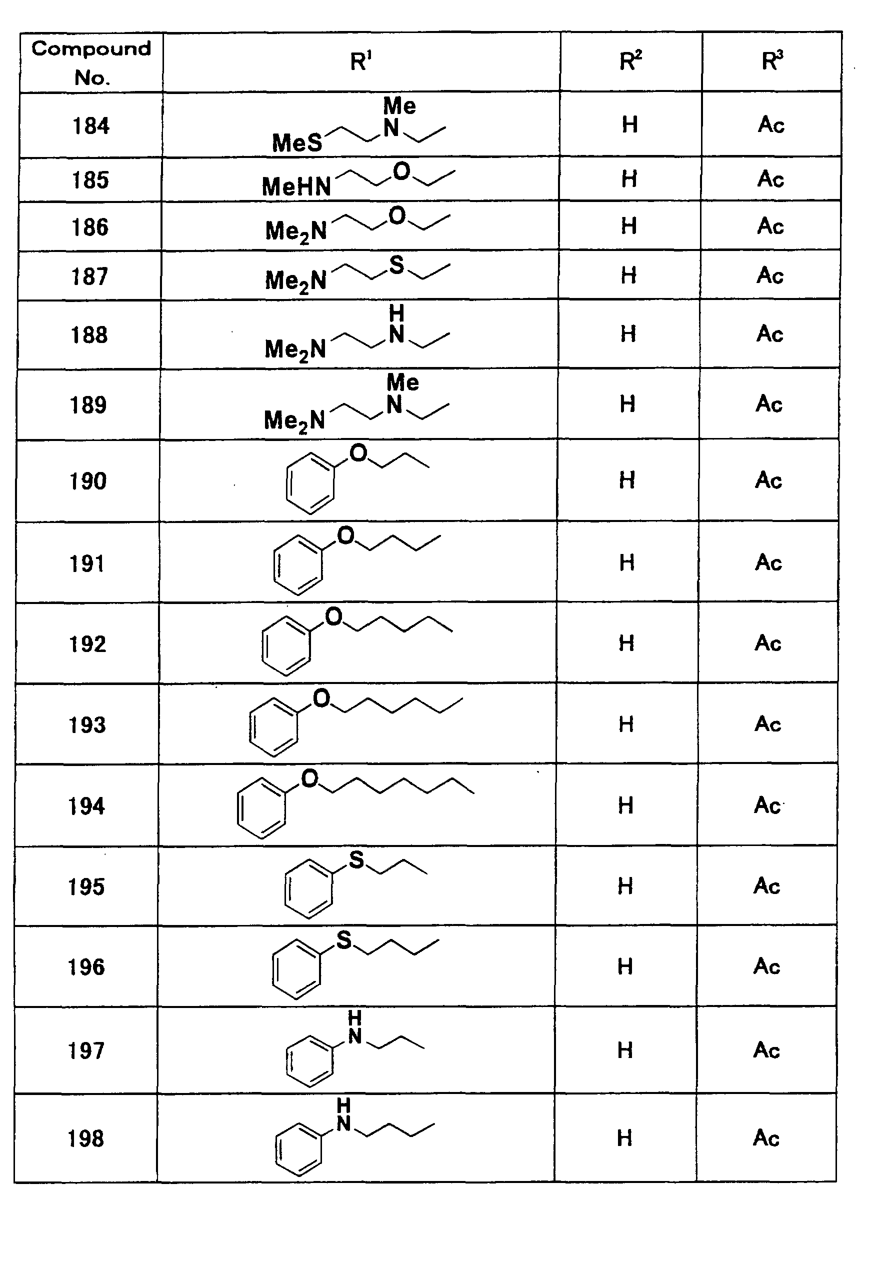

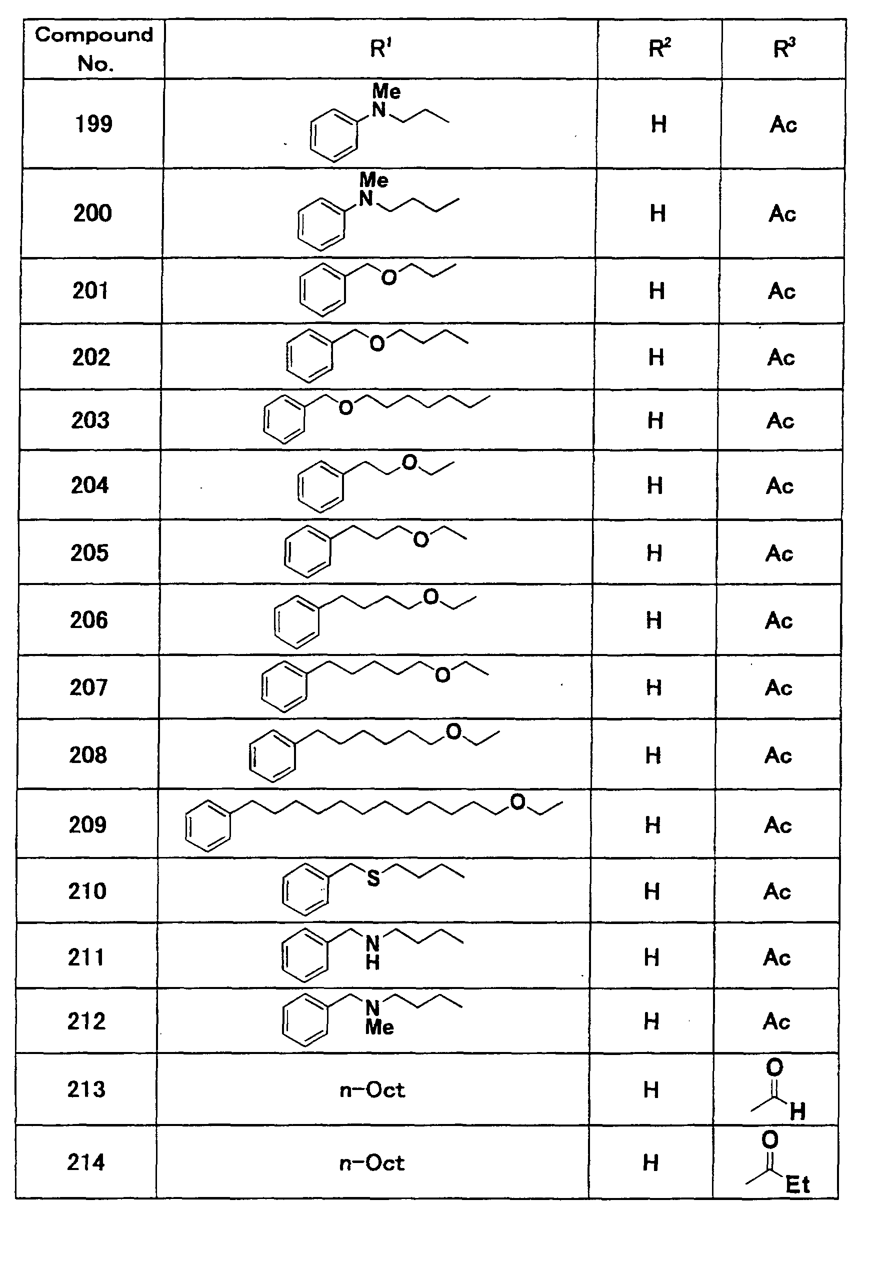

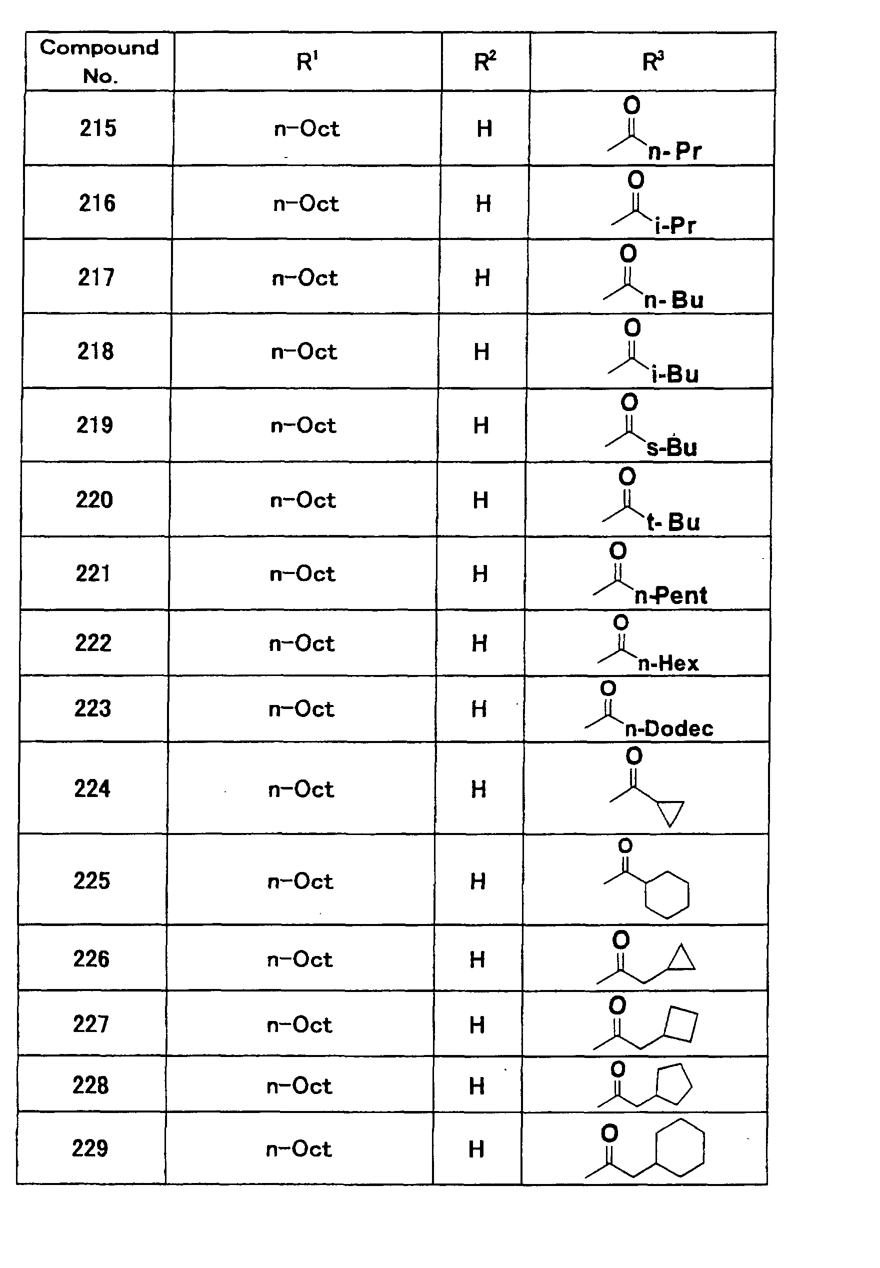

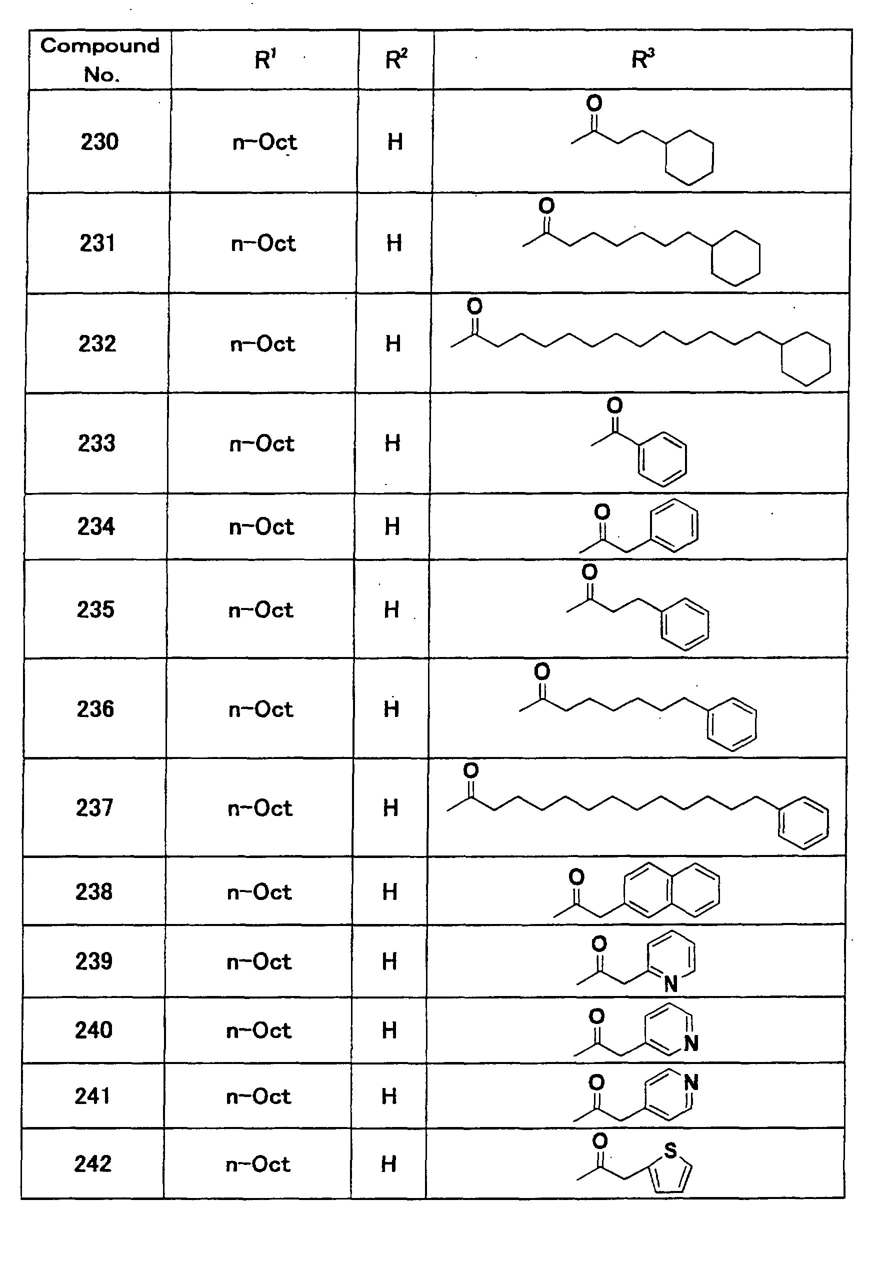

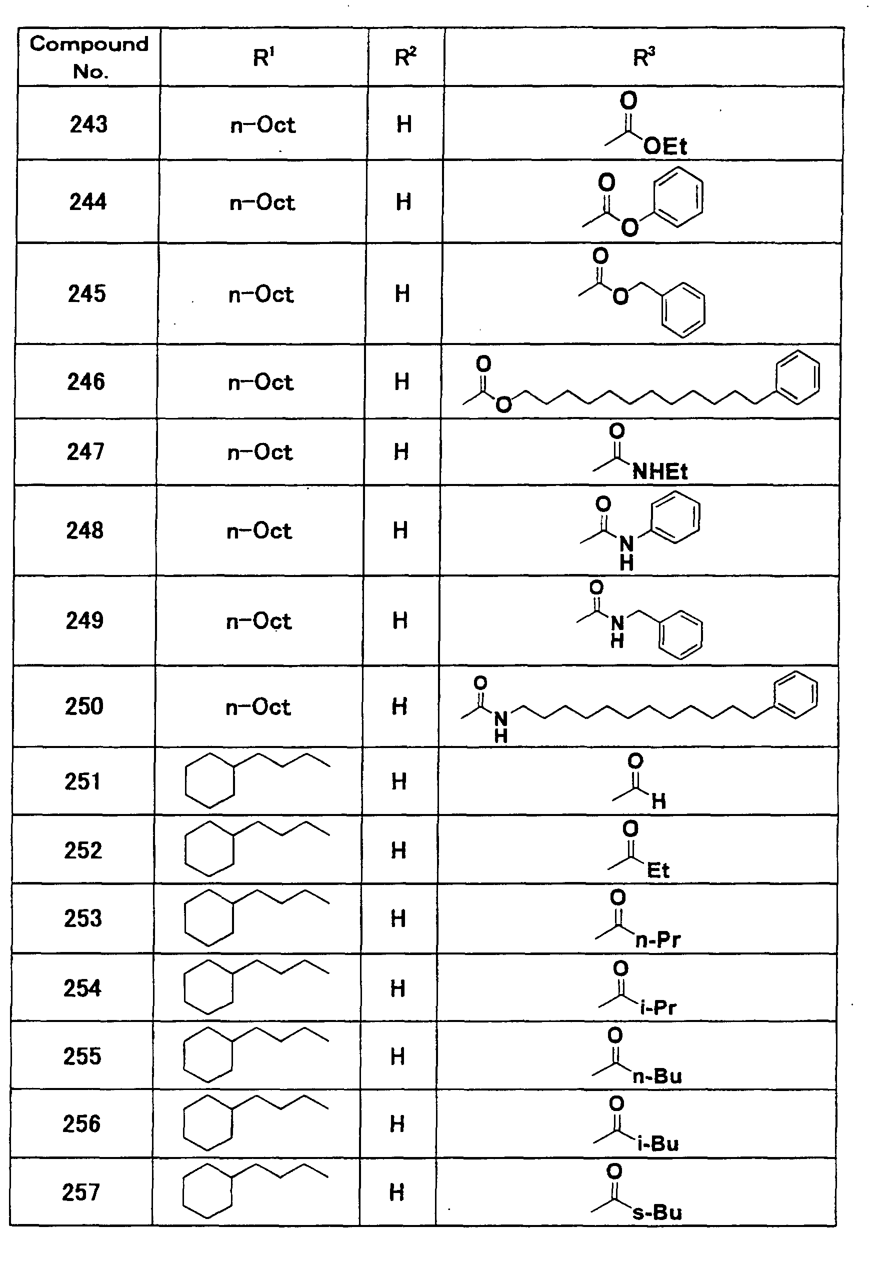

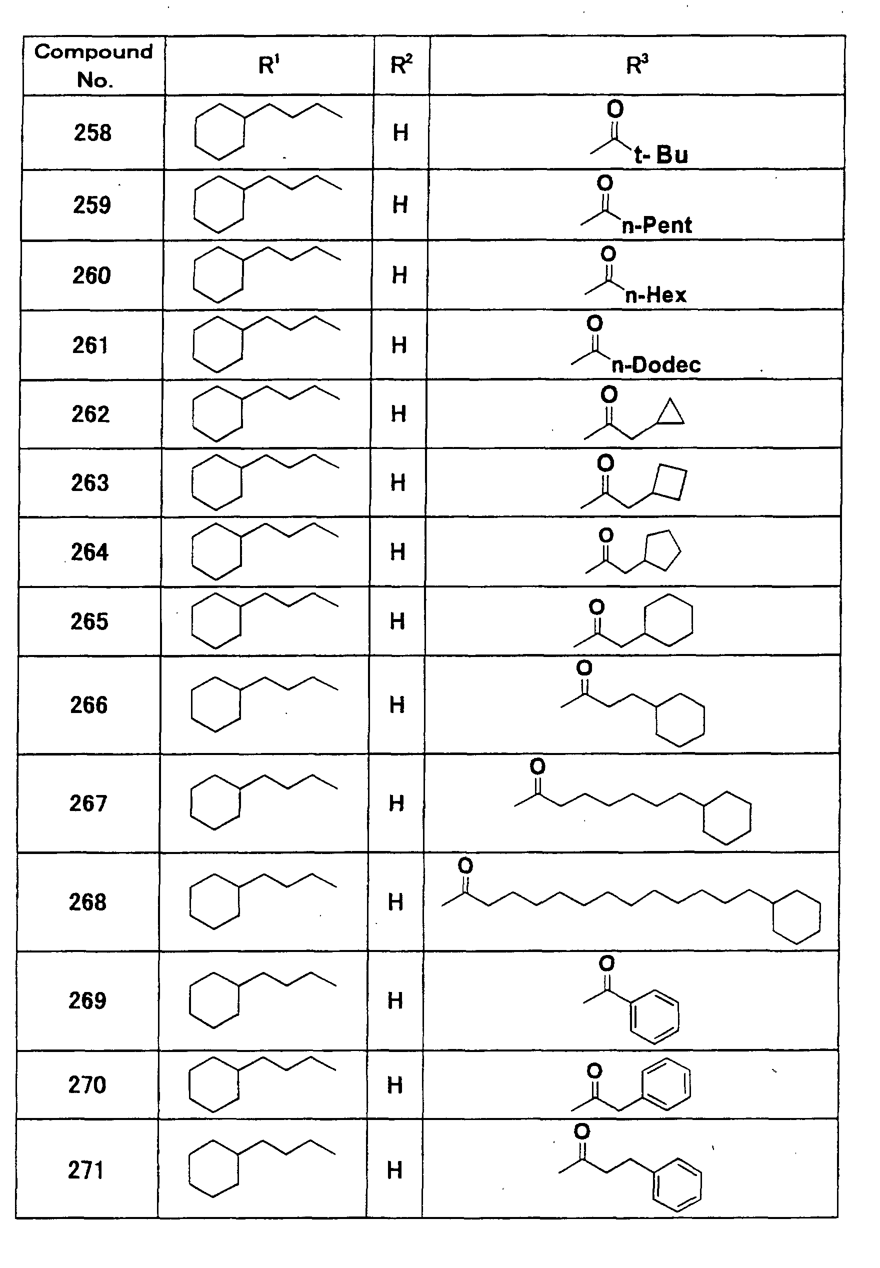

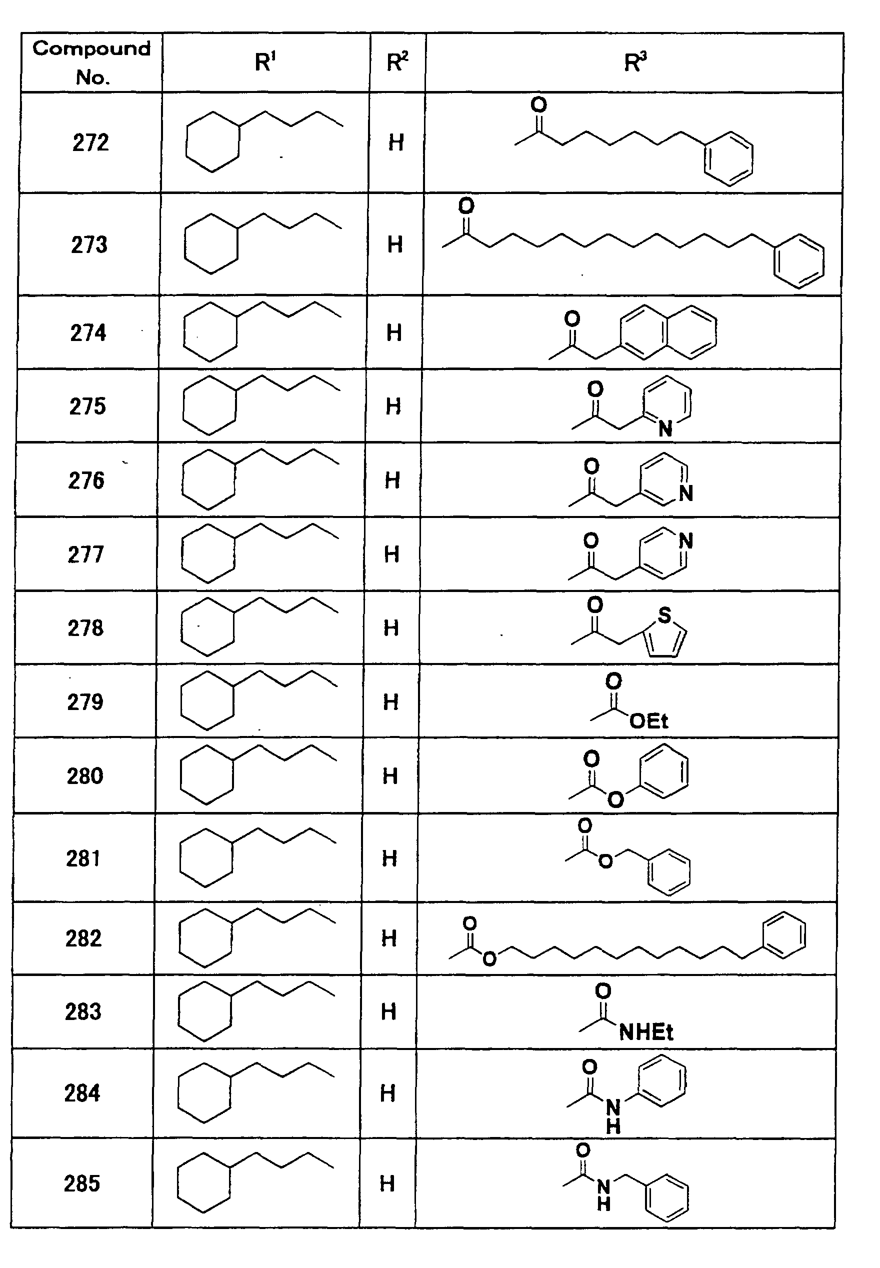

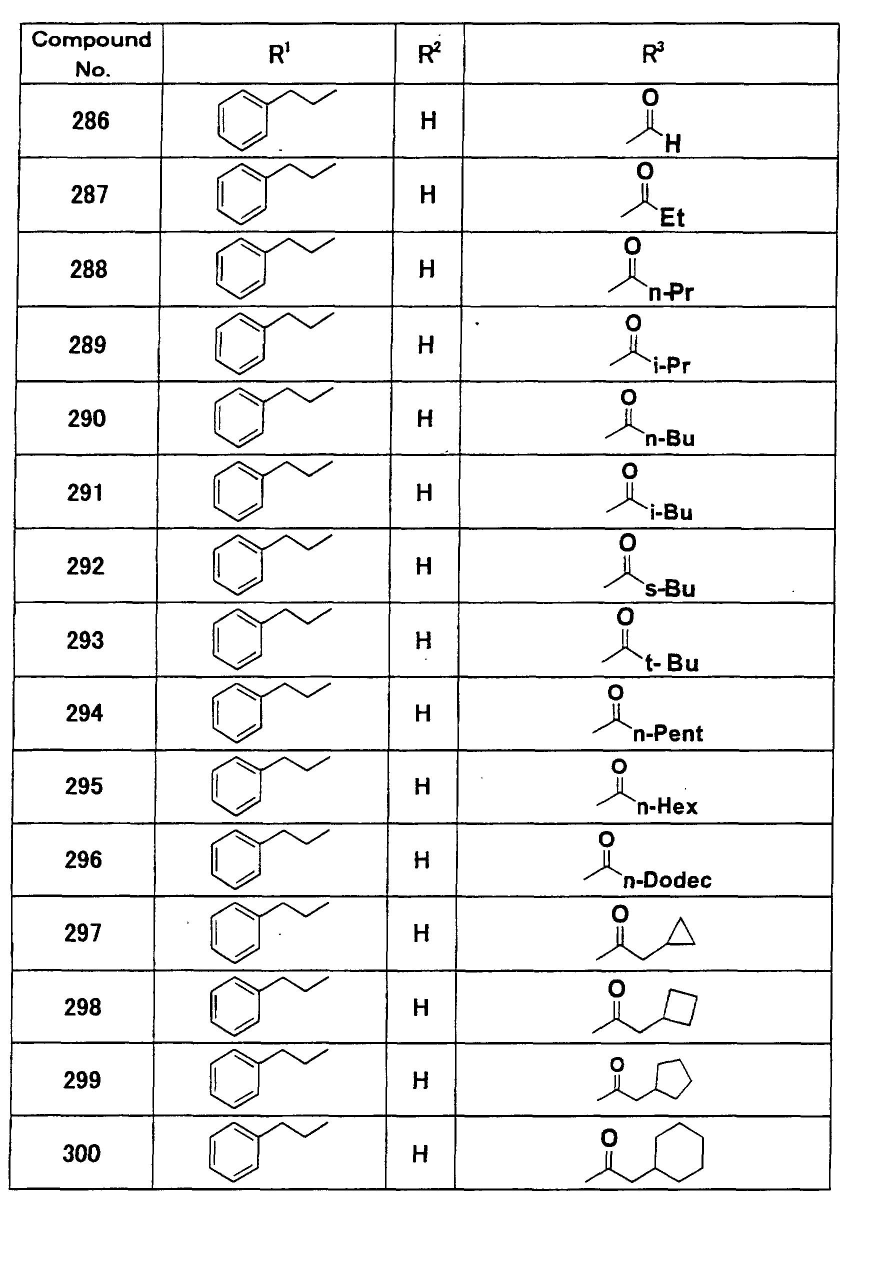

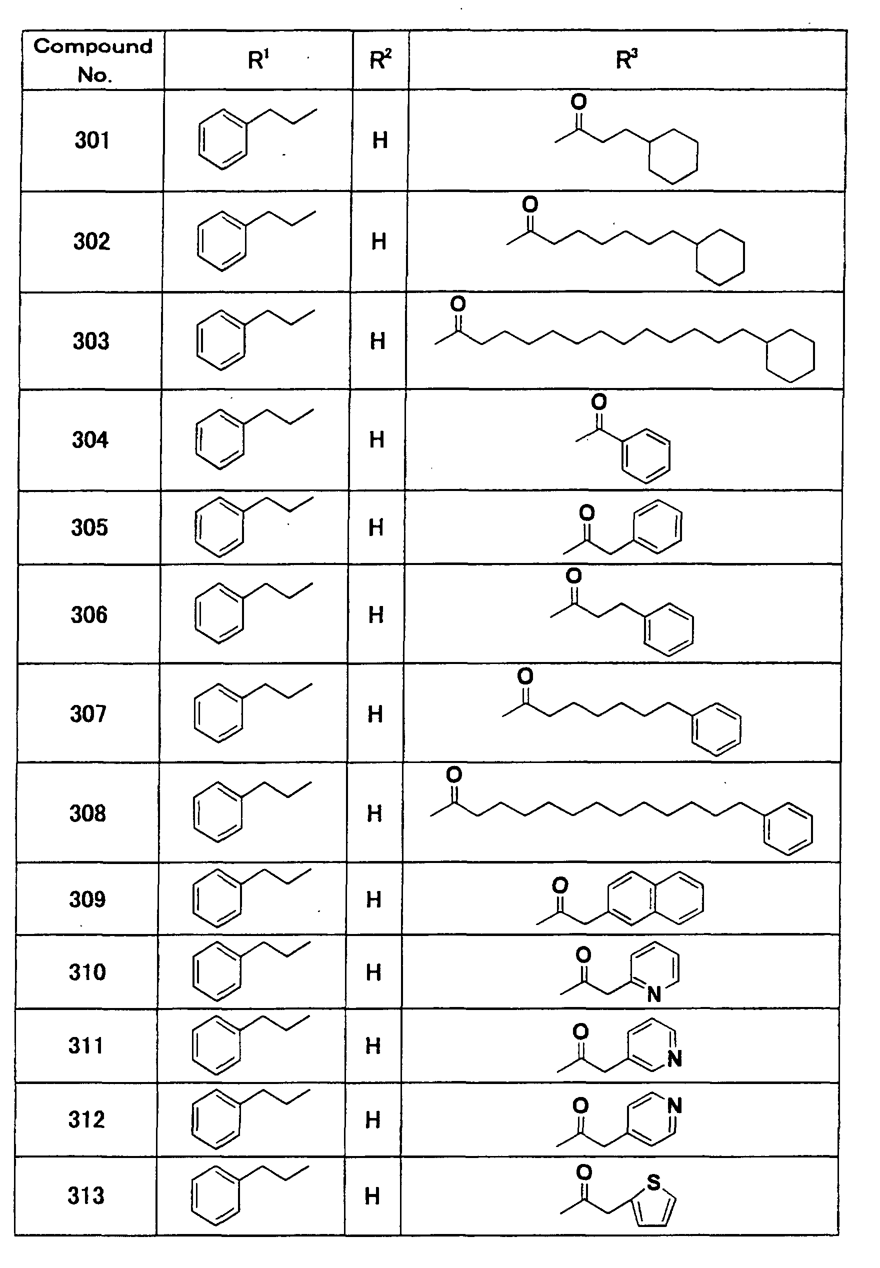

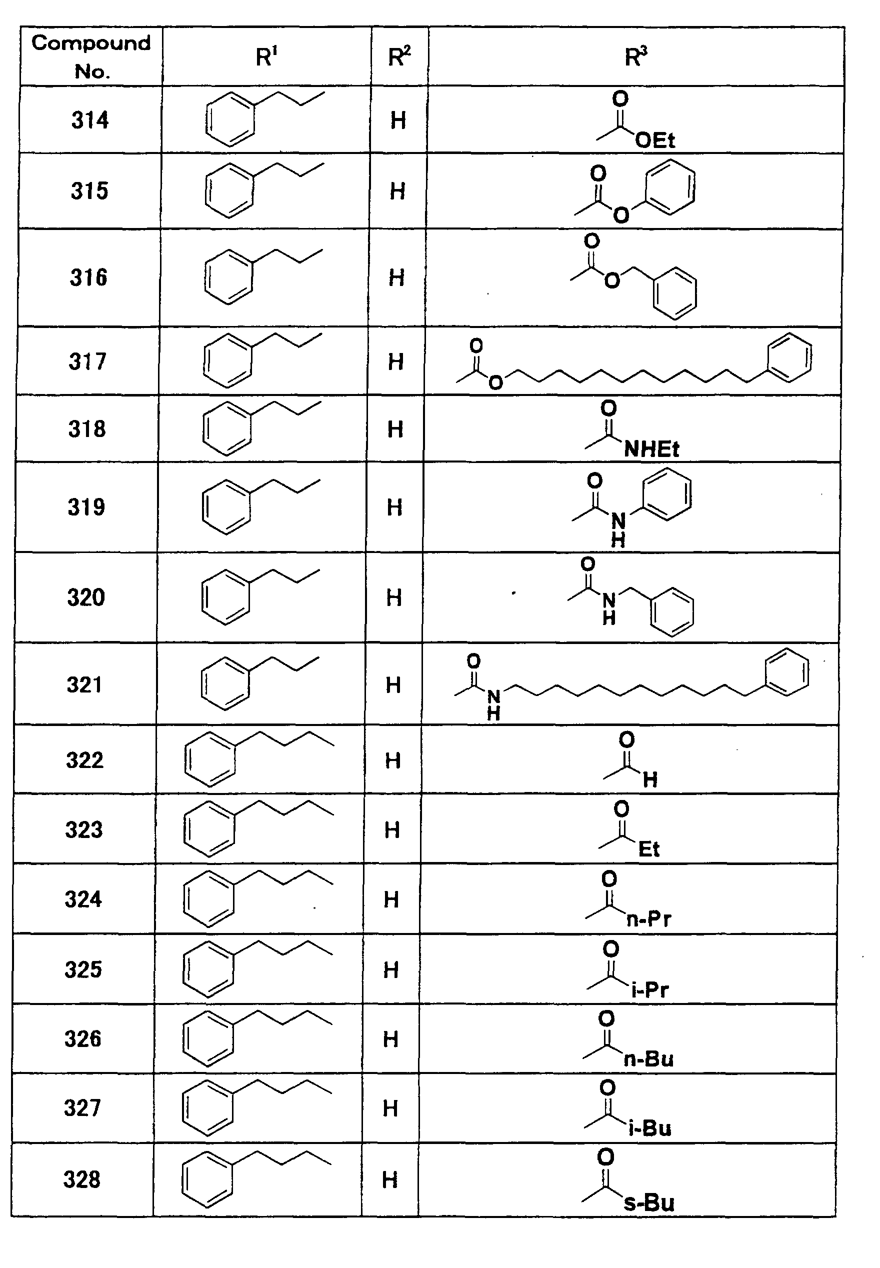

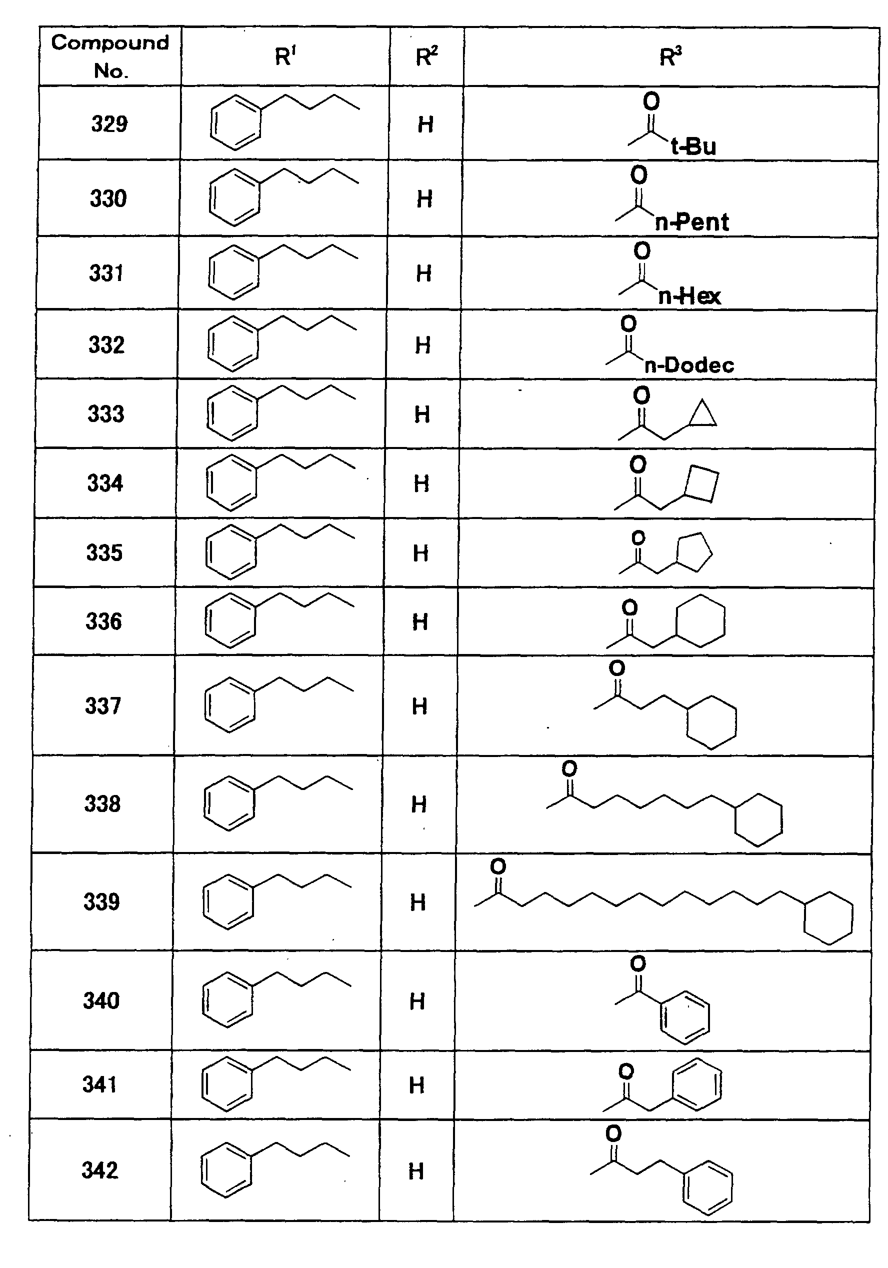

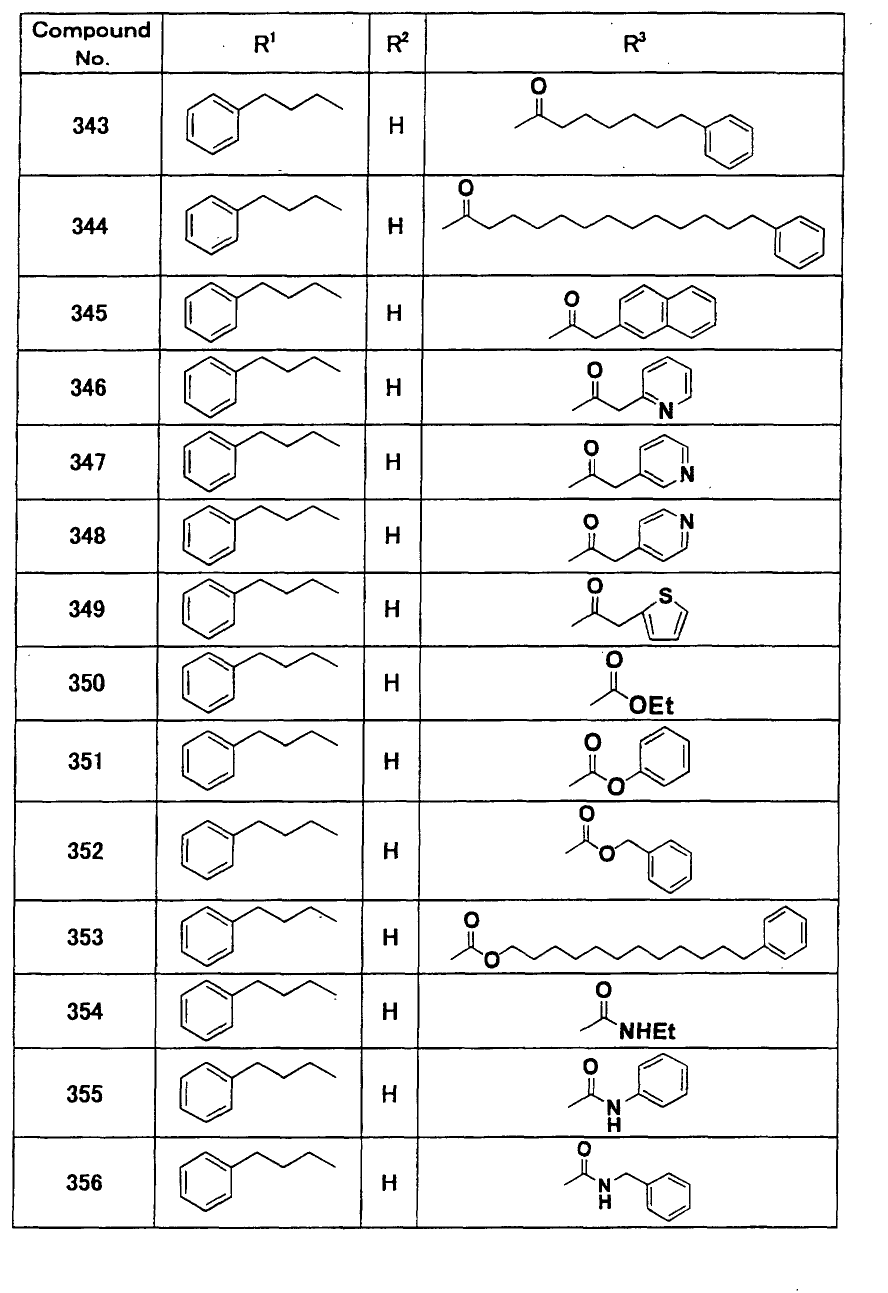

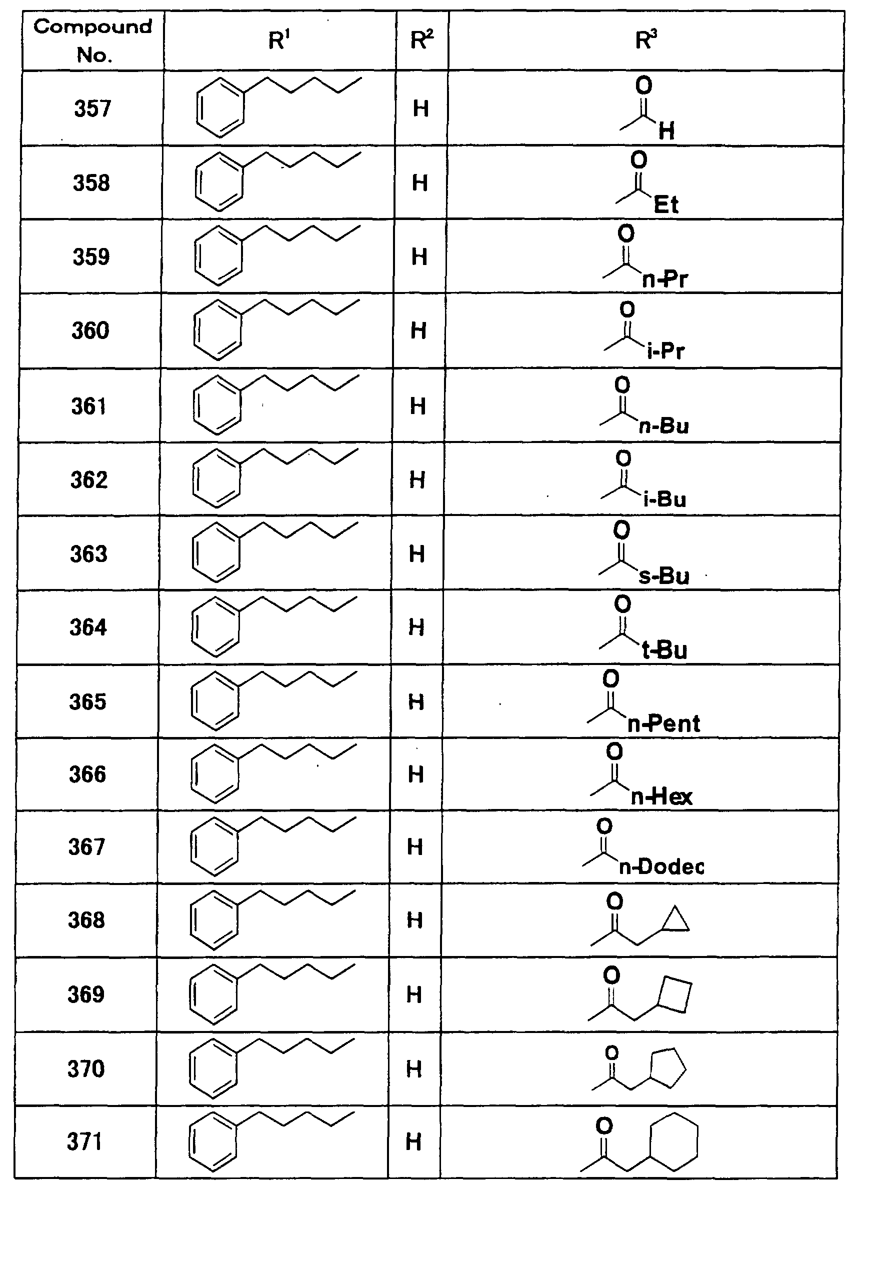

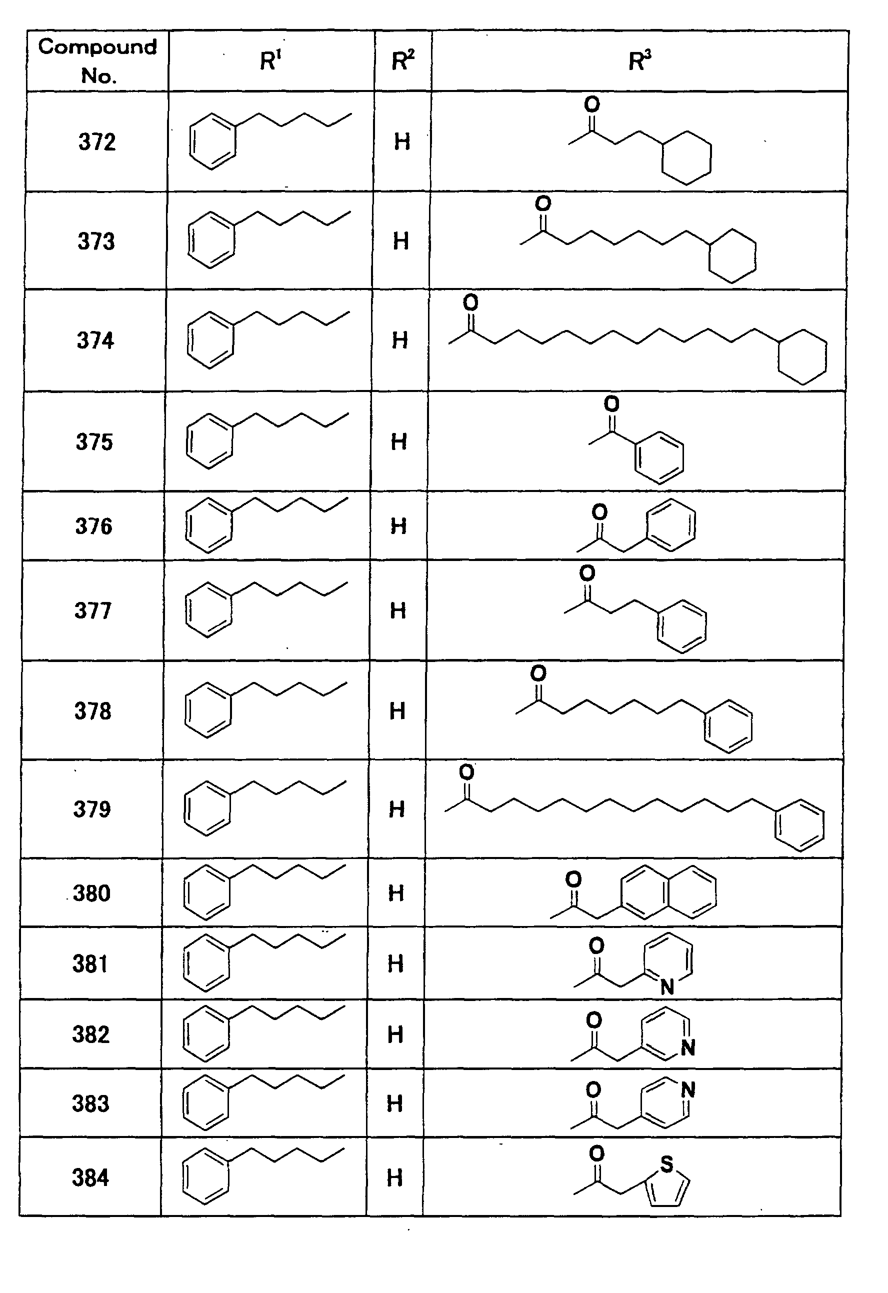

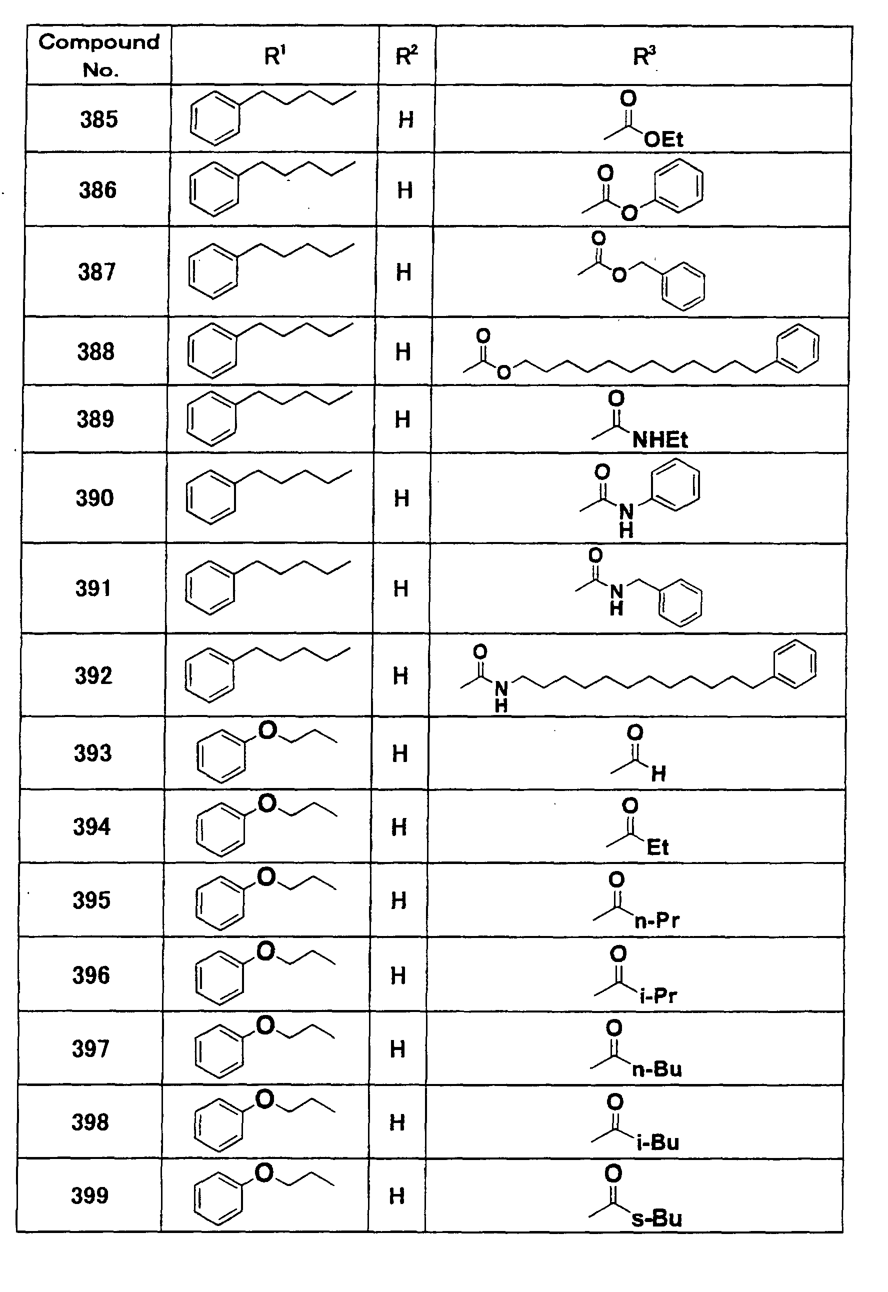

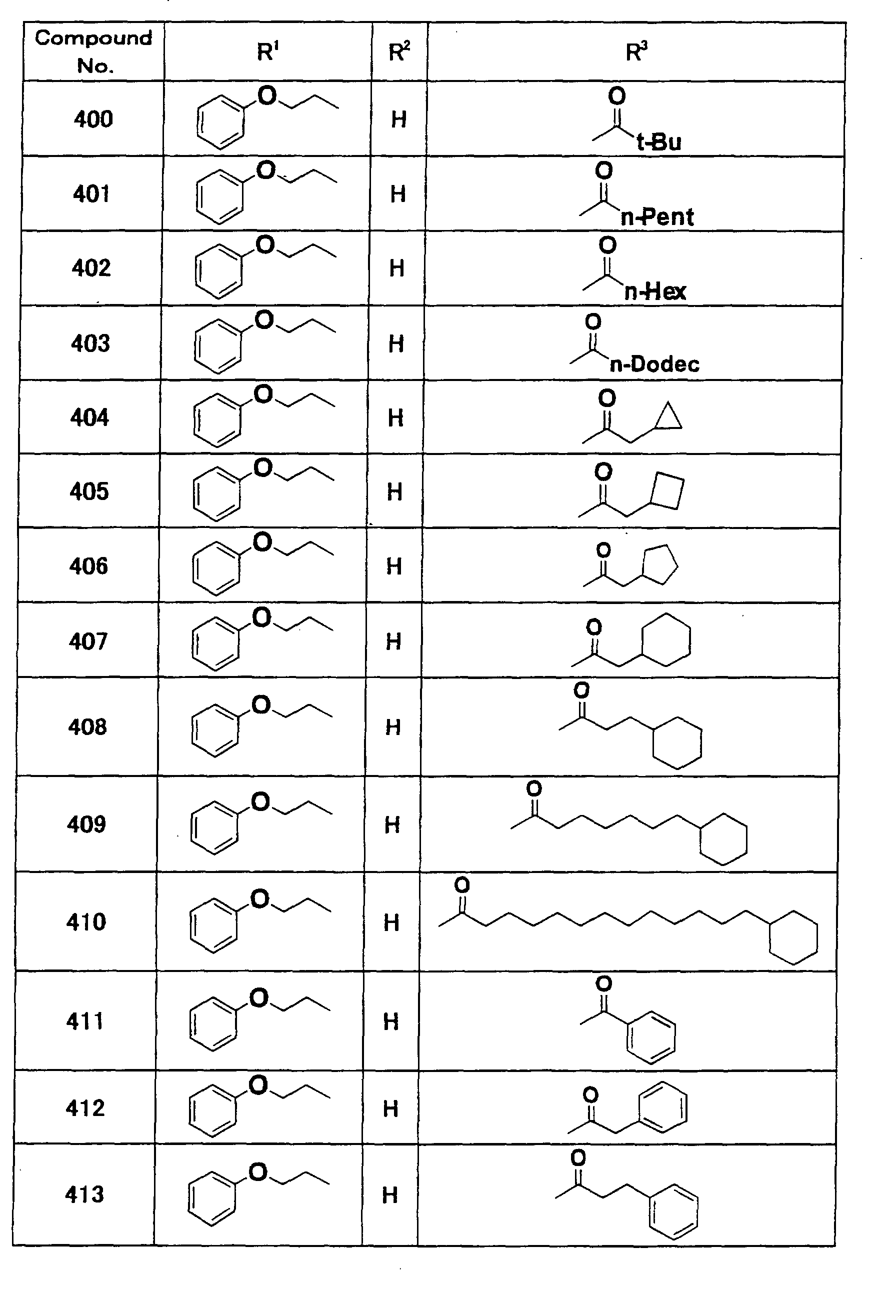

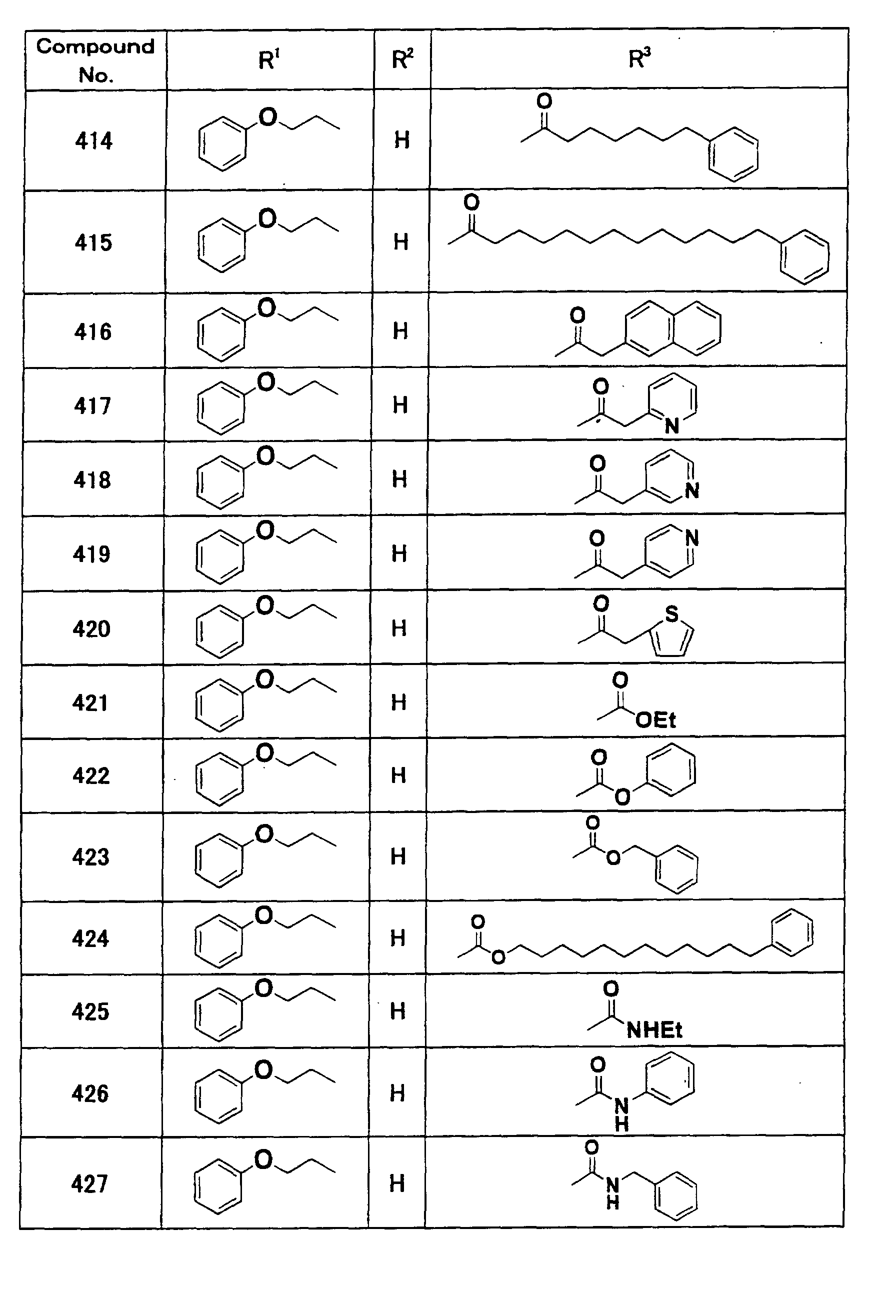

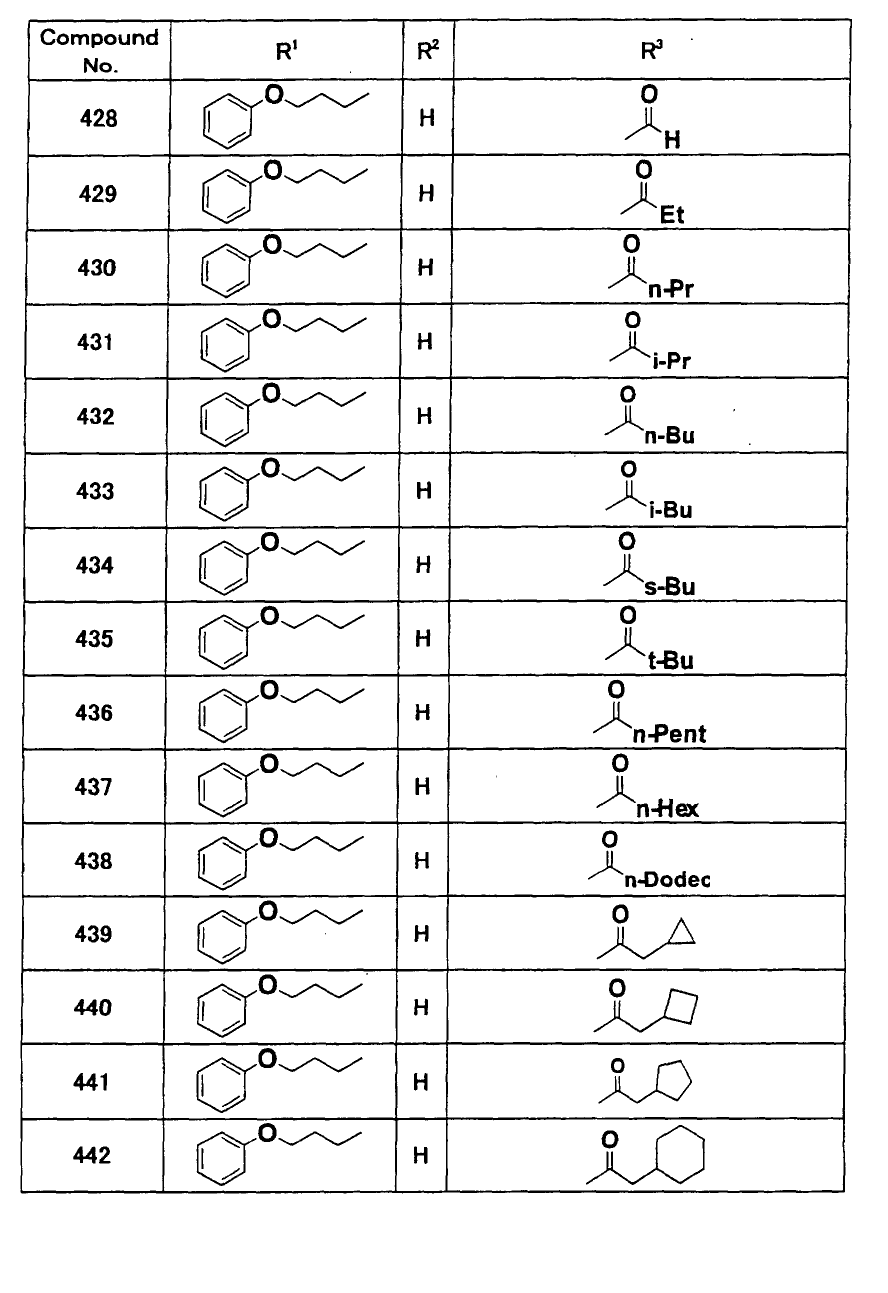

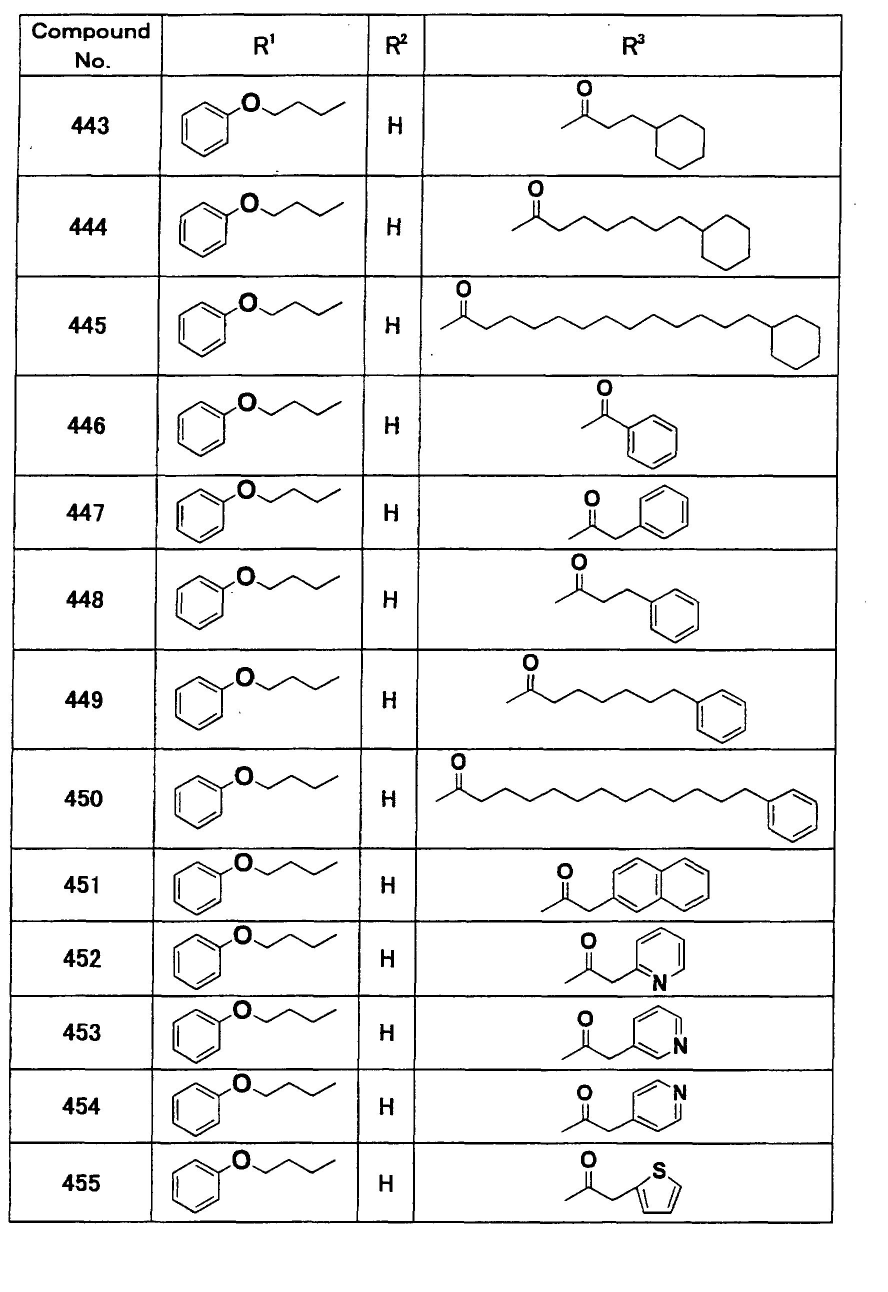

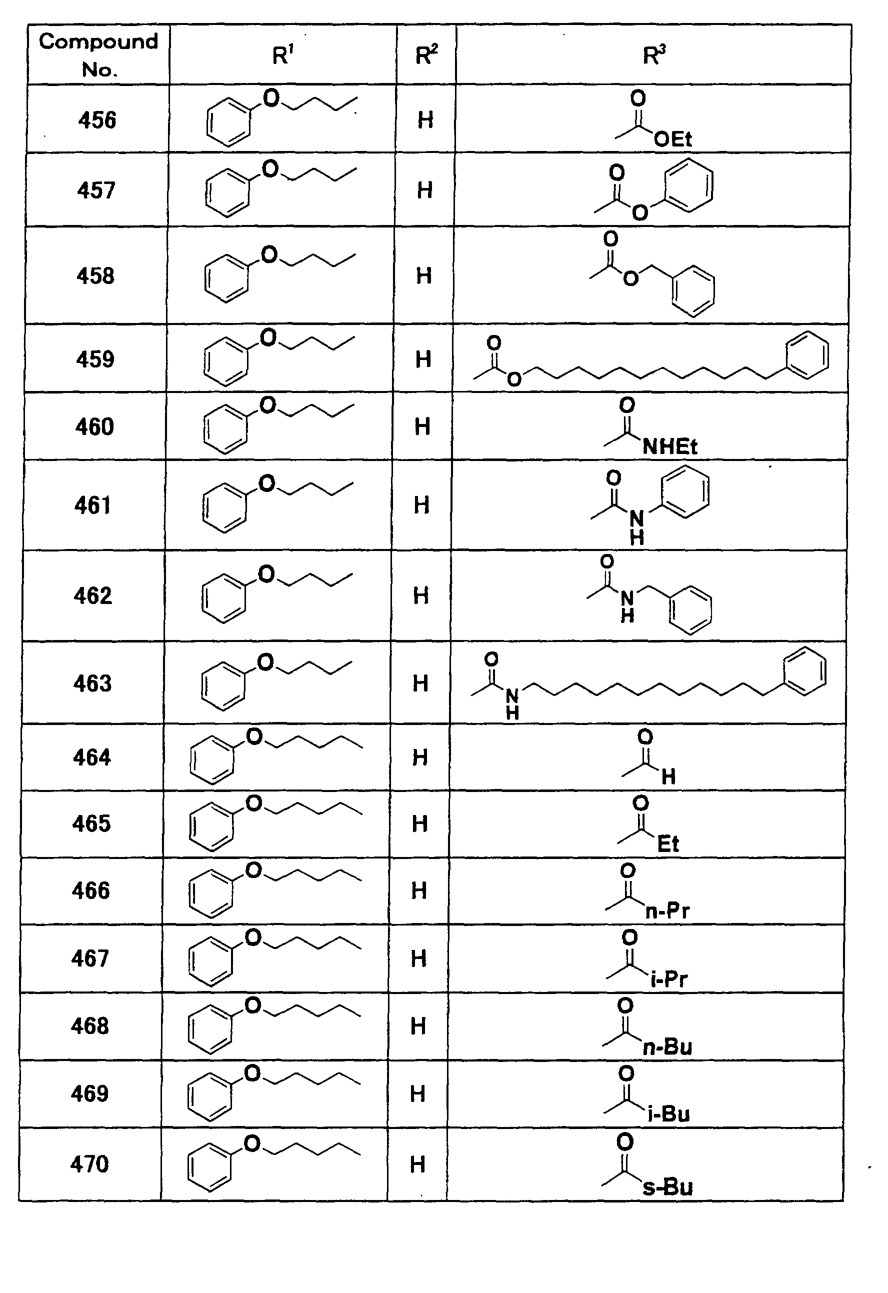

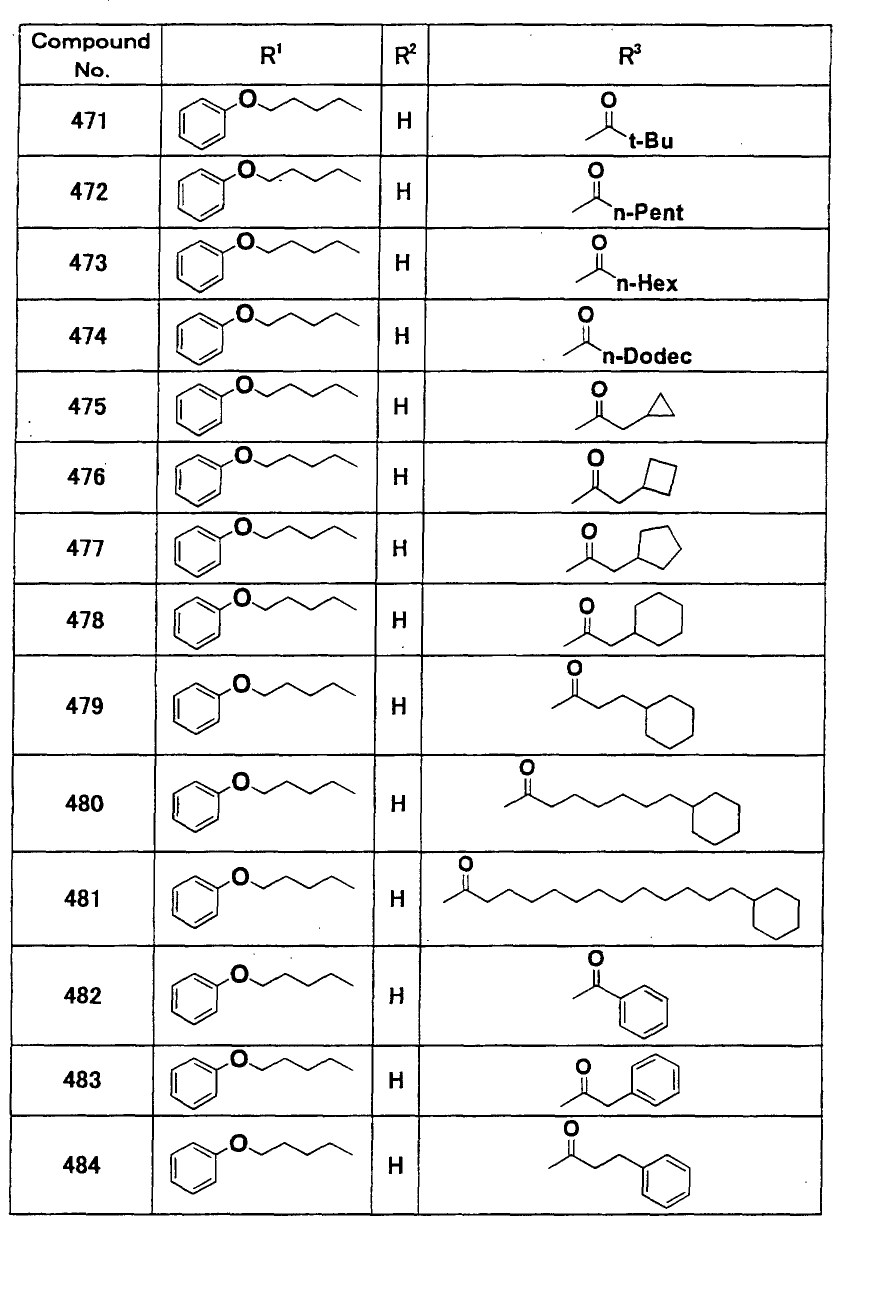

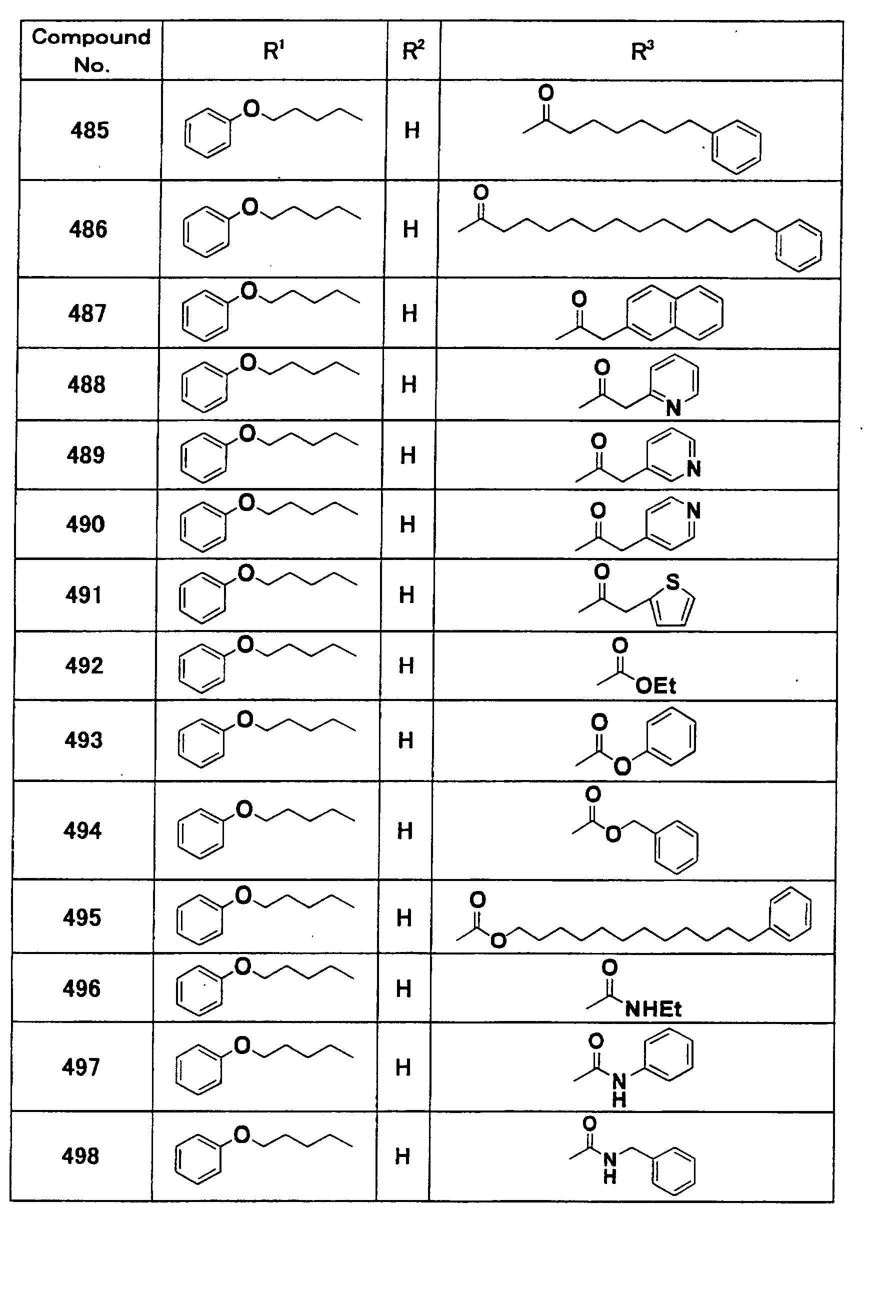

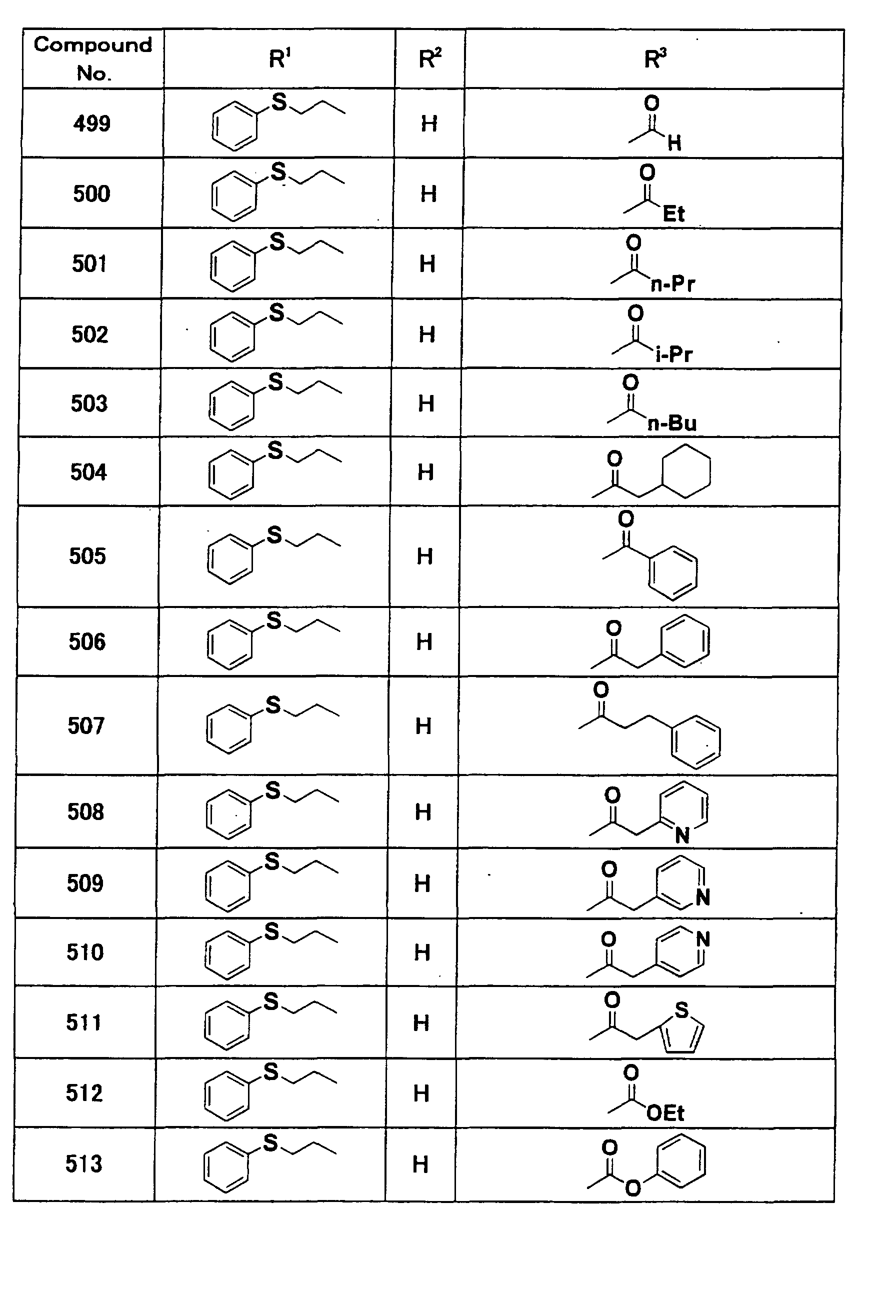

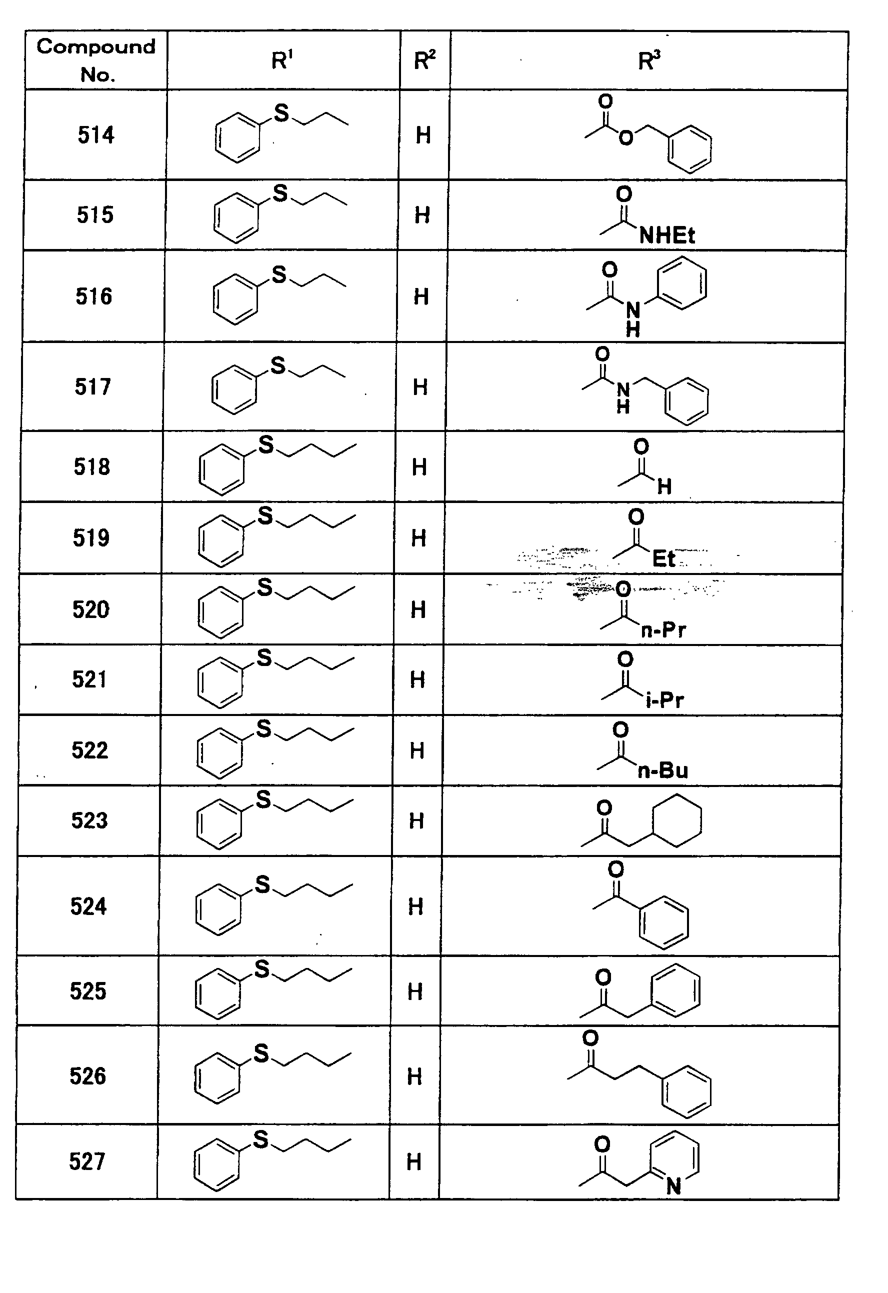

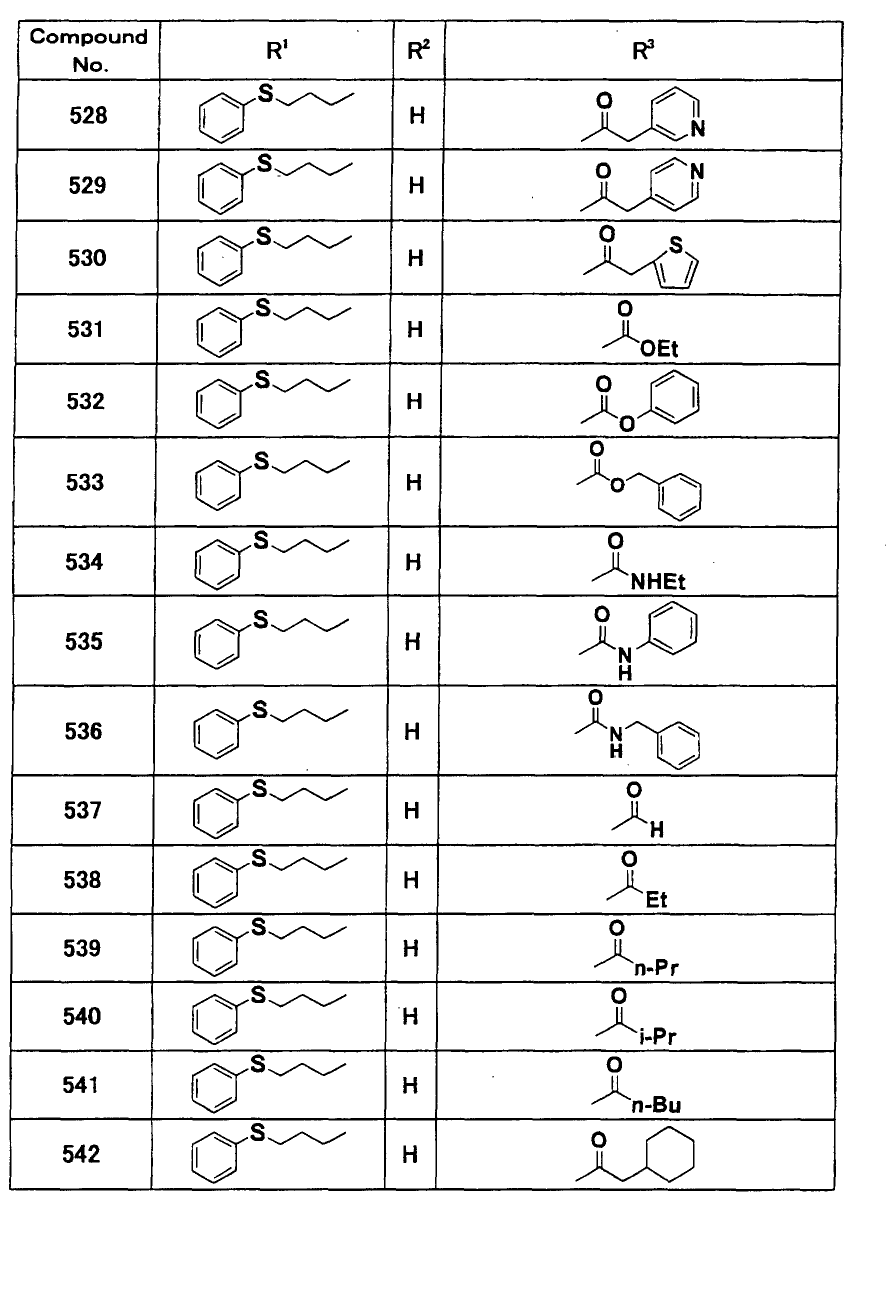

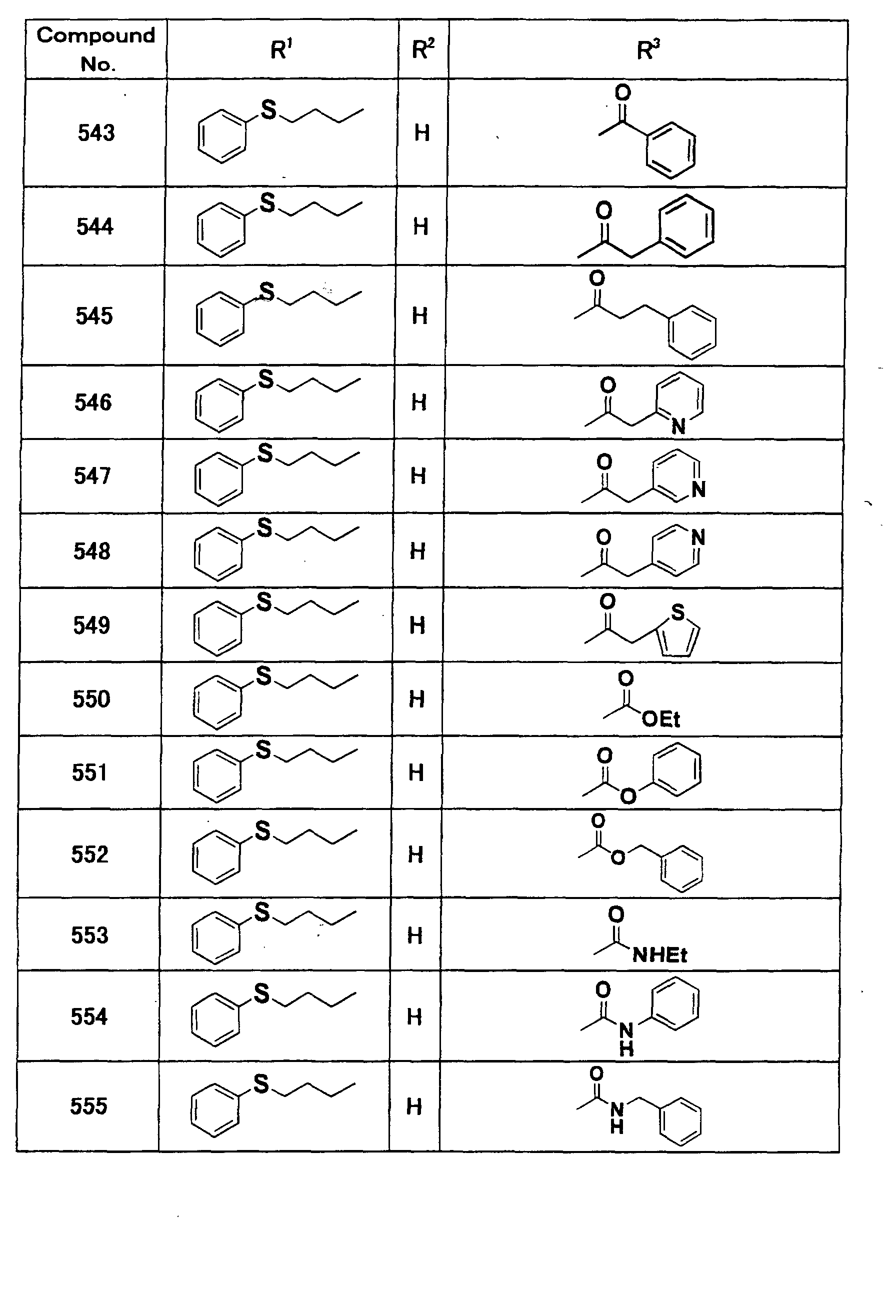

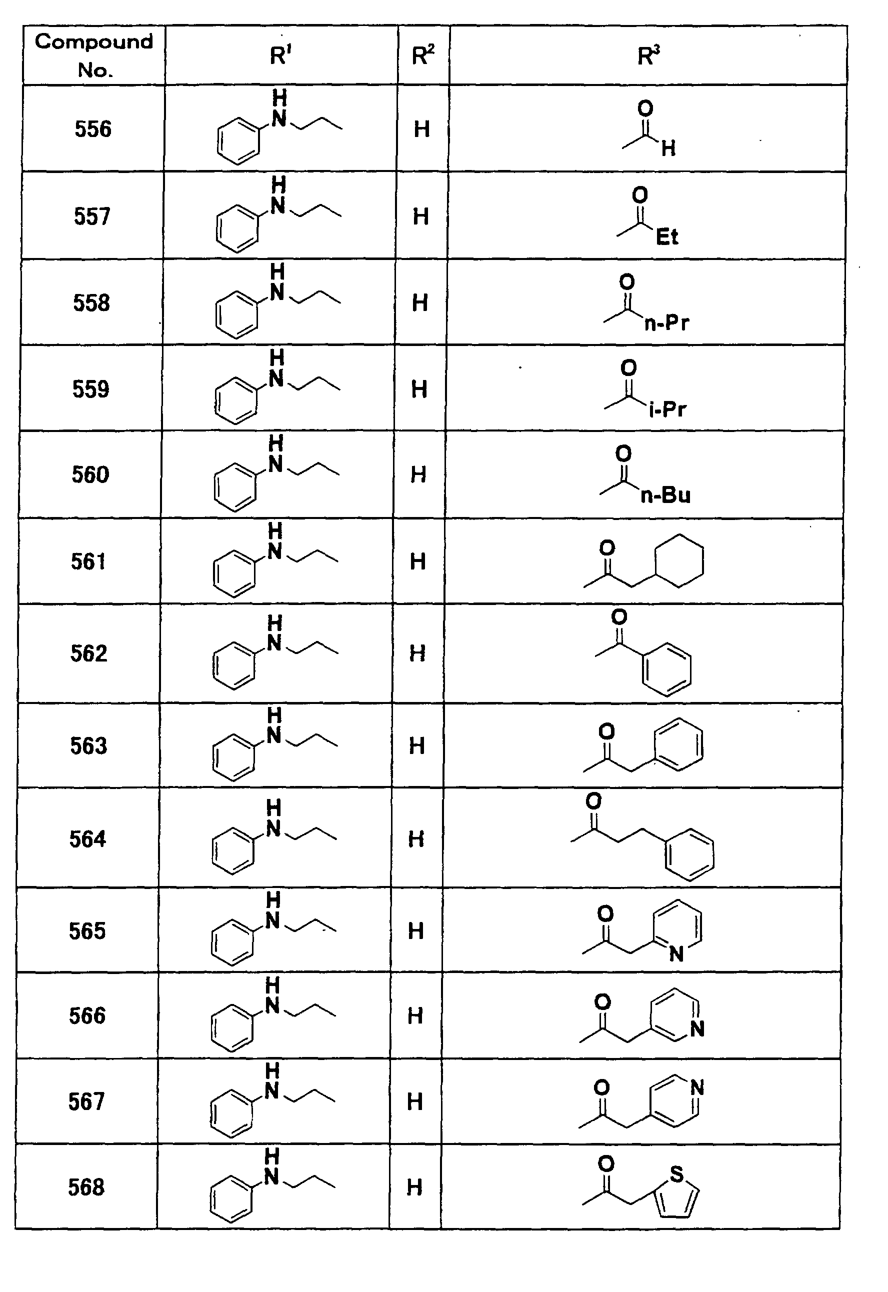

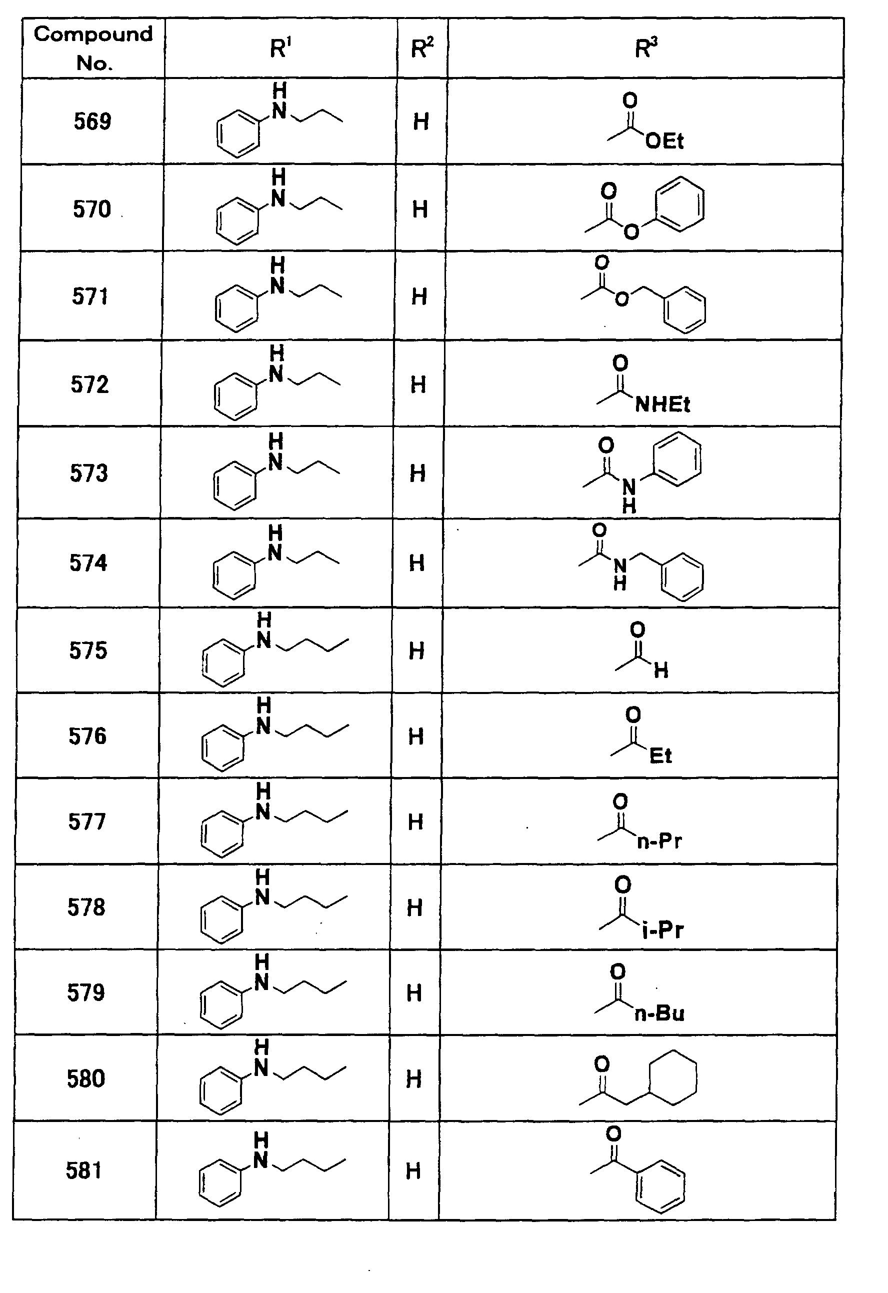

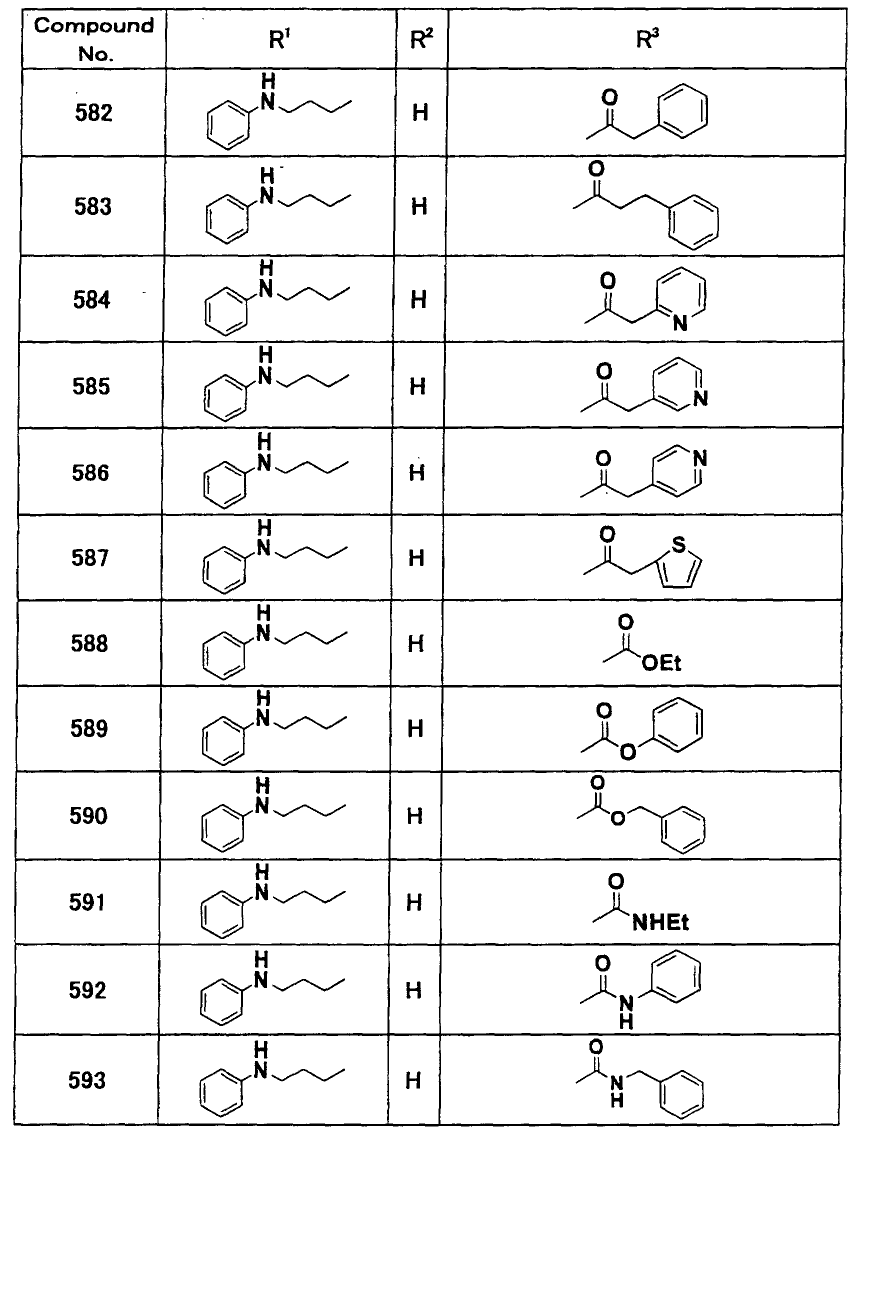

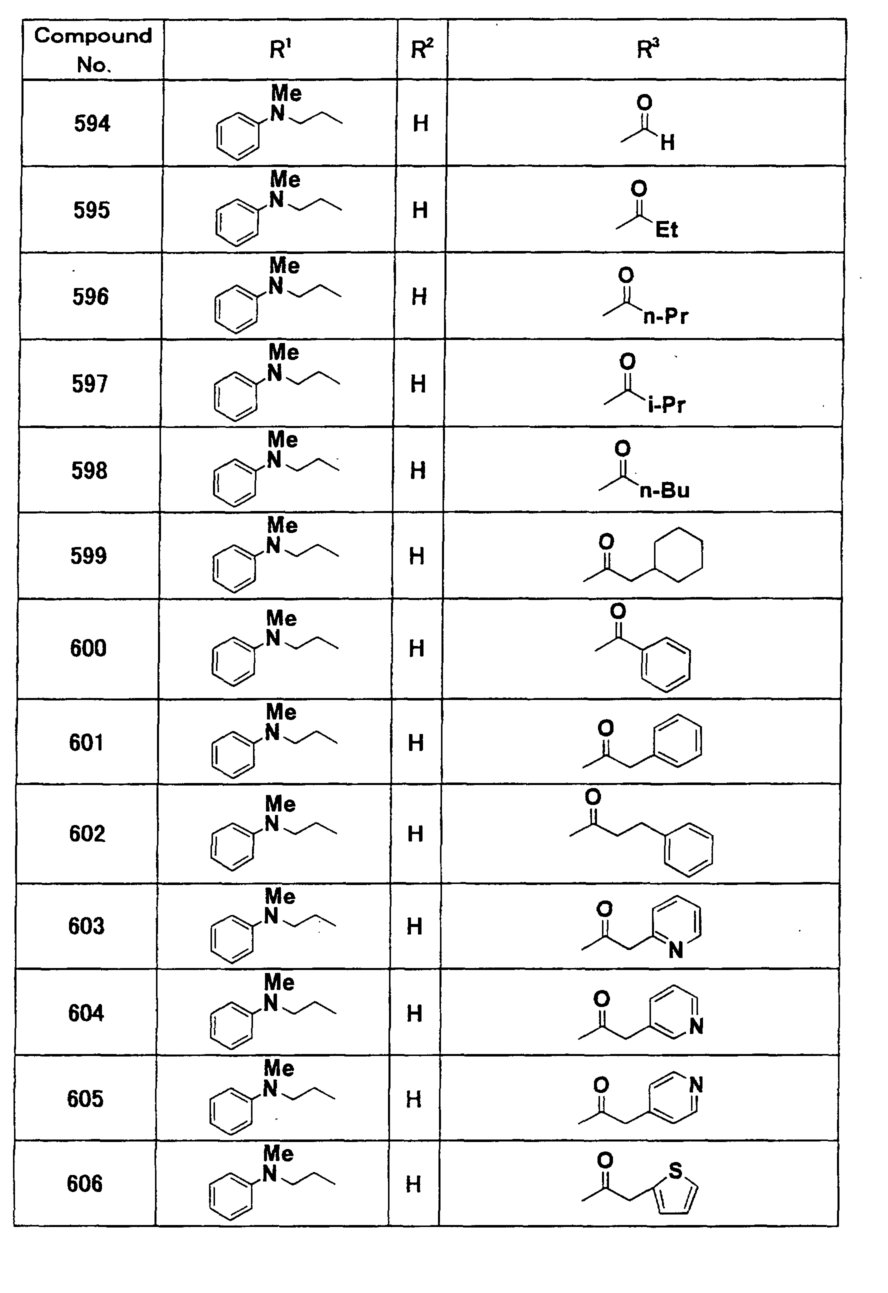

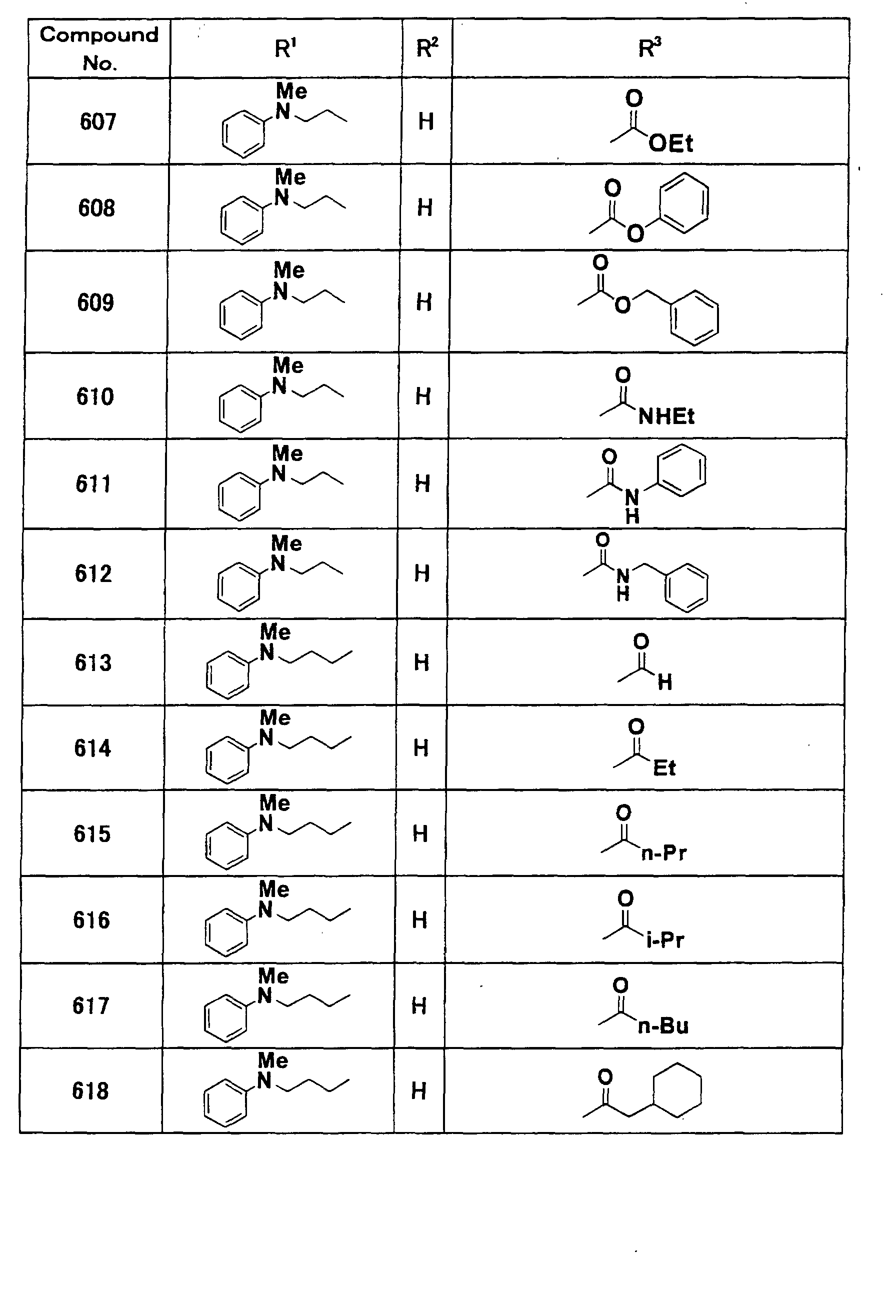

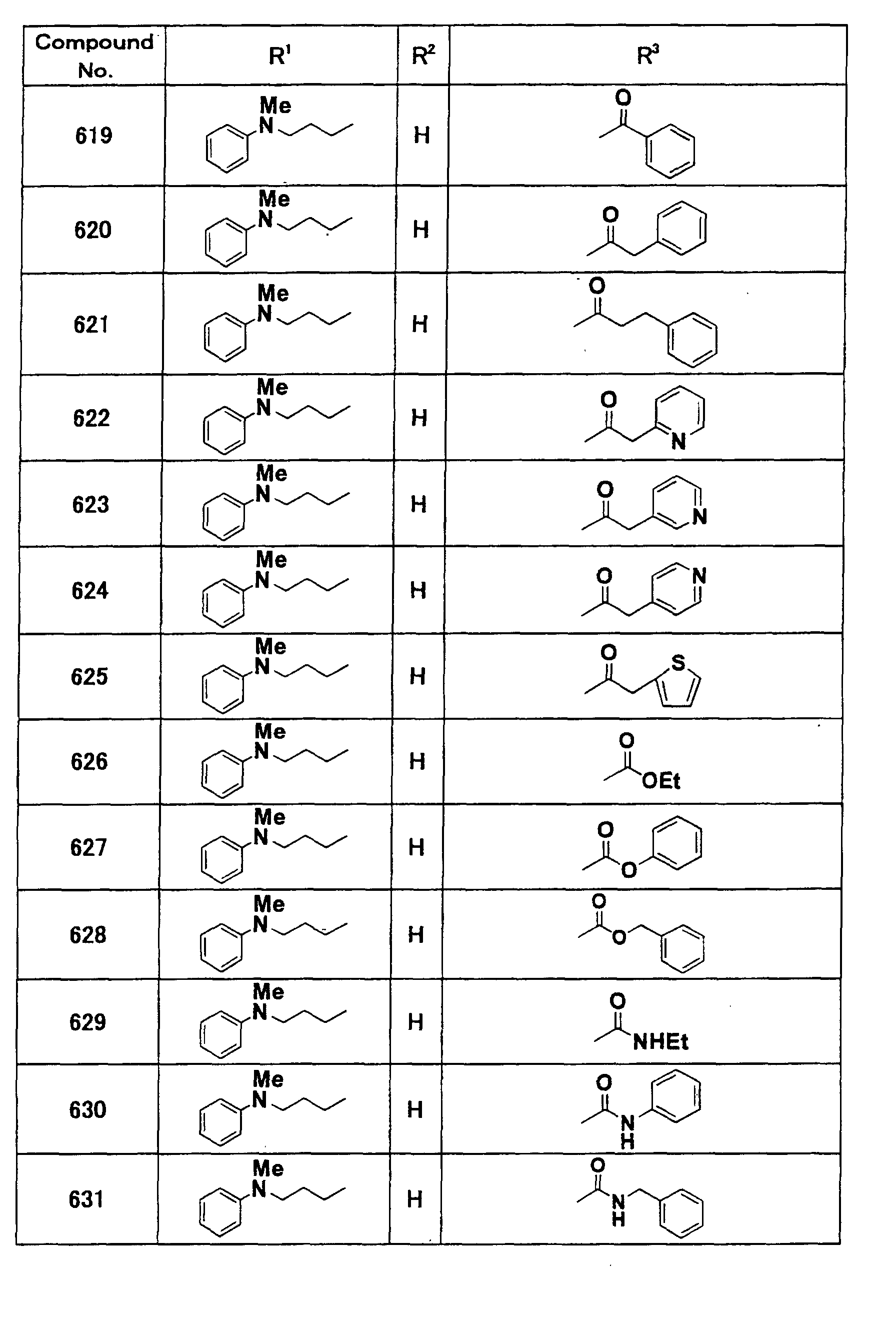

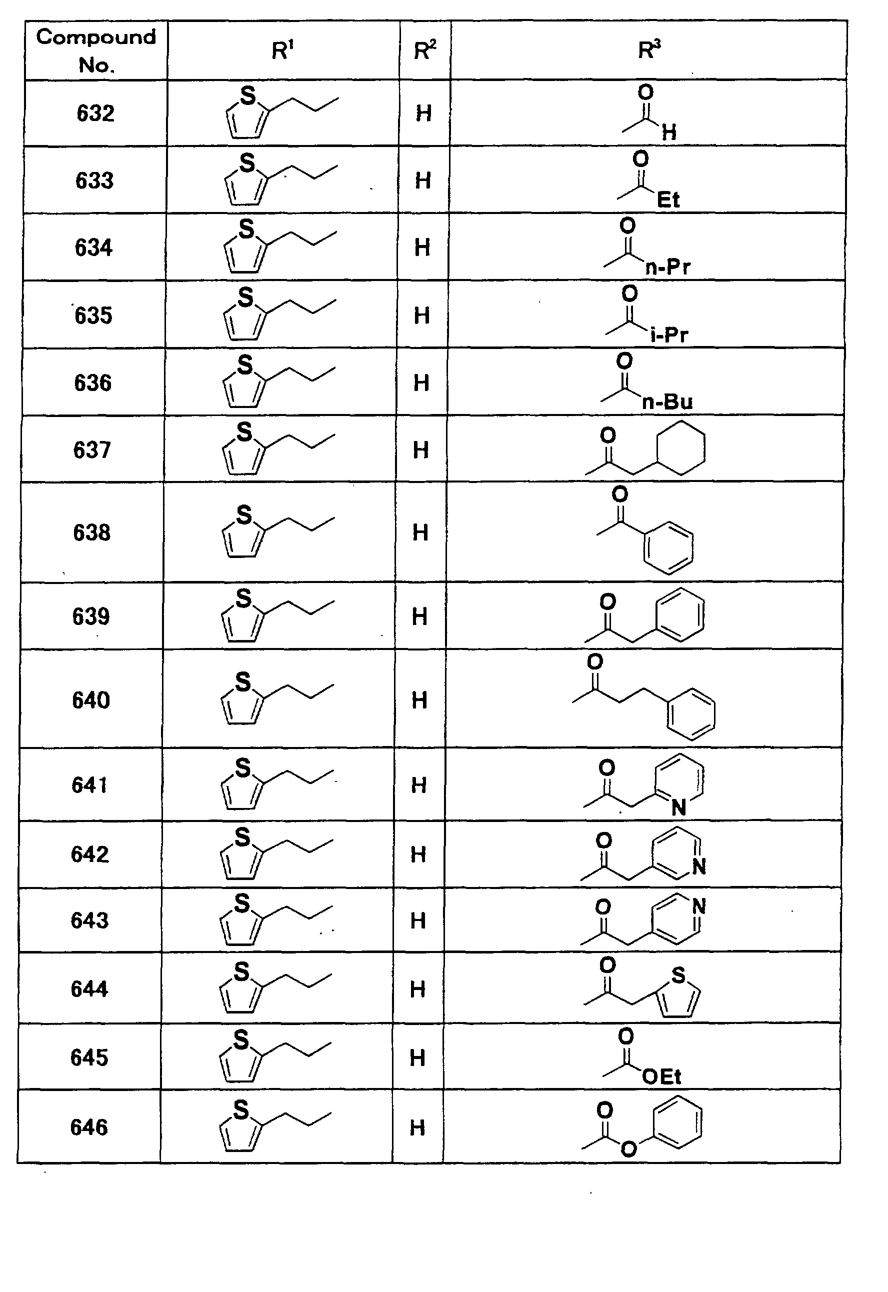

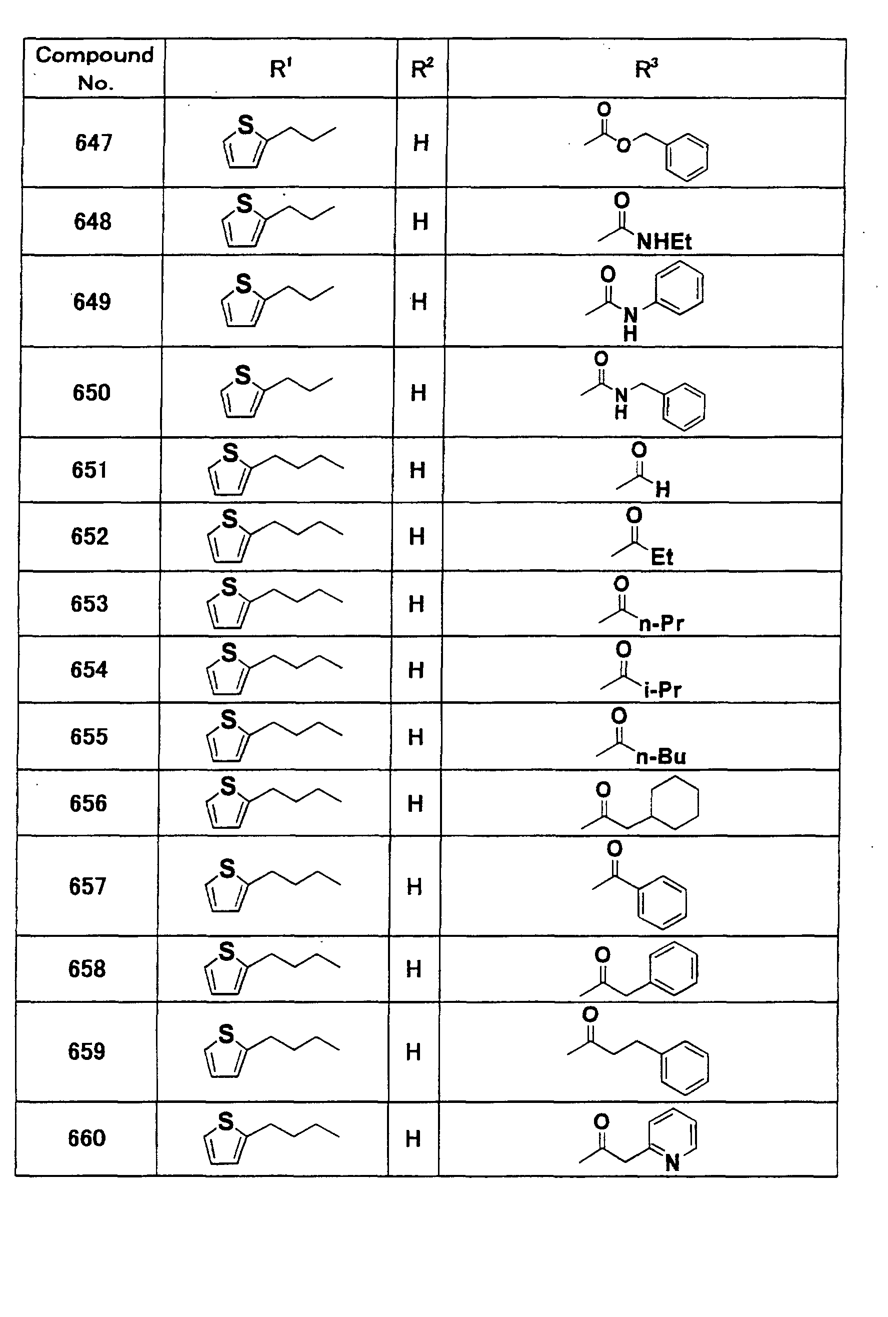

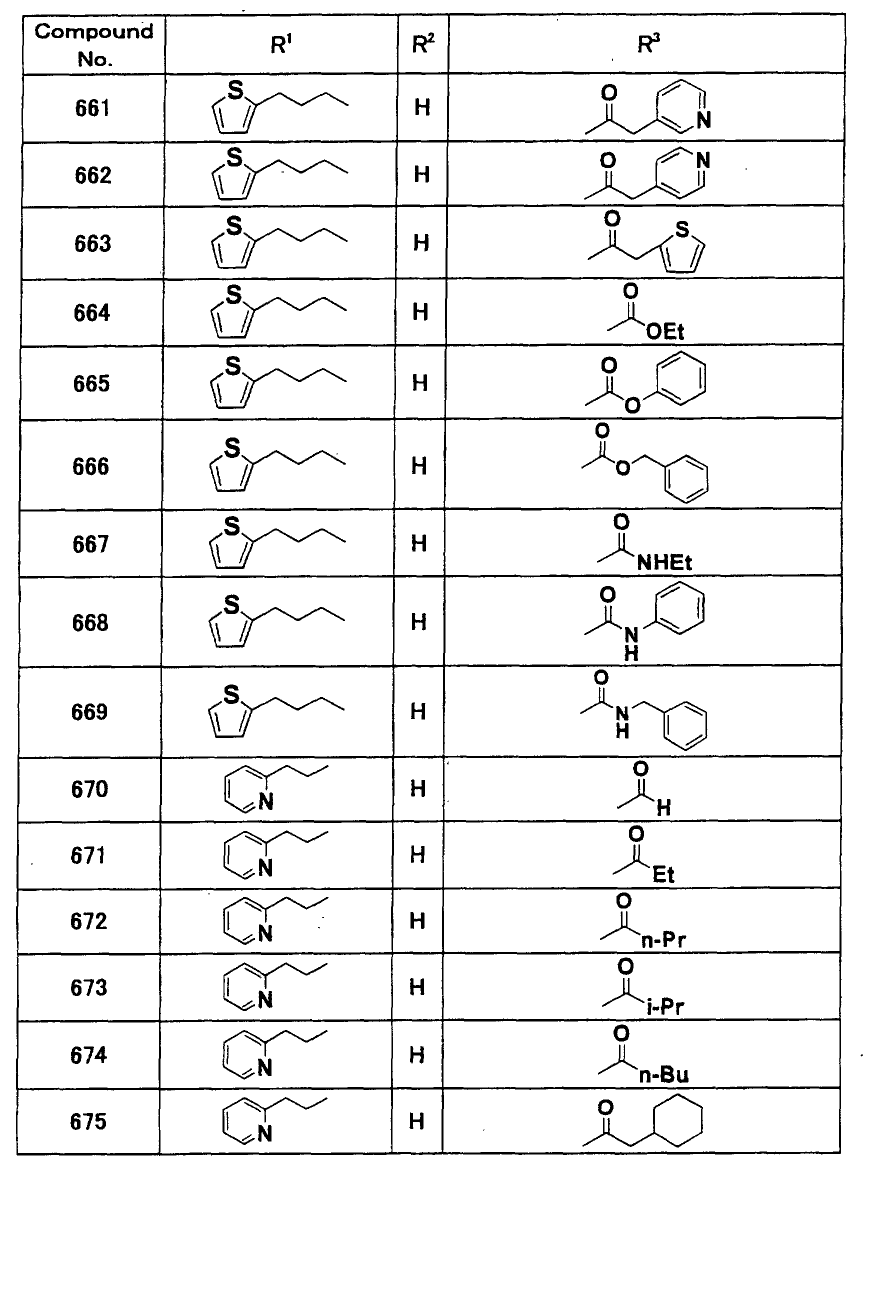

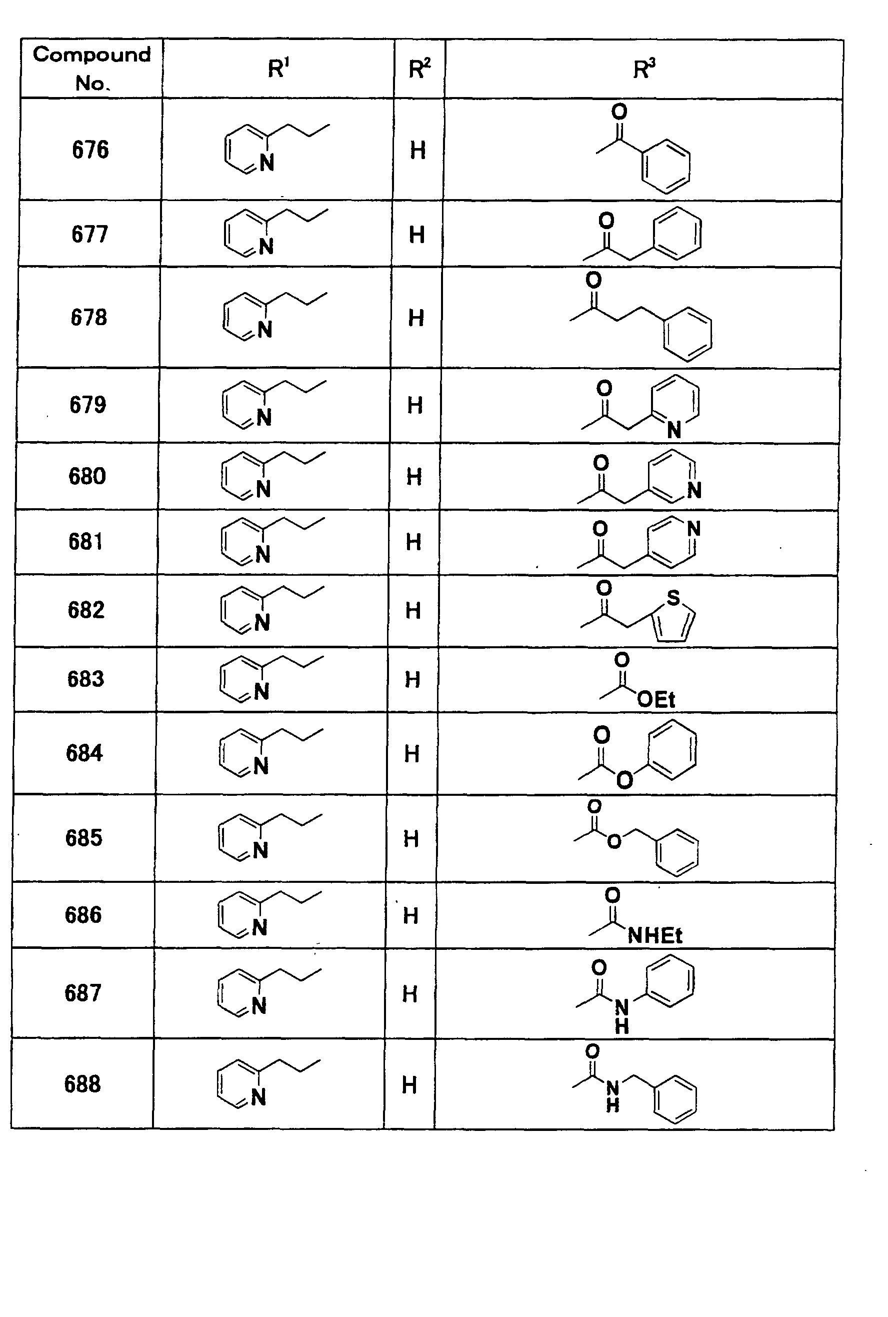

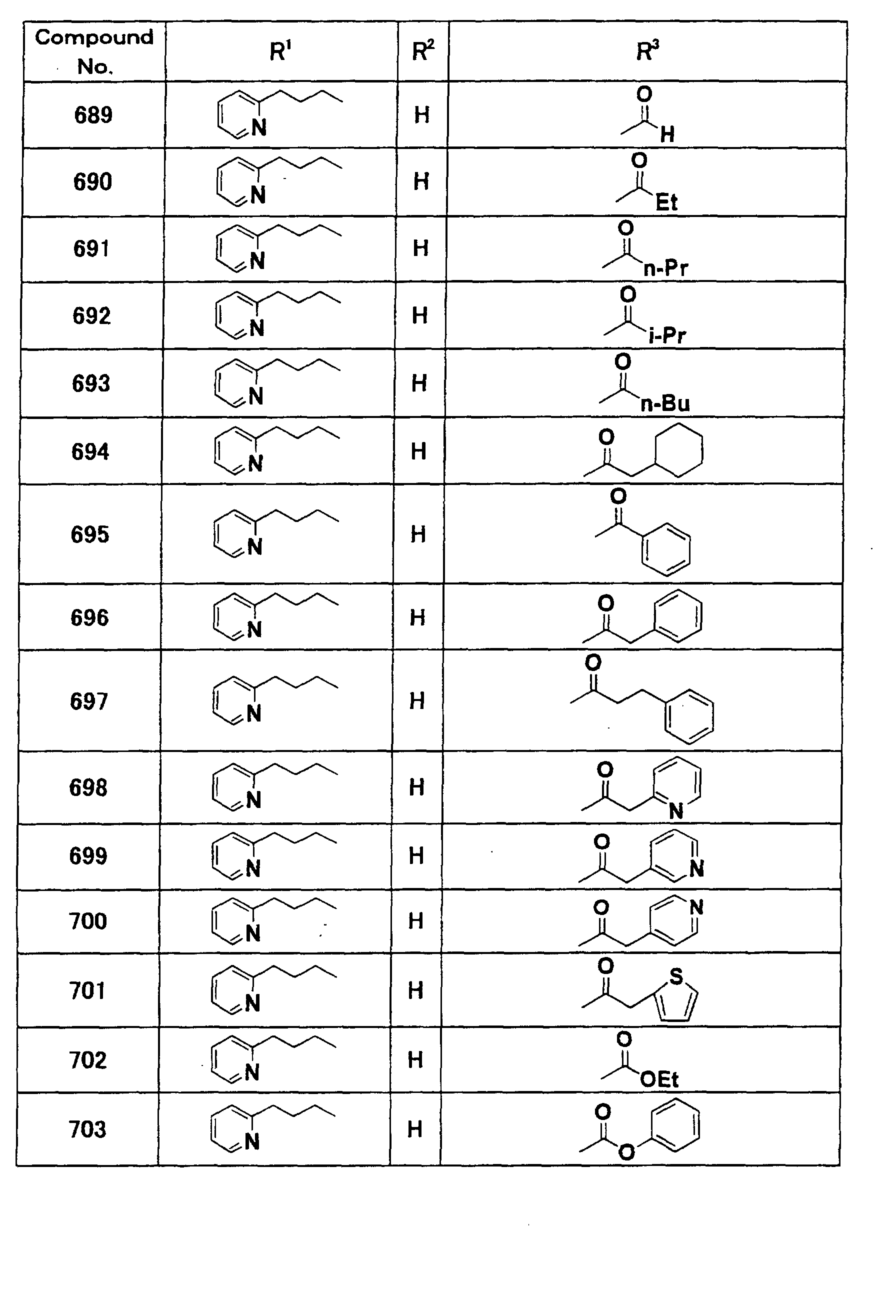

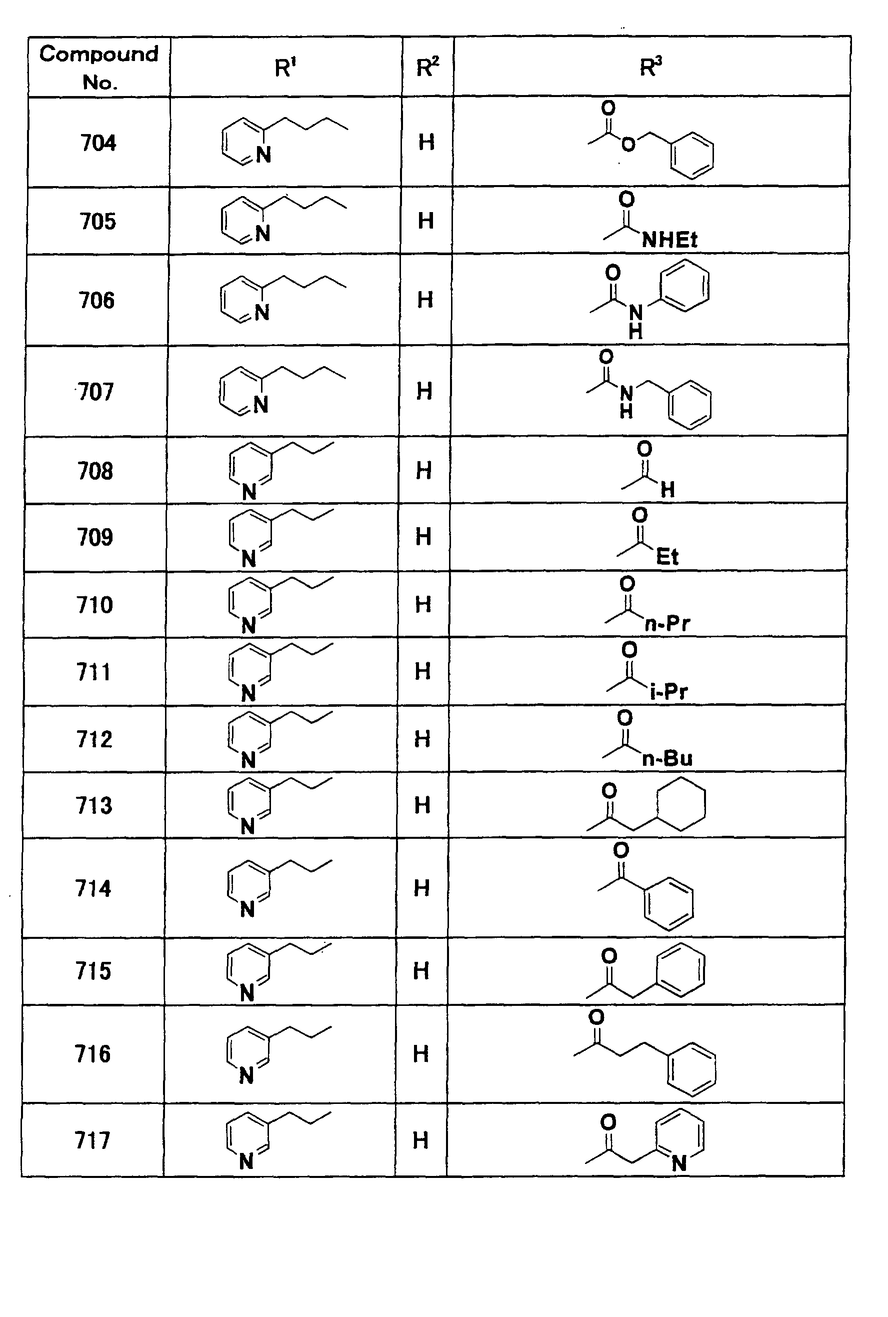

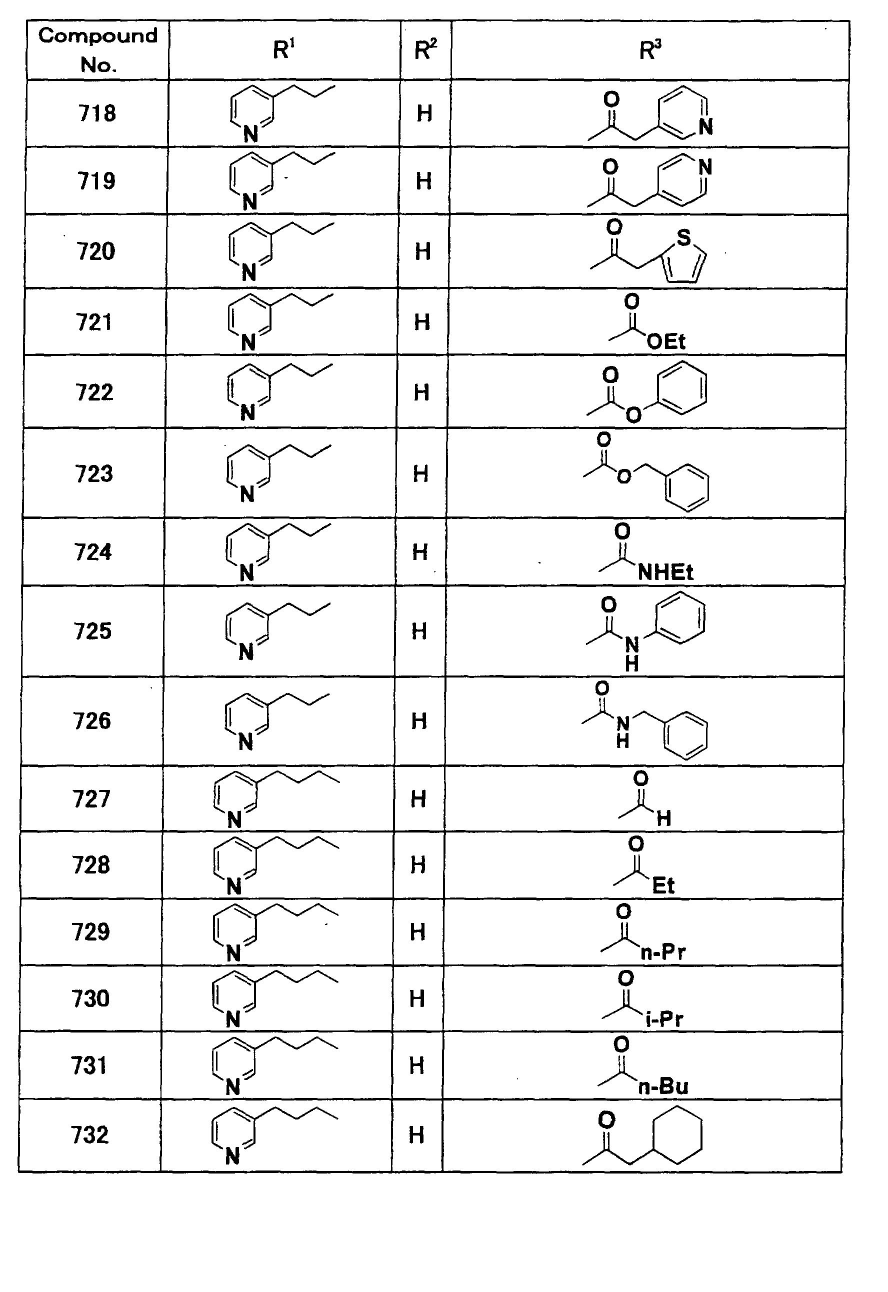

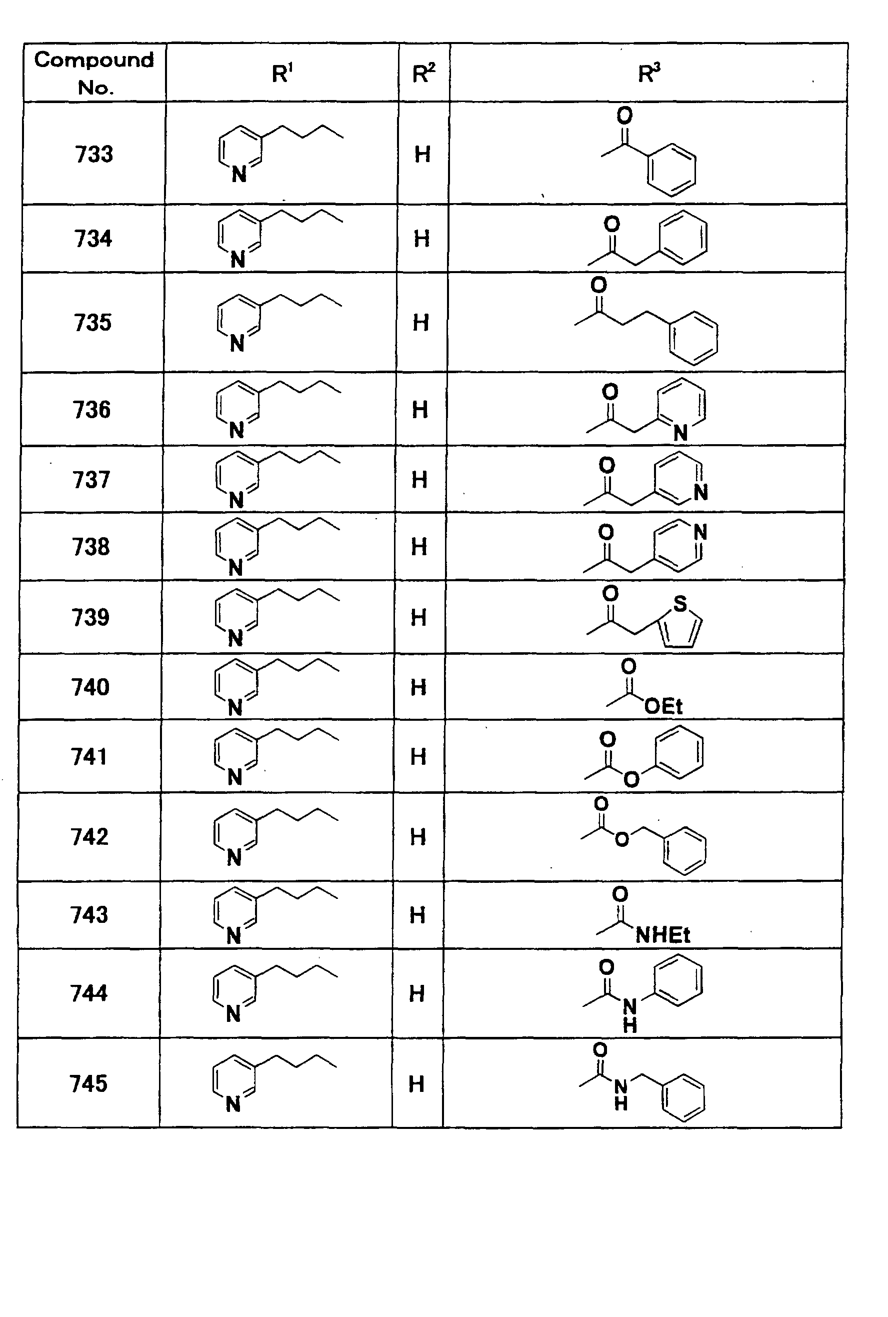

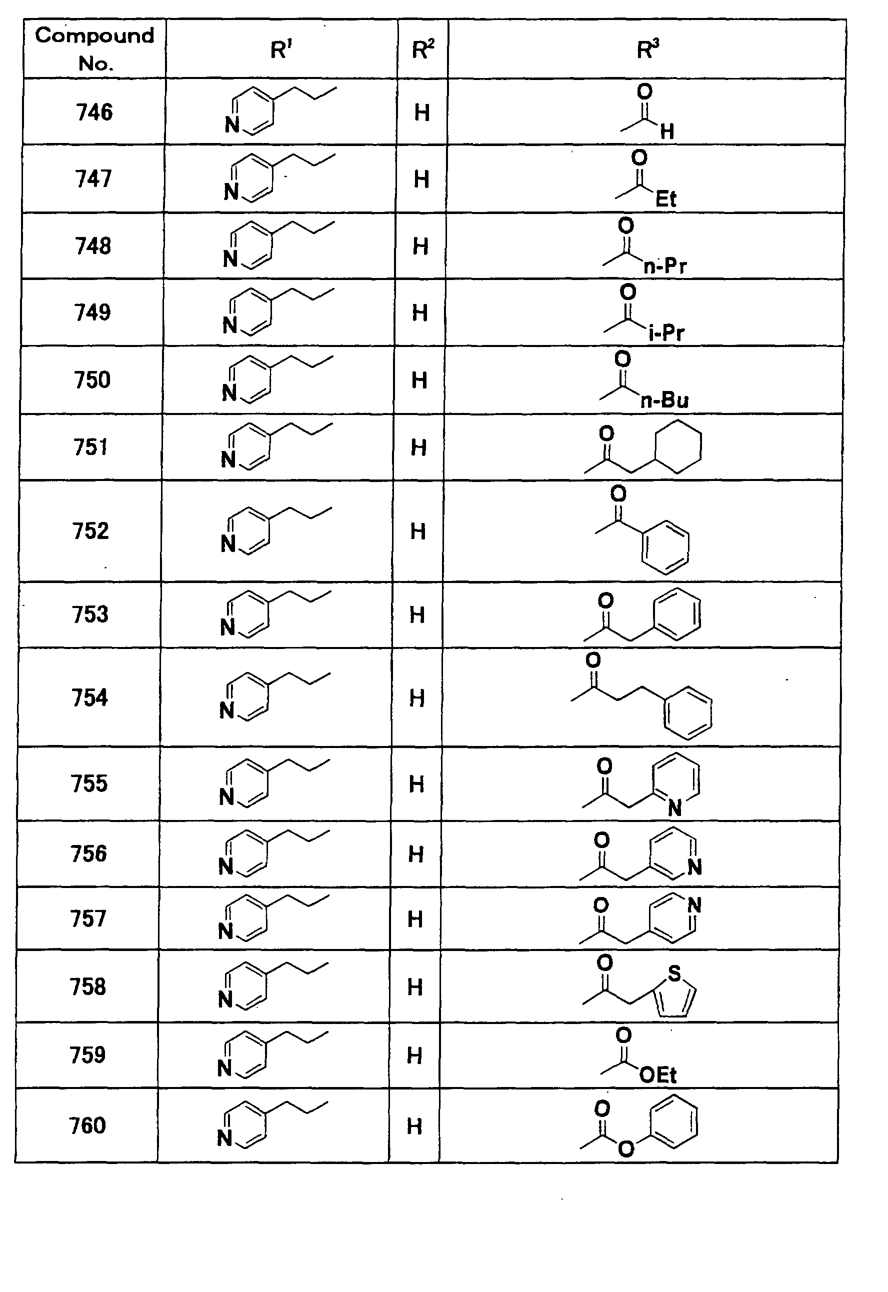

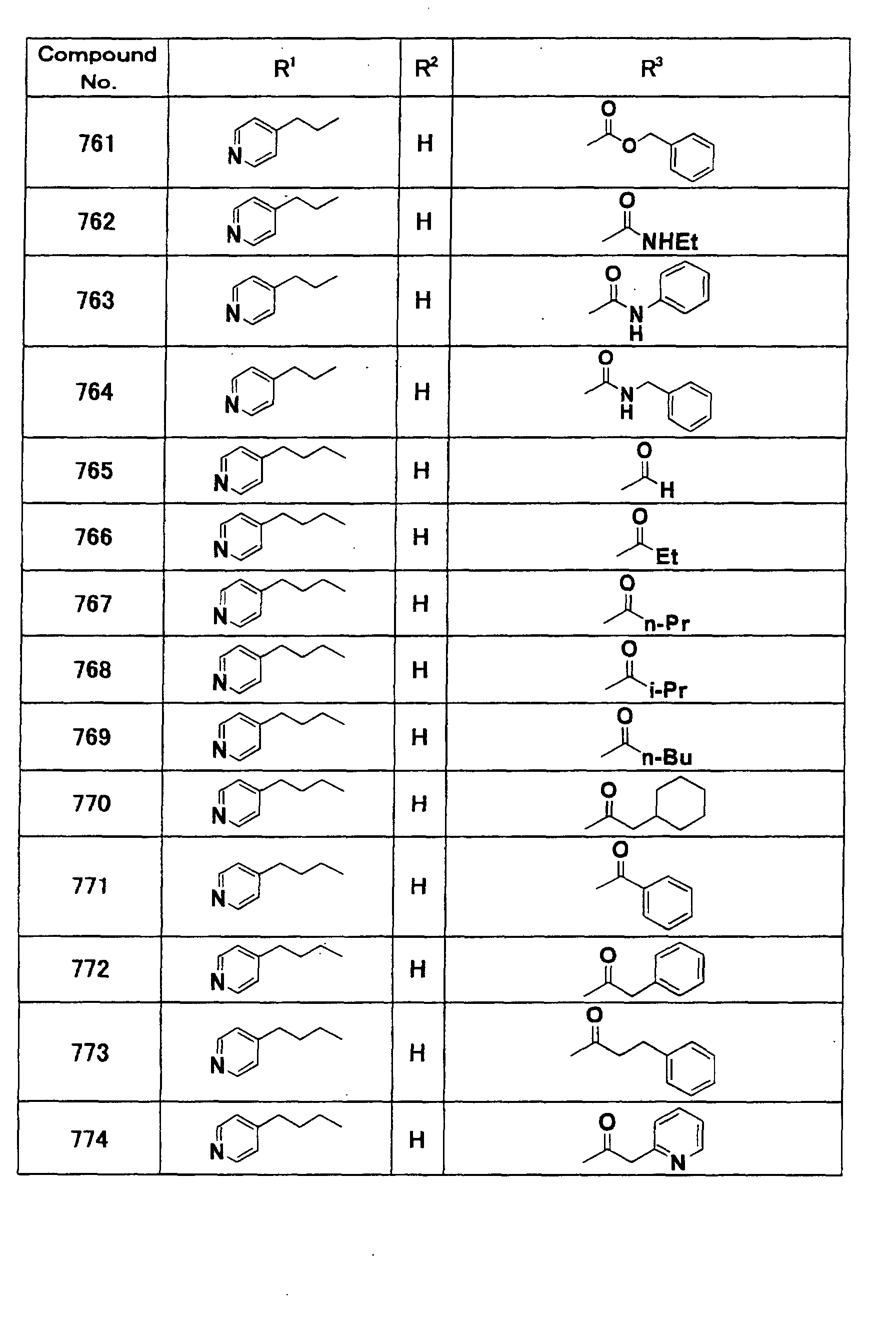

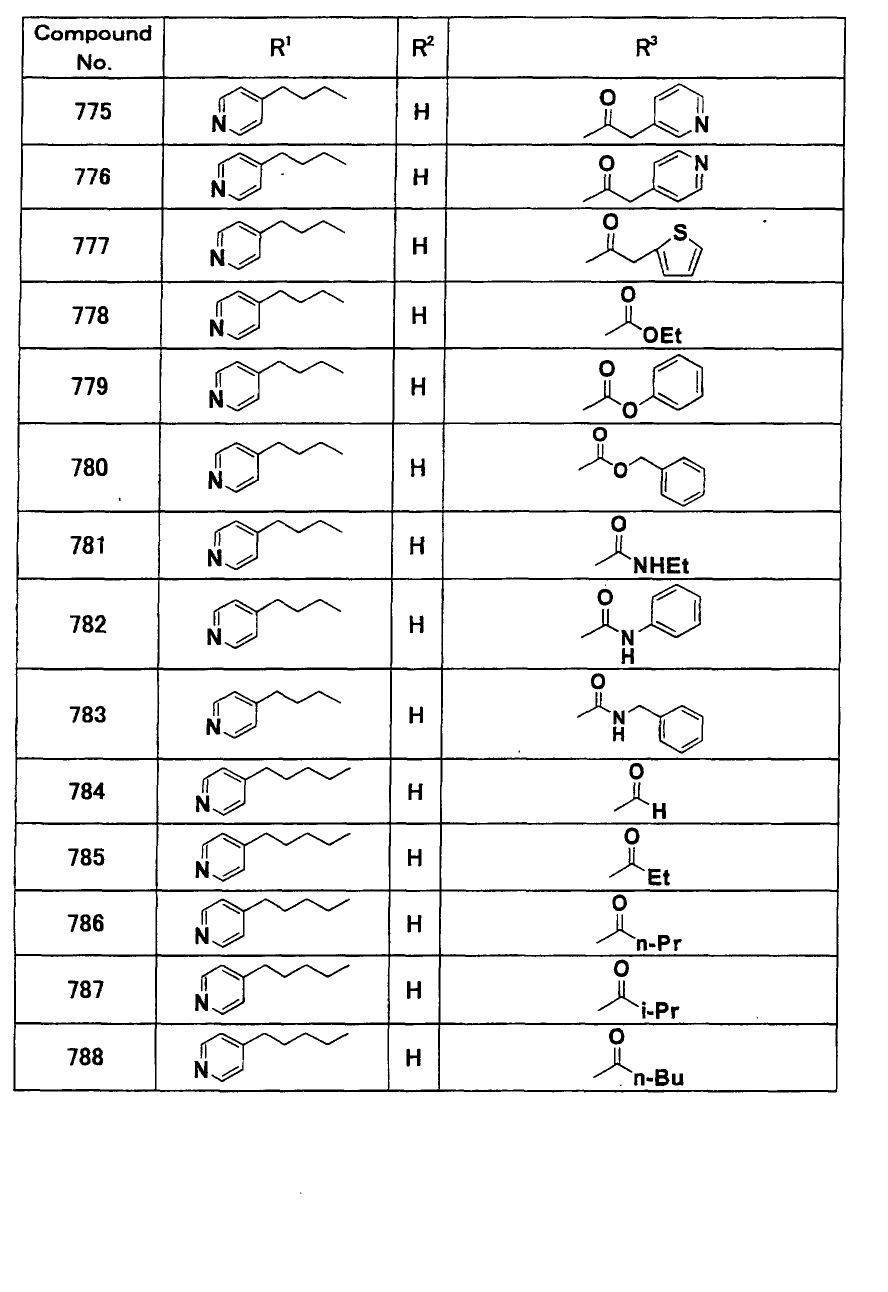

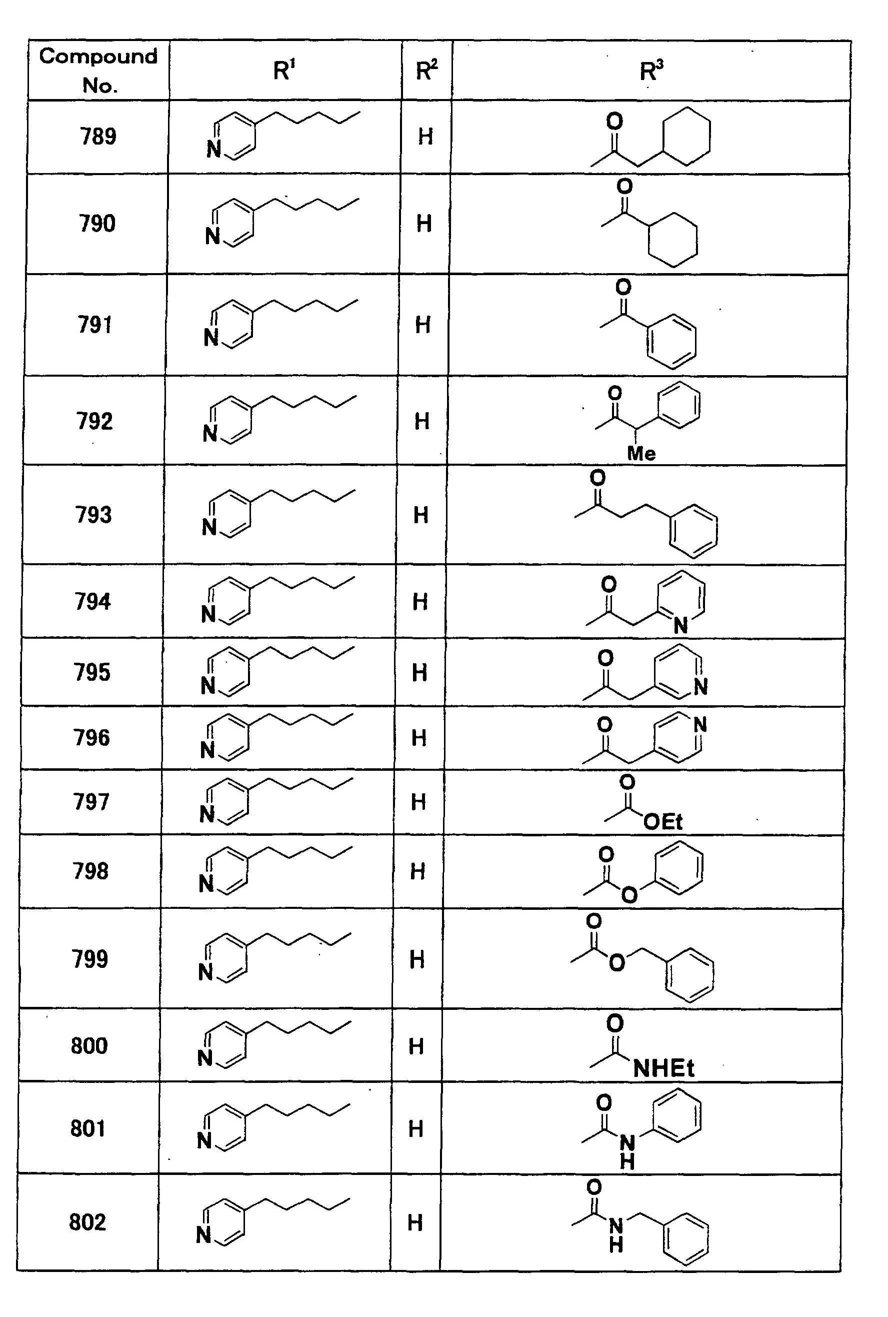

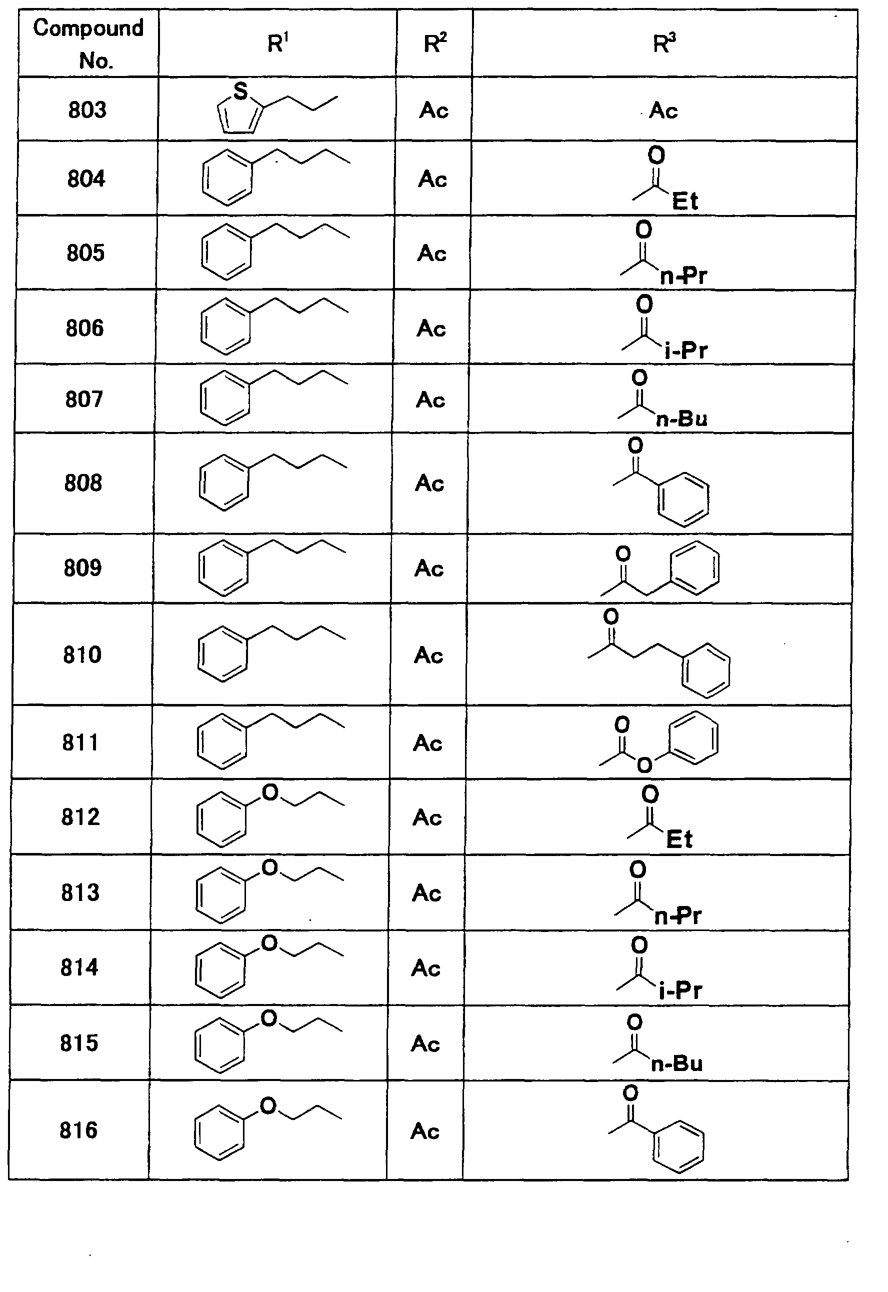

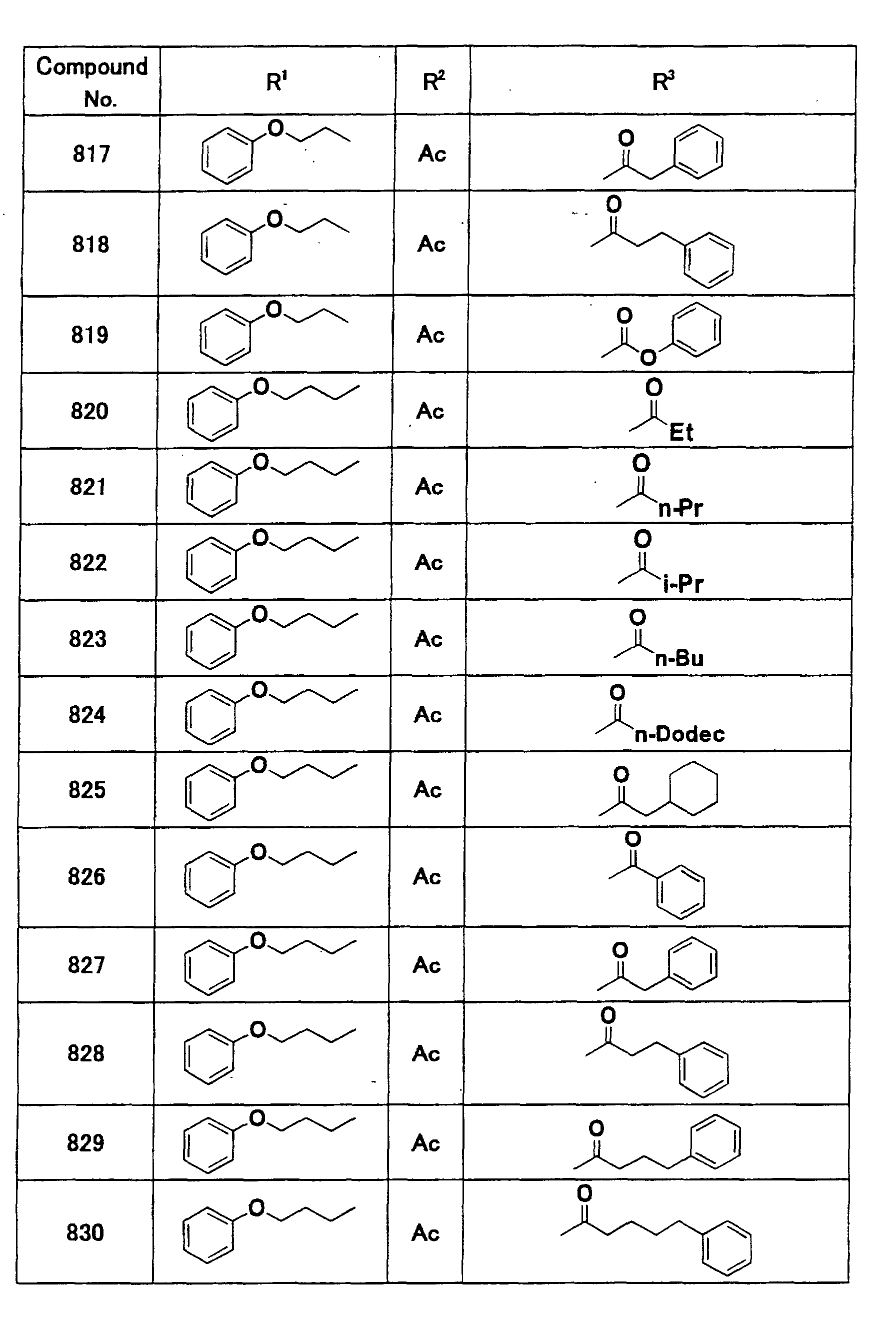

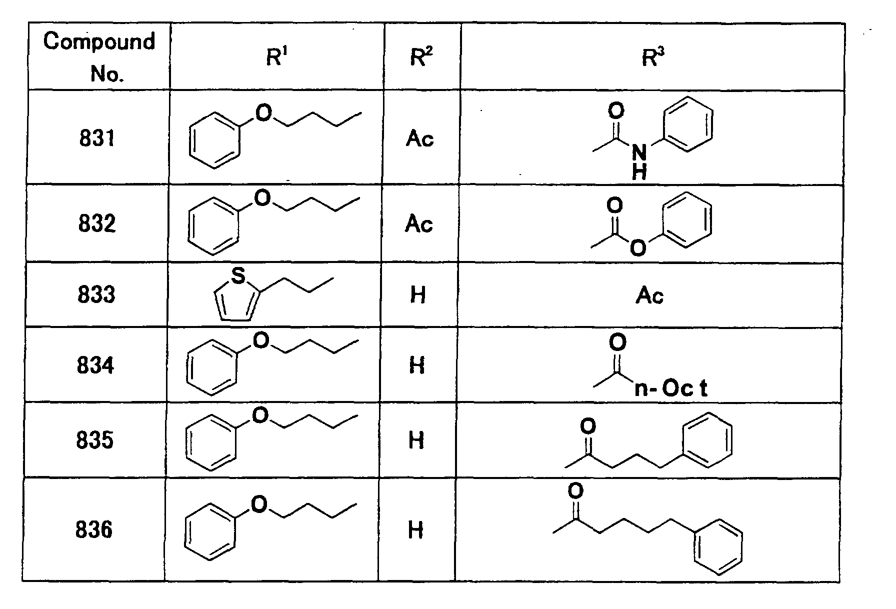

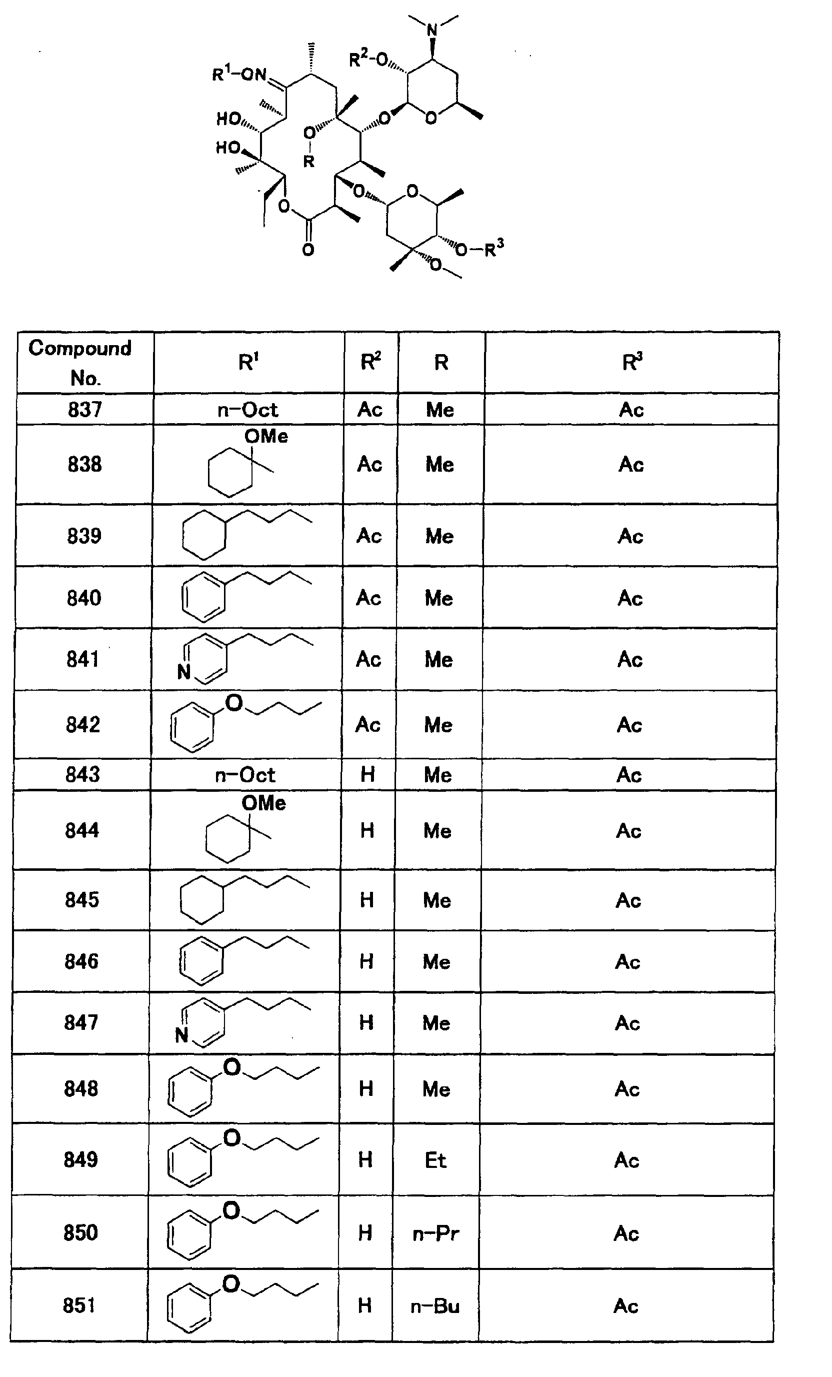

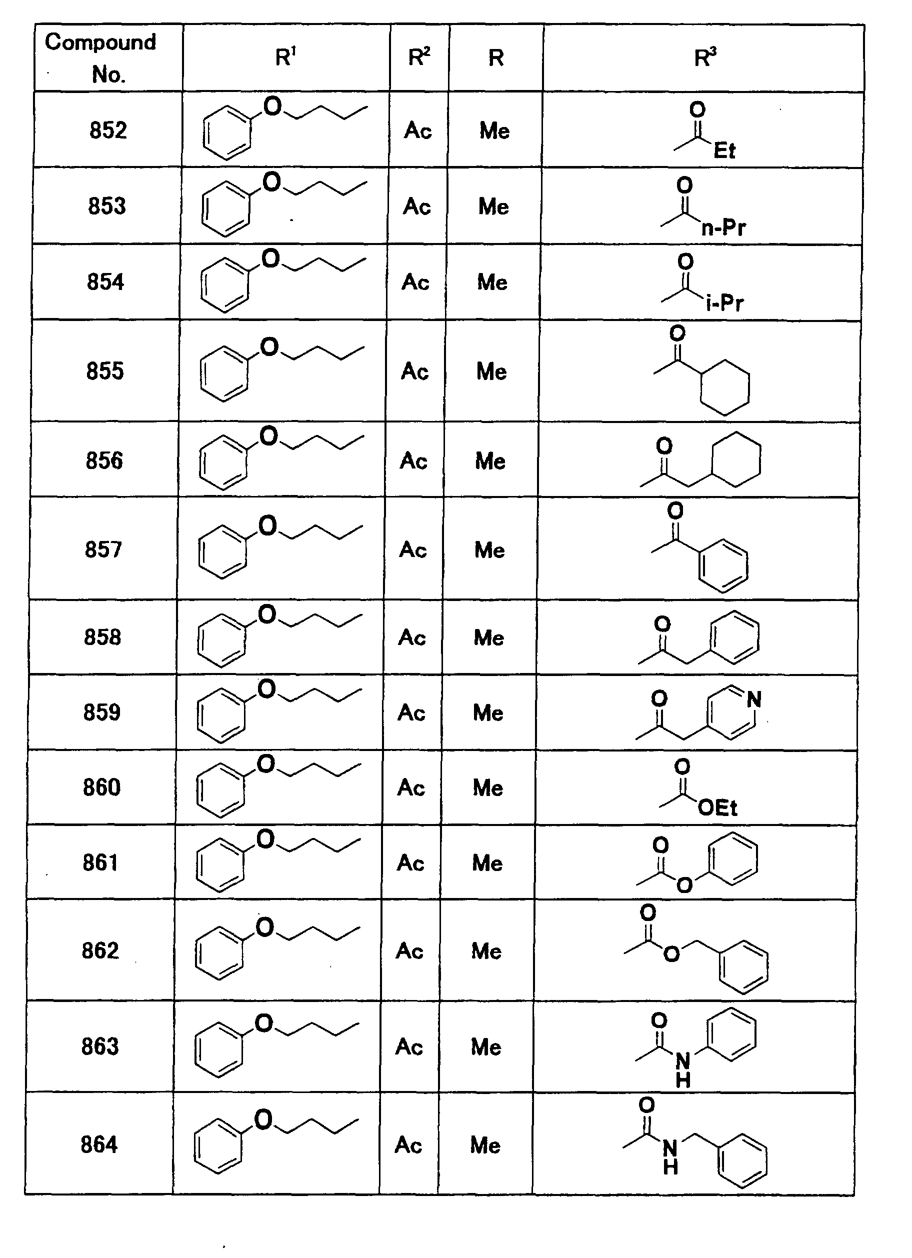

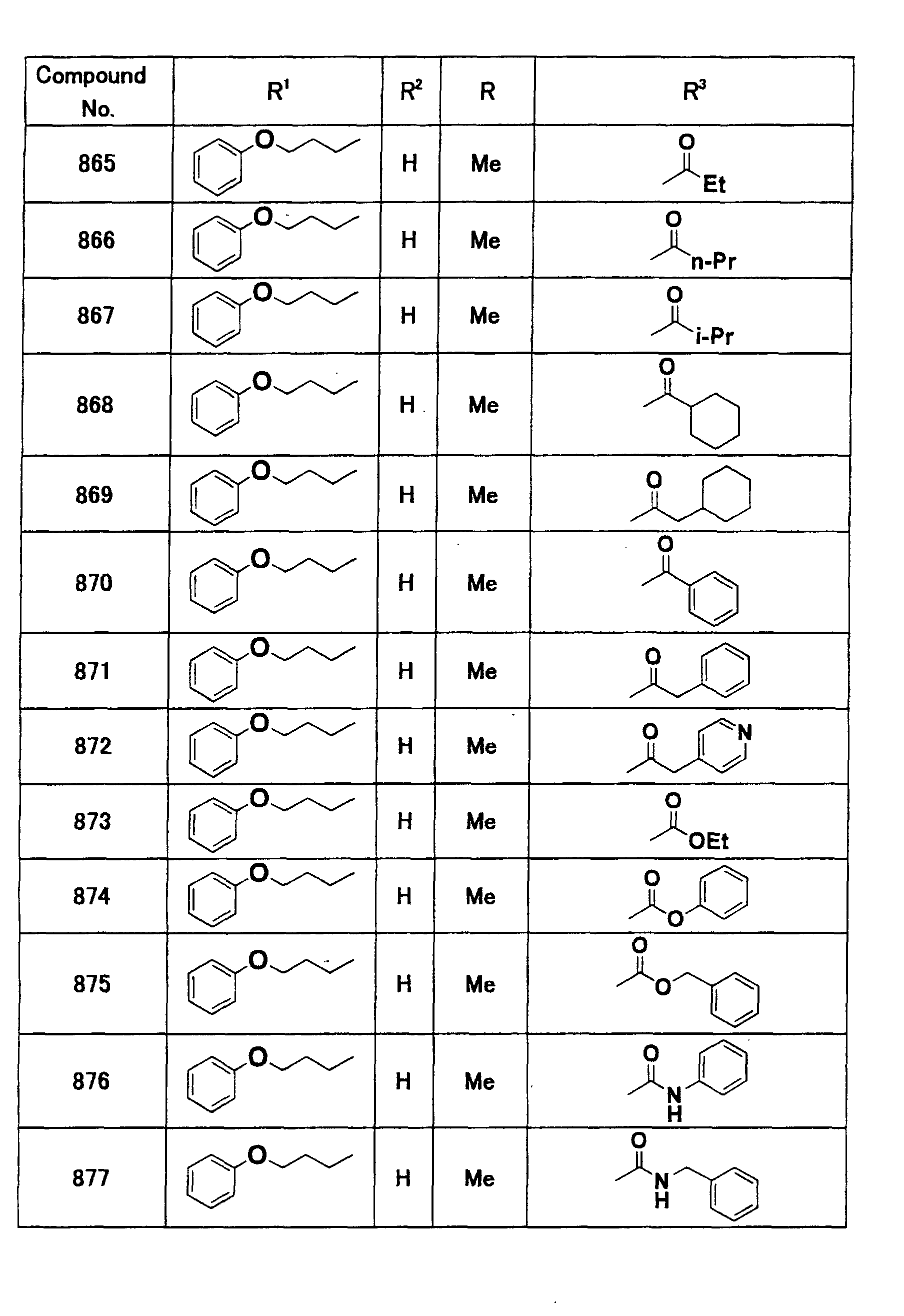

The novel erythromycin derivatives represented by the aforementioned general formula (I) of the present invention can be prepared by, for example, the methods explained below. However, the method for preparing the compounds of the present invention is not limited to these methods.

According to the first embodiment of the method for preparing the compounds of the present invention, compounds wherein both of R² and R³ are acetyl groups that fall within the compounds represented by the aforementioned general formula (I) can be prepared by allowing a compound represented by the following general formula (III): 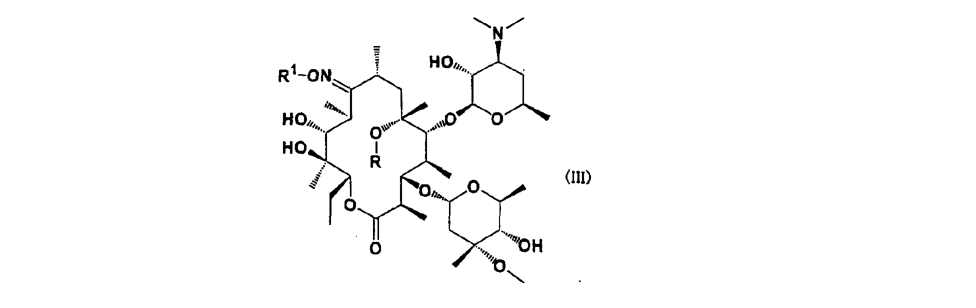 wherein R and R¹ have the same meanings as those defined above, to react with acetyl chloride or acetic anhydride in the presence or absence of a base without solvent or in a solvent.

Example of the base used in the method for preparing include, for example, organic bases such as triethylamine, pyridine, N,N-diisopropylethylamine, 4-dimethylaminopyridine, 1,8-diazabicyclo[5.4.0]-7-undecene and 1,2,2,6,6-pentamethylpiperidine and the like, or inorganic bases such as sodium carbonate, potassium carbonate, sodium hydrogencarbonate and potassium hydrogencarbonate and the like. The solvent used in the method for preparing may be any solvent so long as it per se is inert in the reaction and does not inhibit the reaction. Examples of such a solvent include, for example, halogenated hydrocarbon solvents such as dichloromethane, 1,2-dichloroethane and chloroform, aromatic hydrocarbon solvents such as benzene and toluene, aprotic polar solvents such as acetone, acetonitrile, N,N-dimethylformamide, N-methyl-2-pyrrolidone, dimethyl sulfoxide, tetramethylene sulfolan, tetramethylene sulfoxide and hexamethylenephosphoric triamide, ester solvents such as methyl acetate and ethyl acetate, ether solvents such as tetrahydrofuran, diethyl ether and 1,4-dioxane, organic base solvents such as pyridine, picoline, lutidine and collidine, or mixed solvents thereof. The reaction is performed at a temperature range of from under ice-cooling to 200°C.

According to the second embodiment of the method for preparing the compounds of the present invention, compounds wherein R² is acetyl group that fall within the compounds represented by the aforementioned general formula (I) can be prepared by allowing a compound represented by the following general formula (IV): 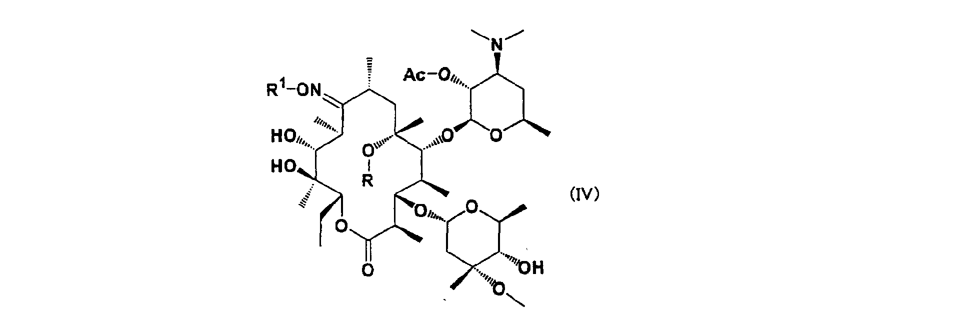 wherein R and R¹ have the same meanings as those defined above, and Ac represents an acetyl group, to react with formic acid in the presence or absence of perchloric acid without solvent or in a solvent, or to react with an acid anhydride represented by the following general formula (V):

R⁶-C(=O)-OW (V)

wherein R⁶ represents a hydrogen atom, an alkyl group which may be substituted, a cycloalkyl group which may be substituted, a (cycloalkyl)alkyl group which may be substituted, an aryl group which may be substituted, or an aralkyl group which may be substituted, and W represents an acid anhydride residue, or to react with an acid halide or halogenated carbonate derivative represented by the following general formula (VI):

R⁷-C(=O)-Q (VI)

wherein R⁷ represents a hydrogen atom, an alkyl group which may be substituted, a cycloalkyl group which may be substituted, a (cycloalkyl)alkyl group which may be substituted, an aryl group which may be substituted, an aralkyl group which may be substituted, an alkoxy group which may be substituted, an aryloxy group which may be substituted, or an aralkyloxy group which may be substituted, and Q represents a halogen atom, in the presence or absence of a base without solvent or in a solvent, or to react with an isocyanate derivative represented by the following general formula (VII):

R⁸-N=C=O (VII)

wherein R⁸ represents an alkyl group which may be substituted, an aryl group which may be substituted or an aralkyl group which may be substituted, in the presence or absence of a base without solvent or in a solvent.

Example of the base used in the method for preparing include, for example, organic bases such as triethylamine, pyridine, N,N-diisopropylethylamine, 4-dimethylaminopyridine, 1,8-diazabicyclo[5.4.0]-7-undecene and 1,2,2,6,6-pentamethylpiperidine, or inorganic bases such as sodium carbonate, potassium carbonate, sodium hydrogencarbonate, and potassium hydrogencarbonate. The solvent used in the method for preparing may be any solvent so long as it per se is inert in the reaction and does not inhibit the reaction. Examples of such a solvent include, for example, halogenated hydrocarbon solvents such as dichloromethane, 1,2-dichloroethane, and chloroform, aromatic hydrocarbon solvents such as benzene and toluene, aprotic polar solvents such as acetone, acetonitrile, N,N-dimethylformamide, N-methyl-2-pyrrolidone, dimethyl sulfoxide, tetramethylene sulfolan, tetramethylene sulfoxide, and hexamethylenephosphoric triamide, ester solvents such as methyl acetate and ethyl acetate, ether solvents such as tetrahydrofuran, diethyl ether and 1,4-dioxane, organic base solvents such as pyridine, picoline, lutidine and collidine, or mixed solvents thereof. The reaction is performed at a temperature range of from under ice-cooling to 200°C.

According to the third embodiment of the method for preparing the compounds of the present invention, the compounds represented by the aforementioned general formula (I) can be prepared by allowing a compound represented by the following general formula (VIII): 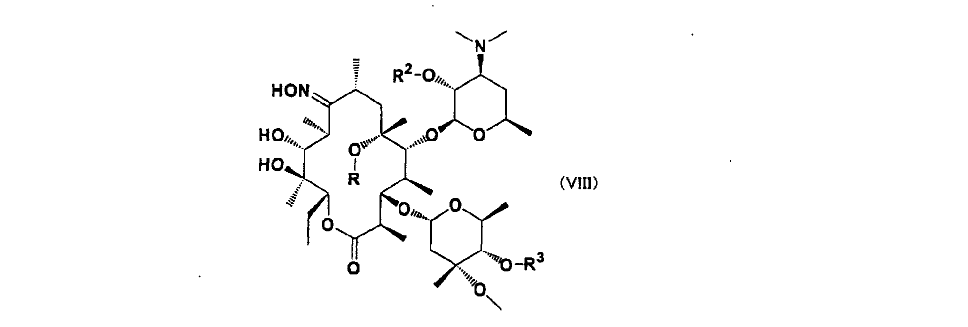 wherein R, R² and R³ have the same meanings as those defined above, to react with a compound represented by the following general formula (IX):

R¹-T (IX)

wherein R¹ have the same meaning as those defined above, and T represents a halogen atom, mesyloxy group, tosyloxy group or trifluoromethylsulfonyloxy group, and tetrabutylammonium iodide in the presence or absence of sodium iodide or a base without solvent or in a solvent.

Example of the base used in the method for preparing include, for example, organic bases such as triethylamine, pyridine, N,N-diisopropylethylamine, 4-dimethylaminopyridine, 1,8-diazabicyclo[5.4.0]-7-undecene and 1,2,2,6,6-pentamethylpiperidine, or inorganic bases such as sodium carbonate, potassium carbonate, sodium hydrogencarbonate, potassium hydrogencarbonate, sodium hydride, sodium hydroxide and potassium hydroxide. The solvent to be used may be any solvent so long as it per se is inert in the reaction and does not inhibit the reaction. Examples of the solvent include, for example, halogenated hydrocarbon solvents such as dichloromethane, 1,2-dichloroethane and chloroform, aromatic hydrocarbon solvents such as benzene and toluene, aprotic polar solvents such as acetone, acetonitrile, N,N-dimethylformamide, N-methyl-2-pyrrolidone, dimethyl sulfoxide, tetramethylene sulfolan, tetramethylene sulfoxide and hexamethylenephosphoric triamide, ester solvents such as methyl acetate and ethyl acetate, ether solvents such as tetrahydrofuran, diethyl ether and 1,4-dioxane, organic base solvents such as pyridine, picoline, lutidine and collidine or mixed solvents thereof. The reaction is performed at a temperature range of from under ice-cooling to 200°C.

According to the fourth embodiment of the method for preparing the compounds of the present invention, compounds wherein R¹ is a cycloalkyl group substituted with an alkoxyl group that fall within the compounds represented by the aforementioned general formula (I) can be prepared by allowing a compound represented by the aforementioned general formula (VIII), to react with a cycloalkyl compound represented by the following general formula (X): 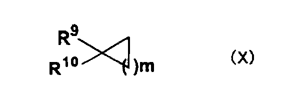 wherein R⁹ and R¹⁰ independently represent an alkoxyl group, and m represents an integer of from 1 to 4, or to react with a cycloalkene compound represented by the following general formula (XI): 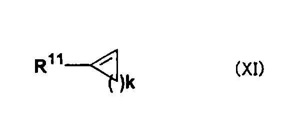 wherein R¹¹ represents an alkoxyl group, and k represents an integer of from 1 to 4, in the presence or absence of an acid catalyst without solvent or in a solvent.

Example of the acid used in the method for preparing include, for example, pyridine hydrochloride, pyridine trifluoroacetate, pyridine p-toluenesulfonate and the like. The solvent to be used may be any solvent so long as it per se is inert in the reaction and does not inhibit the reaction. Examples of the solvent include, for example, halogenated hydrocarbon solvents such as dichloromethane, 1,2-dichloroethane and chloroform, aromatic hydrocarbon solvents such as benzene and toluene, aprotic polar solvents such as acetone, acetonitrile, N,N-dimethylformamide, N-methyl-2-pyrrolidone, dimethyl sulfoxide, tetramethylene sulfolan, tetramethylene sulfoxide and hexamethylenephosphoric triamide, ester solvents such as methyl acetate and ethyl acetate, ether solvents such as tetrahydrofuran, diethyl ether and 1,4-dioxane or mixed solvents thereof. The reaction is performed at a temperature range of from under ice-cooling to 200°C.

According to the fifth embodiment of the method for preparing the compounds of the present invention, compounds wherein R² is a hydrogen atom that fall within the compounds represented by the aforementioned general formula (I) can be prepared by hydrolyzing a compound represented by the aforementioned general formula (I) wherein R² is acetyl group without a solvent or in a solvent in the presence or absence of an acid or a base.

Examples of the acid used in the method for preparing include, for example, hydrochloric acid, sulfuric acid and the like. Examples of the base to be used include, for example, sodium hydrogencarbonate, sodium carbonate, sodium hydroxide, lithium hydroxide, barium hydroxide, sodium methoxide, sodium ethoxide, sodium tert-butoxide, potassium tert-butoxide and the like. Although these acids or alkalis can be used as an aqueous solution, they can also be used as a solution in an alcoholic solvents such as methanol, ethanol, n-propanol, isopropyl alcohol, n-butanol, sec-butyl alcohol and tert-butyl alcohol, aprotic polar solvents such as acetone, acetonitrile, N,N-dimethylformamide, dimethyl sulfoxide, tetramethylene sulfolan, tetramethylene sulfoxide and hexamethylenephosphoric triamide, ether solvents such as tetrahydrofuran and 1,4-dioxane, or water-containing solvents comprising any of these solvents and the like. The reaction is performed at a temperature range of from under ice-cooling to 200°C.

Some of the compounds represented by the aforementioned general formulas (III), (IV) and (VIII), which are used as the starting materials of the methods for preparing the compounds of the present invention, are already known compounds which are disclosed in Japanese Patent Unexamined Publication (KOKAI) Nos. 56-100799/1981, 63-107921/1988, 3-204893/1991 and the like. They can be prepared, for example, as explained below. The details of the preparation of novel compounds will be shown as reference examples. 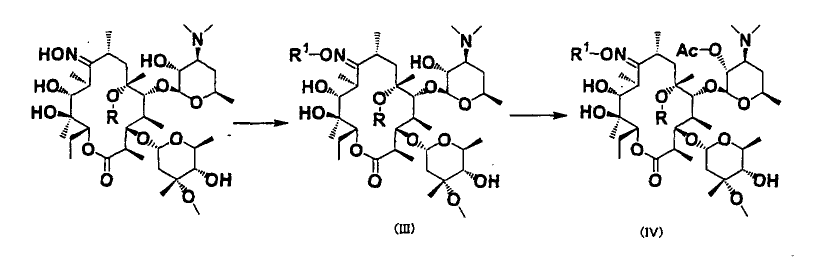 (In the formulas, R and R¹ have the same meanings as those defined above, and Ac represents an acetyl group.)

As compounds relating to the compounds of the present invention represented by the aforementioned general formula (I), novel compounds represented by the following general formula (XII): 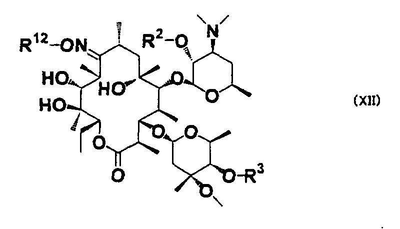 wherein R² and R³ have the same meanings as those defined above and R¹² represents a C₂₋₄ alkyl group which may be substituted, can be prepared according to the aforementioned preparations of the compounds of the present invention. The details of the preparations of these compounds will also be described as reference examples.

The medicament comprising at least one of the novel erythromycin derivatives represented by the aforementioned general formula (I) or salts thereof as an active ingredient are usually administered as preparations for oral administration such as capsules, tablets, subtilized granules, granules, powders and syrups, or preparations such as injections, suppositories, eye drops, ophthalmologic ointments, ear drops, nasal drops and dermatological preparations. These preparations can be manufactured in a conventional manner by admixing with physiologically and pharmaceutically acceptable additives. For preparations for oral administration and suppositories, pharmaceutical additives such as, for example, excipients such as lactose, sucrose, D-mannitol, corn starch and crystalline cellulose; disintegrating agents such as carboxymethylcellulose, calcium carboxymethylcellulose, partly pregelatinized starch, croscarmellose sodium and crospovidone; binders such as hydroxypropylcellulose, hydroxypropylmethylcellulose and polyvinylpyrrolidone; lubricants such as magnesium stearate, talc, hardened oil, dimethylpolysiloxane, hydrated silicon dioxide, colloidal silicon dioxide and carnauba wax; coating agents such as hydroxypropylmethylcellulose, sucrose and titanium oxide; plasticizers such as triethyl citrate, polyethylene glycol and fatty acid glycerin esters; bases such as polyethylene glycol and hard fat and the like may be used. For injections, eye drops and ear drops, pharmaceutical additives such as, for example, dissolving agents and dissolving aids that can constitute aqueous preparations or preparations to be dissolved upon use such as distilled water for injection, physiological saline and propylene glycol; pH modifiers such as inorganic or organic acids and bases, isotonic agents such as sodium chloride, glucose and glycerin, stabilizers and the like may be used. For ophthalmologic ointments and dermatological preparations, pharmaceutical additives suitable for ointments, creams and patches such as white soft paraffine, macrogol, grycerin, liquid paraffine and cotton cloth may be used.

When the medicament comprising the compound of the present invention as an active ingredient is administered to a patient, doses may vary depending on symptoms of the patient. The dose for an adult may generally be 10 to 2000 mg per day for oral administration, or 1 to 1000 mg per day for parenteral administration, which daily doses may be administered at one time or several times as divided portions. It is desirable that the doses are suitably increased or decreased depending on the purpose of the administration, i.e., therapeutic or preventive treatment, a region of infection and the type of pathogenic bacteria, the age and symptoms of a patient and the like.

### EXAMPLES

The present invention will be further explained with reference to the following reference examples and examples. However, the present invention is not limited to these examples. In the following tables, Me represents methyl group, Et represents ethyl group, n-Pr represents n-propyl group, i-Pr represents isopropyl group, n-Bu represents n-butyl group, n-Hex represents n-hexyl group, n-Oct represents n-octyl group, n-Dodec represents n-dodecyl group, and Ac represents acetyl group. The parenthesized descriptions in the description physicochemical properties indicate recrystallization solvents.

### Reference Example 1: Erythromycin A 9-[O-(3-phenoxypropyl)oxime]

Powdered potassium hydroxide (0.43 g) was added to a mixture of 4.00 g of erythromycin A 9-oxime, 0.10 g of tetrabutylammonium iodide, 0.12 g of sodium iodide and 1.30 ml of 3-phenoxypropyl bromide in 40 ml of tetrahydrofuran at room temperature with stirring, and the mixture was stirred at room temperature for 16.5 hours. The reaction mixture was concentrated under reduced pressure, and then the resulting residue was made alkaline with saturated aqueous sodium hydrogencarbonate solution, and the mixture was extracted with ethyl acetate. The extract was washed successively with water and saturated brine, dried over sodium sulfate, and the solvent was removed under reduced pressure. The residue was added with diethyl ether and diisopropyl ether for solidification to give 2.80 g of a colorless solid.
NMR spectrum δ (CDCl₃) ppm: 0.85 (3H, t, J=7.5Hz), 1.02 (3H, d, J=6.5Hz), 1.08-1.80 (31H, m), 1.87-2.05 (2H, m), 2.08-2.18 (2H, m), 2.21 (1H, d, J=10.5Hz), 2.29 (6H, s), 2.33-2.48 (1H, m), 2.36 (1H, d, J=15.5Hz), 2.63-2.72 (1H, m), 2.85-2.96 (1H, m), 3.02 (1H, t, J=10Hz), 3.10 (1H, s), 3.22 (1H, dd, J=10, 7.5Hz), 3.32 (3H, s), 3.41 (1H, s), 3.44-3.53 (1H, m), 3.57 (1H, d, J=7.5Hz), 3.64-3.76 (2H, m), 3.96-4.10 (4H, m), 4.16-4.27 (2H, m), 4.38 (1H, s), 4.42 (1H, d, J=7.5Hz), 4.92 (1H, d, J=5Hz), 5.12 (1H, dd, J=11, 2Hz), 6.84-6.96 (3H, m), 7.21-7.32 (2H, m)

The compounds of Reference Examples 2 through 18 were obtained in the same manner as that described in Reference Example 1.

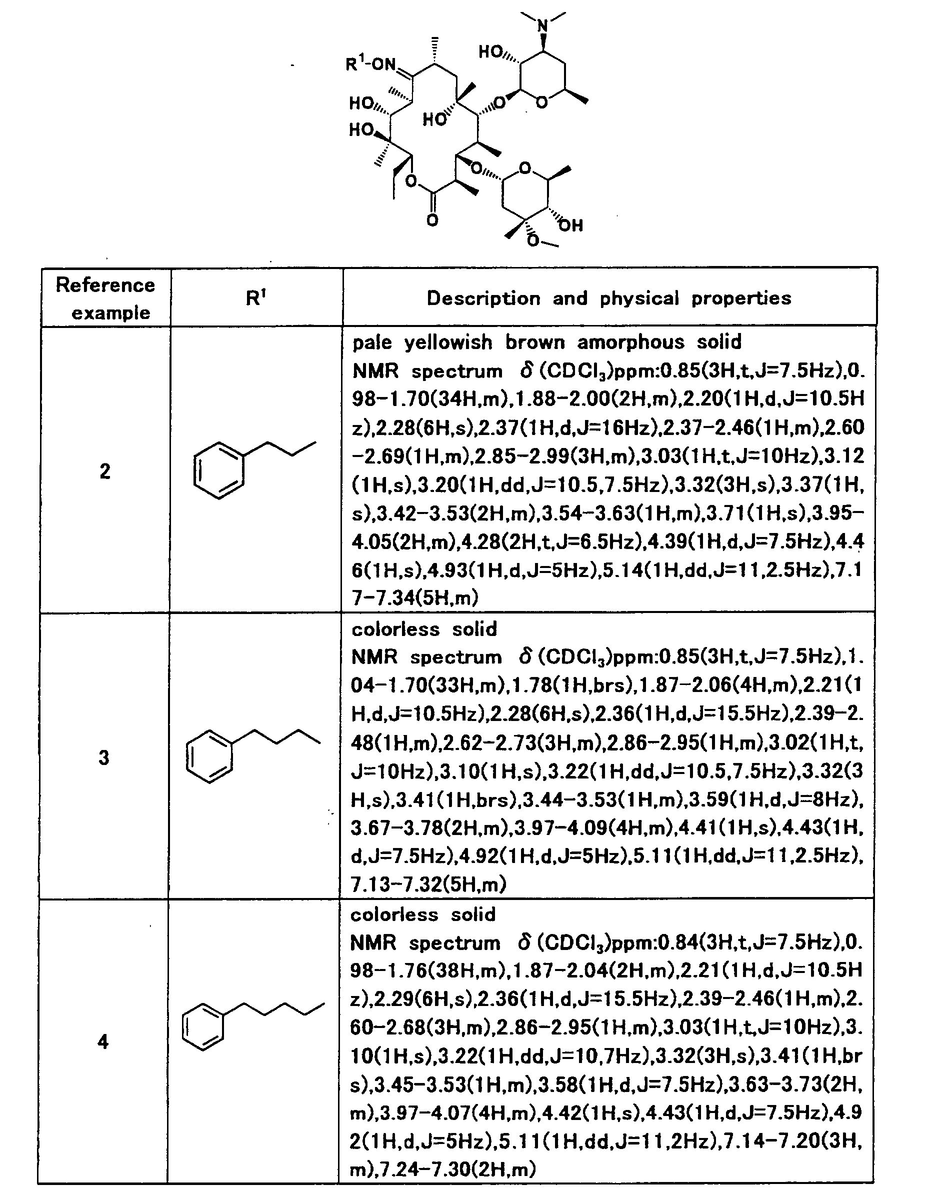

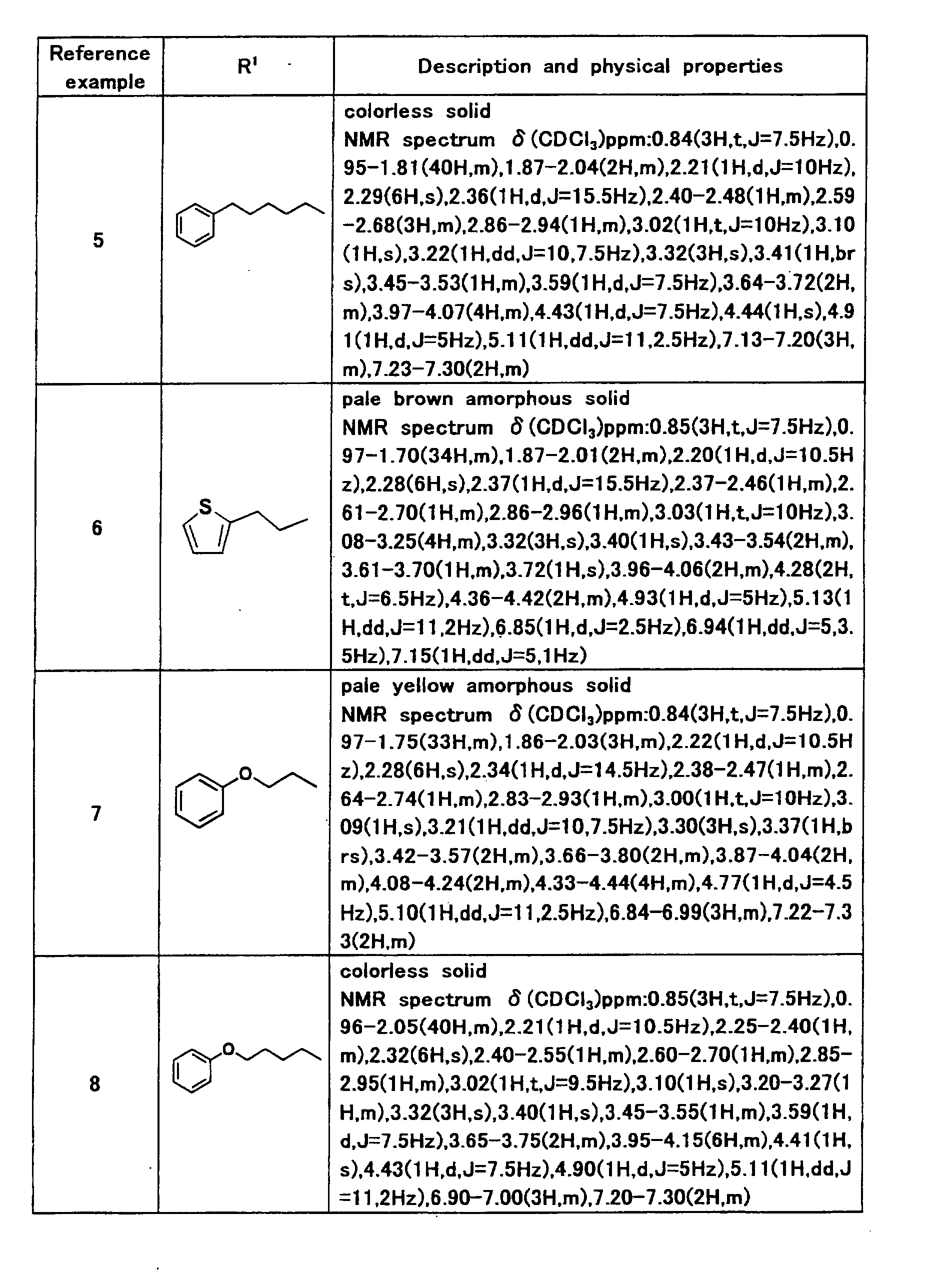

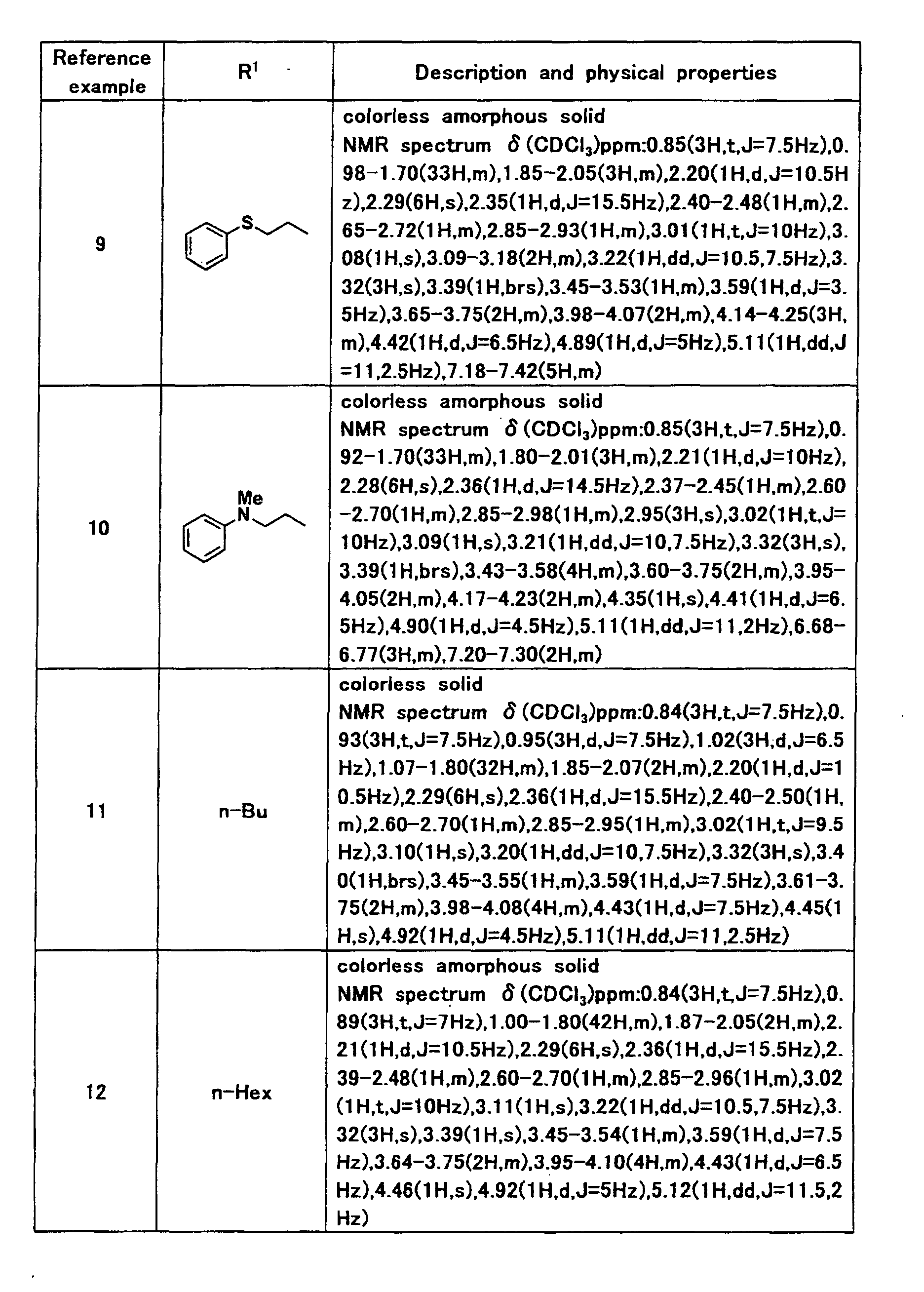

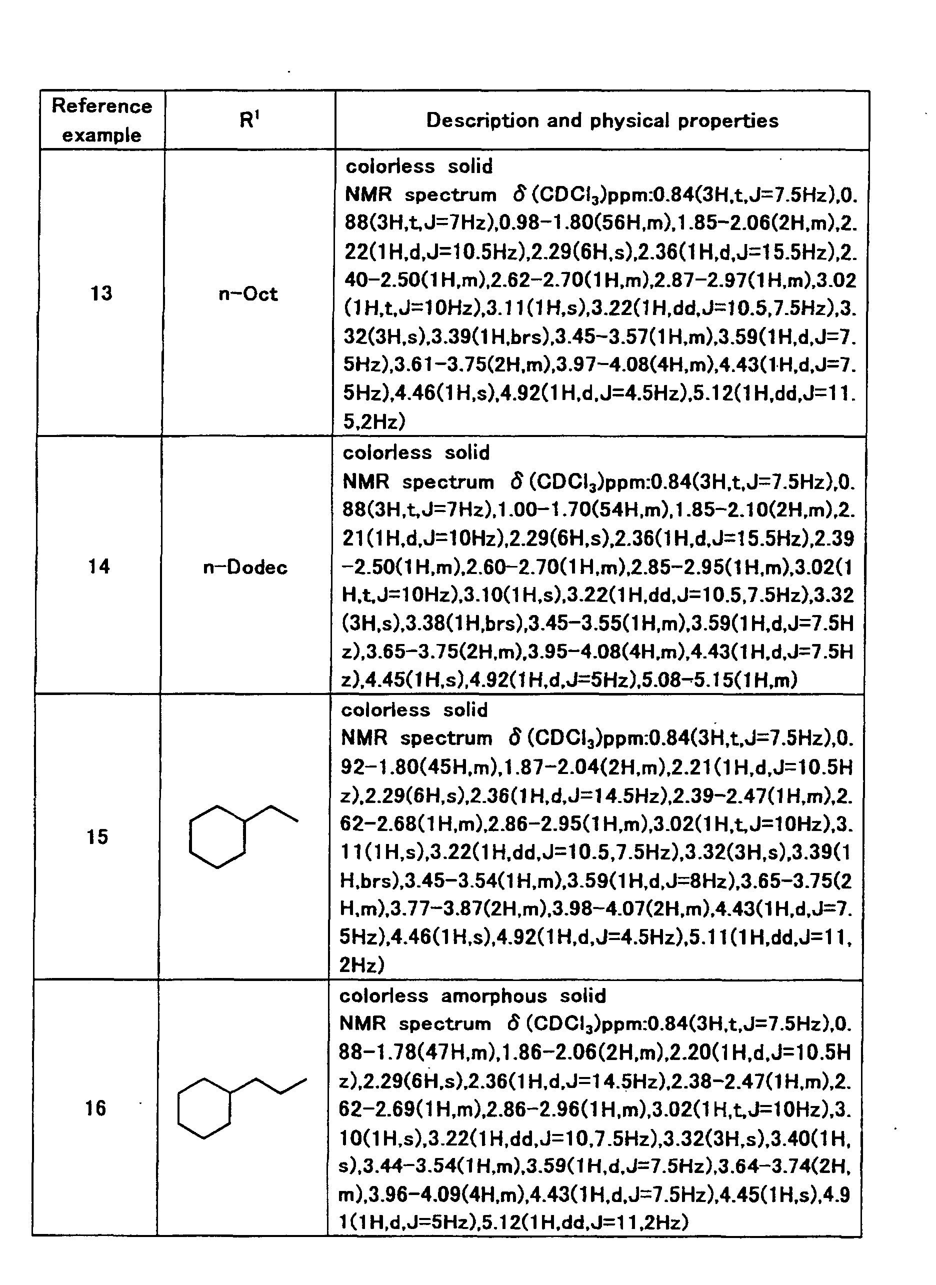

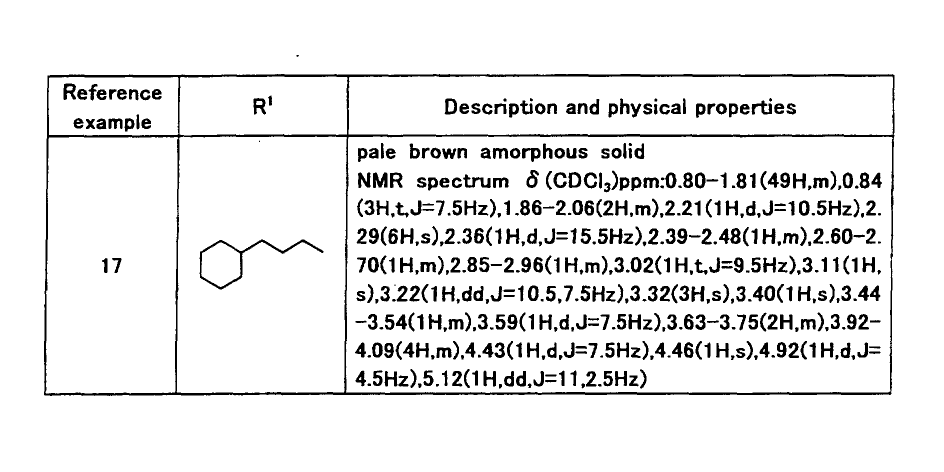

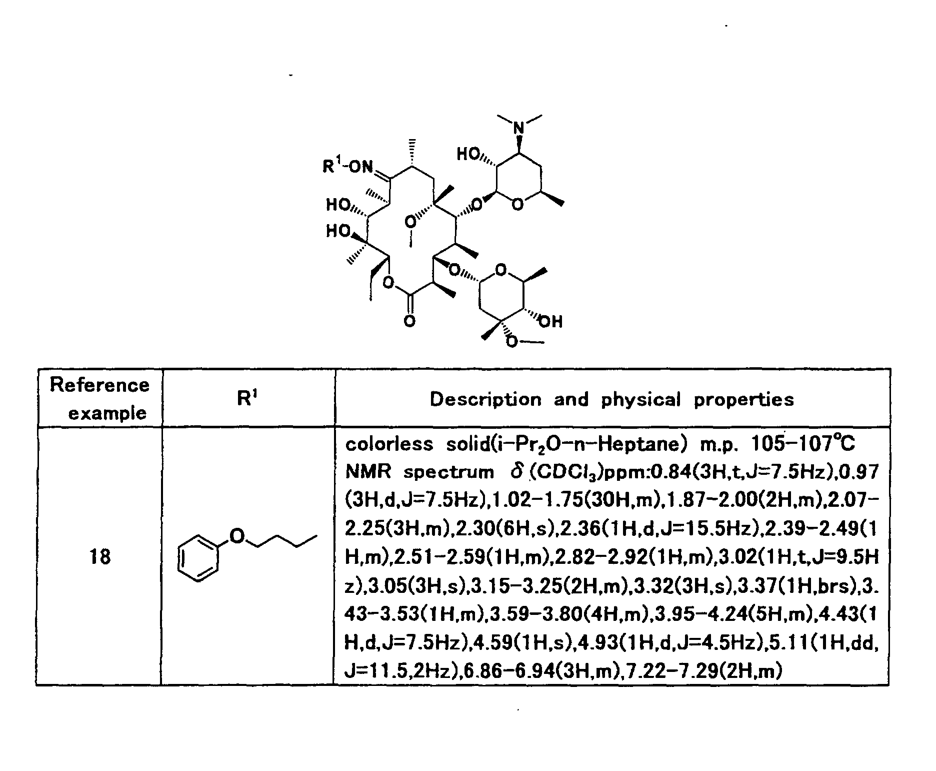

### Reference Example 19: 2'-O-Acetylerythromycin A 9-[O-(3-phenoxypropyl)oxime]

To a solution of 8.00 g of erythromycin A 9-[O-(3-phenoxypropyl)oxime] in 80 ml of acetone, 1.00 ml of acetic anhydride was added dropwise, and the mixture was stirred at room temperature for 6 hours. The reaction mixture was concentrated under reduced pressure. The resulting residue was added with diisopropyl ether, and then the mixture was heated and filtered under heating. The filtrate was cooled, and precipitated crystals were collected by filtration to obtain 6.00 g of a colorless solid. The solid was recrystallized from a mixture of acetone and diisopropyl ether to give a colorless solid having a melting point of 99-101°C.
NMR spectrum δ (CDCl₃) ppm: 0.84 (3H, t, J=7.5Hz), 0.93 (3H, d, J=7.5Hz), 1.03 (3H, d, J=7.5Hz), 1.10-1.80 (28H, m), 1.87-1.98 (2H, m), 2.02-2.20 (3H, m), 2.06 (3H, s), 2.28 (6H, s), 2.35 (1H, d, J=15.5Hz), 2.55-2.69 (2H, m), 2.83-2.93 (1H, m), 3.04 (1H, t, J=9.5Hz), 3.11 (1H, s), 3.35 (3H, s), 3.43-3.55 (2H, m), 3.61-3.73 (2H, m), 3.90-4.08 (4H, m), 4.15-4.26 (2H, m), 4.39 (1H, s), 4.57 (1H, d, J=8Hz), 4.70-4.81 (1H, m), 4.92 (1H, d, J=5Hz), 5.11 (1H, d, J=9Hz), 6.84-6.96 (3H, m), 7.22-7.31 (2H, m)

The compounds of Reference Examples 20 through 25 were obtained in the same manner as that described in Reference Example 19.

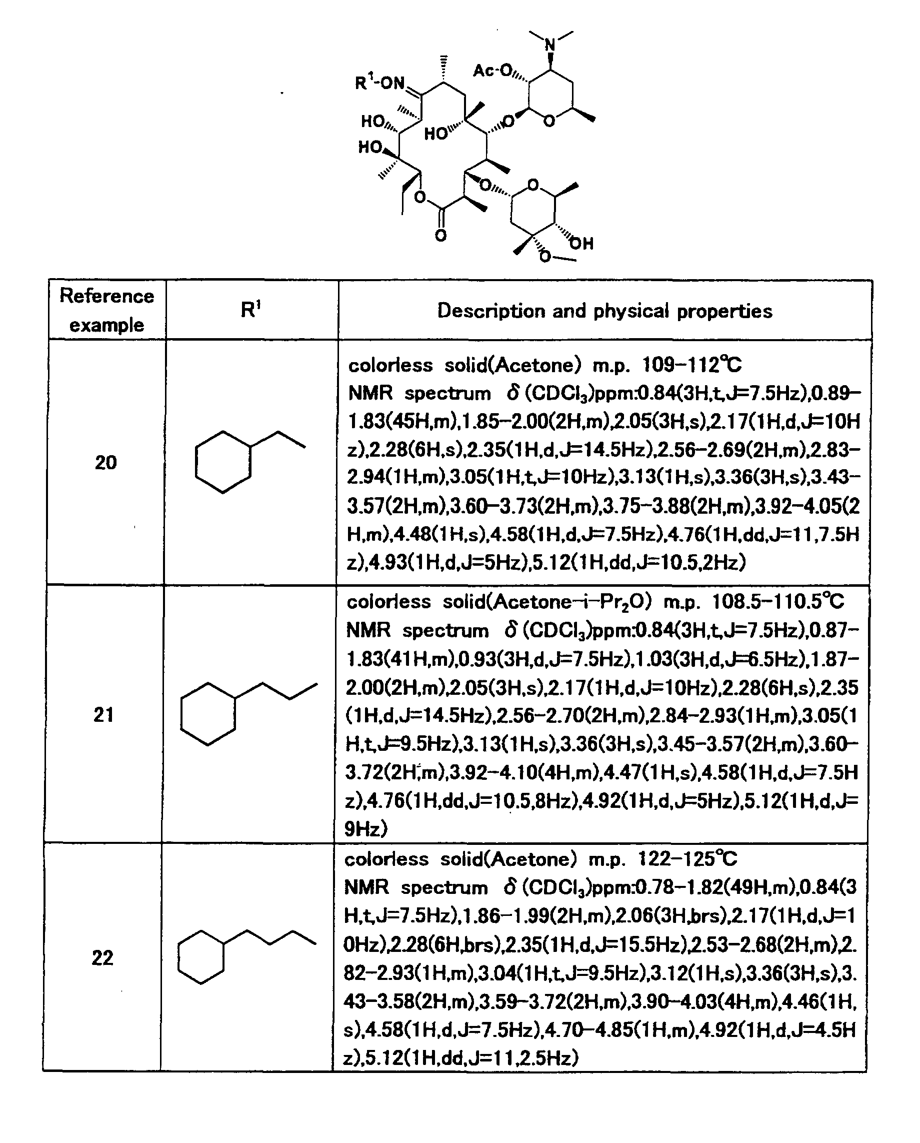

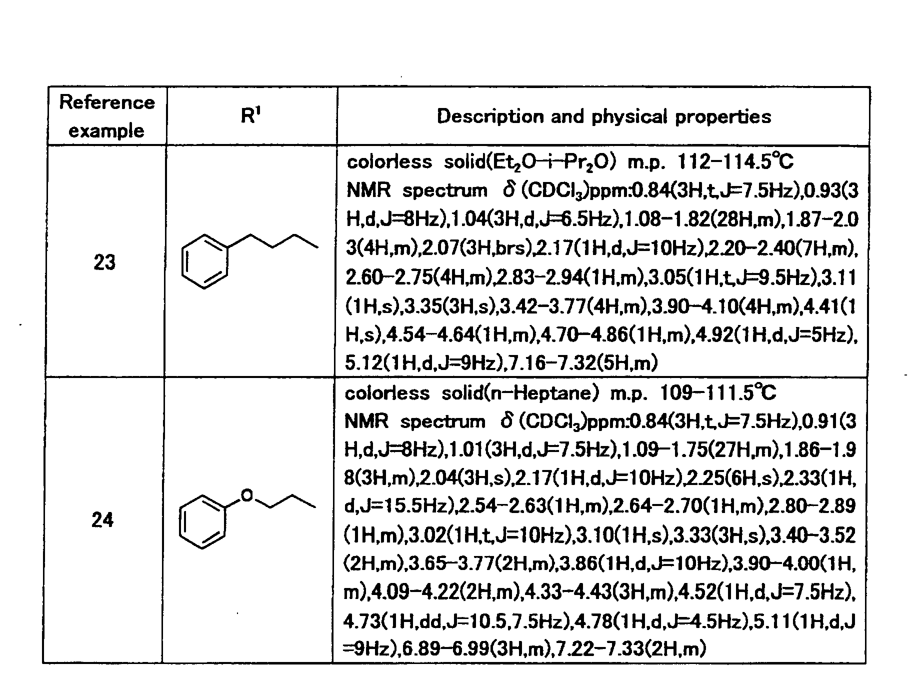

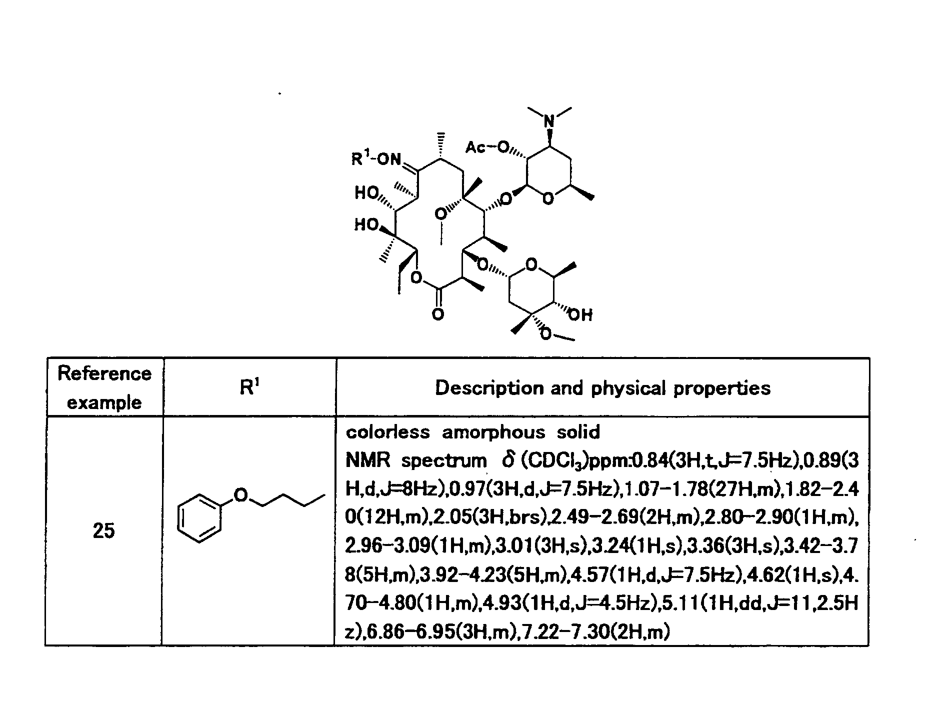

### Reference Example 26: 2',4"-O-Diacetylerythromycin A 9-(O-ethyloxime)

To a solution of 0.50 g of erythromycin A 9-(O-ethyloxime) in 5 ml of pyridine, 0.61 ml of acetic anhydride was added dropwise and the mixture was stirred at room temperature for 64 hours. The reaction mixture was added with water, and the mixture was made alkaline with saturated aqueous sodium hydrogencarbonate solution and extracted with diethyl ether. The extract was washed successively with water and saturated brine, dried over sodium sulfate, and the solvent was removed under reduced pressure. The residue was purified by column chromatography (silica gel, ethyl acetate) to give 0.27 g of a colorless amorphous solid.
NMR spectrum δ (CDCl₃) ppm: 0.84 (3H, t, J=7.5Hz), 0.95 (3H, d, J=8Hz), 1.04 (3H, d, J=6.5Hz), 1.08-1.78 (30H, m), 1.80-2.00 (3H, m), 2.05 (3H, s), 2.10 (3H, s), 2.30 (6H, s), 2.41 (1H, d, J=14.5Hz), 2.60-2.80 (2H, m), 2.84-2.95 (1H, m), 3.12 (1H, s), 3.34 (3H, s), 3.50 (1H, d, J=6.5Hz), 3.59-3.80 (3H, m), 3.96 (1H, d, J=10Hz), 4.06 (2H,q, J=7Hz), 4.25-4.36 (1H, m), 4.46 (1H, s), 4.64-4.84 (3H, m), 4.99 (1H, d, J=5Hz), 5.13 (1H, dd, J=11, 2Hz)

The compounds of Reference Examples 27 and 28 were obtained in the same manner as that described in Reference Example 26.

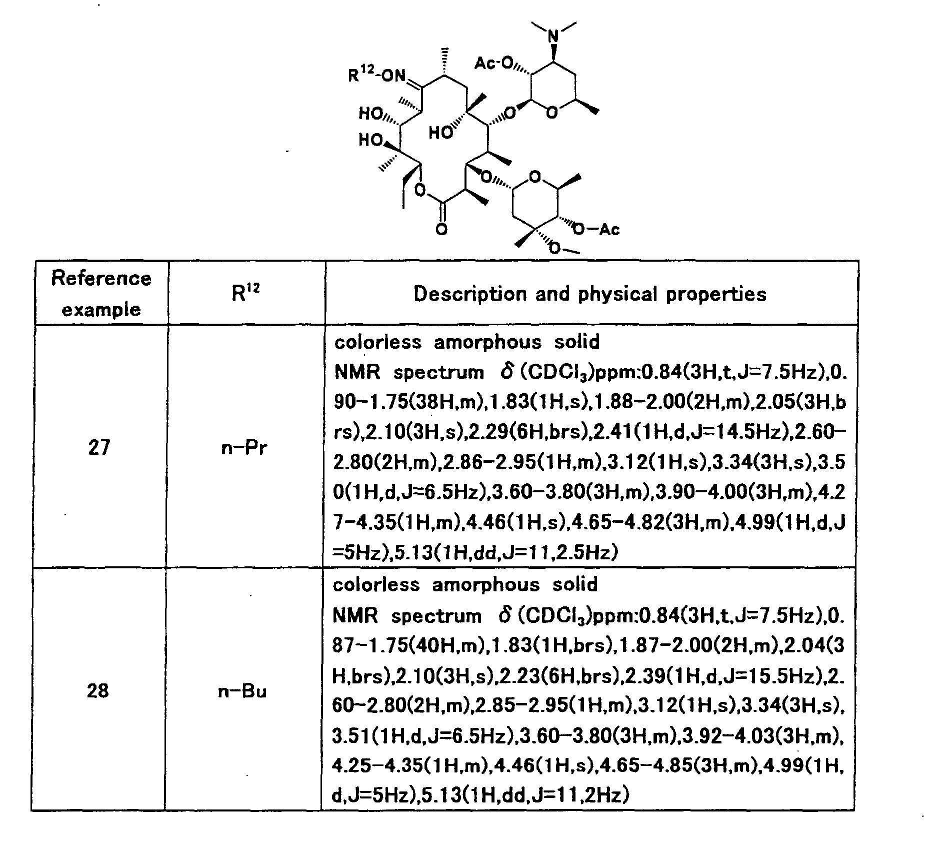

### Reference Example 29: 4"-O-Acetylerythromycin A 9-(O-ethyloxime)

A solution of 0.15 g of 2',4"-O-diacetylerythromycin A 9-(O-ethyloxime) in 3 ml of methanol was stirred at room temperature for 64 hours. The reaction mixture was concentrated under reduced pressure to give 0.10 g of a pale brown amorphous solid.
NMR spectrum δ (CDCl₃) ppm: 0.84 (3H, t, J=7.5Hz), 0.97-2.07 (39H, m), 2.11 (3H, s), 2.31 (6H, s), 2.42 (1H, d, J=14.5Hz), 2.50-2.60 (1H, m), 2.63-2.70 (1H, m), 2.87-2.97 (1H, m), 3.10 (1H, s), 3.22 (1H, dd, J=10.5, 7.5Hz), 3.31 (3H, s), 3.47 (1H, s), 3.59 (1H, d, J=6.5Hz), 3.62-3.80 (3H, m), 4.00-4.11 (3H, m), 4.30-4.41 (1H, m), 4.46 (1H, s), 4.57 (1H, d, J=7.5Hz), 4.68 (1H, d, J=10Hz), 4.99 (1H, d, J=5Hz), 5.13 (1H, dd, J=11, 2Hz)

The compounds of Reference Examples 30 and 31 were obtained in the same manner as that described in Reference Example 29.

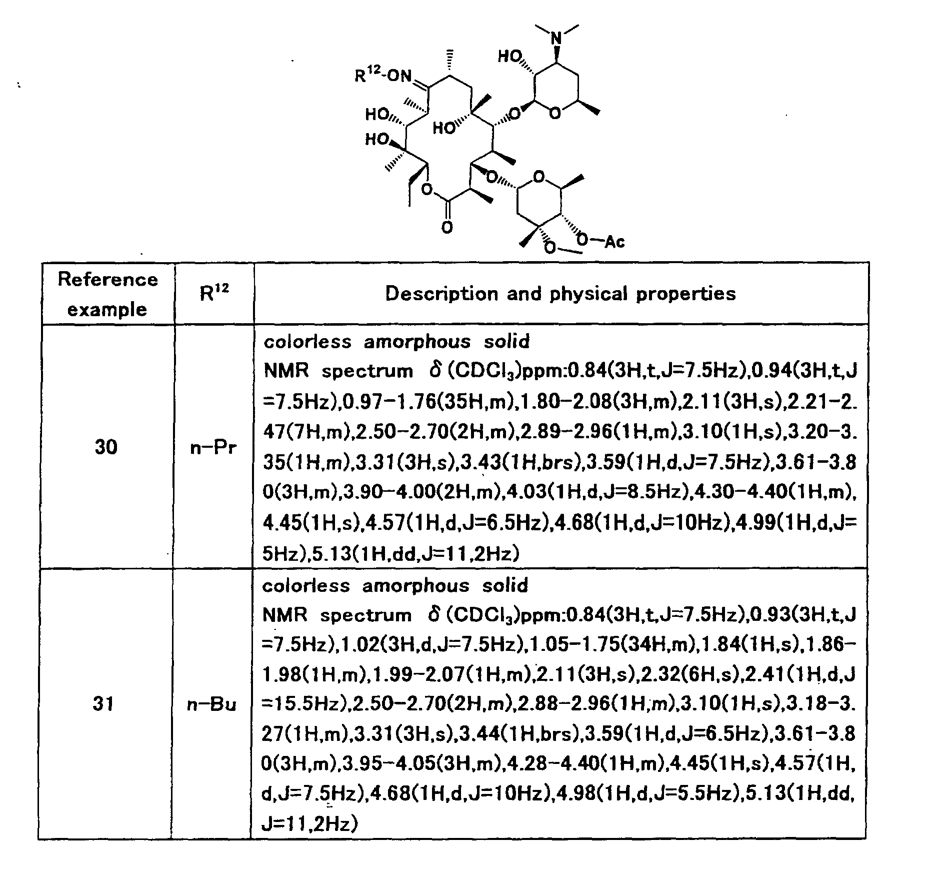

### Example 1: 2',4"-O-Diacetylerythromycin A 9-[O-(3-phenoxypropyl)oxime]

### Method a)

To a solution of 0.50 g of erythromycin A 9-[O-(3-phenoxypropyl)oxime] in 2.3 ml of pyridine, 0.53 ml of acetic anhydride was added dropwise, and the mixture was stirred at room temperature for 29 hours. The reaction mixture was added with water, and the mixture was made alkaline with saturated aqueous sodium hydrogencarbonate solution and extracted with diethyl ether. The extract was washed successively with water and saturated brine, dried over sodium sulfate, and the solvent was removed under reduced pressure. The residue was purified by column chromatography (silica gel, ethyl acetate) to give 0.21 g of a colorless amorphous solid.
NMR spectrum δ (CDCl₃) ppm: 0.84 (3H, t, J=7.5Hz), 0.95 (3H, d, J=7.5Hz), 1.03 (3H, d, J=7.5Hz), 1.06-1.83 (28H, m), 1.87-2.18 (4H, m), 2.05 (3H, s), 2.10 (3H, s), 2.29 (6H, brs), 2.40 (1H, d, J=14.5Hz), 2.61-2.80 (2H, m), 2.83-2.95 (1H, m), 3.10 (1H, s), 3.34 (1H, s), 3.49 (1H, d, J=6Hz), 3.60-3.80 (3H, m), 3.95 (1H, d, J=10Hz), 4.00-4.08 (2H, m), 4.13-4.34 (3H, m), 4.37 (1H, s), 4.62-4.82 (3H, m), 4.98 (1H, d, J=5Hz), 5.13 (1H, dd, J=11, 2.5Hz), 6.86-6.96 (3H, m), 7.22-7.30 (2H, m)

### Method b)

To a solution of 25.0 g of 2'-O-acetylerythromycin A 9-[O-(3-phenoxypropyl)-oxime] in 250 ml of pyridine, 9.50 ml of acetyl chloride was added dropwise under ice-cooling, and the reaction mixture was stirred at the same temperature for 4 hours and then at room temperature for 2 hours. The reaction mixture was poured into ice-water, and the mixture was made alkaline with saturated aqueous sodium hydrogencarbonate solution and extracted with diethyl ether. The extract was washed successively with water and saturated brine, dried over sodium sulfate, and the solvent was removed under reduced pressure to give a colorless amorphous solid. TLC (Rf value) and NMR spectrum of the resulting solid was identical to the solid obtained in Method a.

The compounds of Examples 2 through 52 were obtained in the same manner as that described in Example 1.

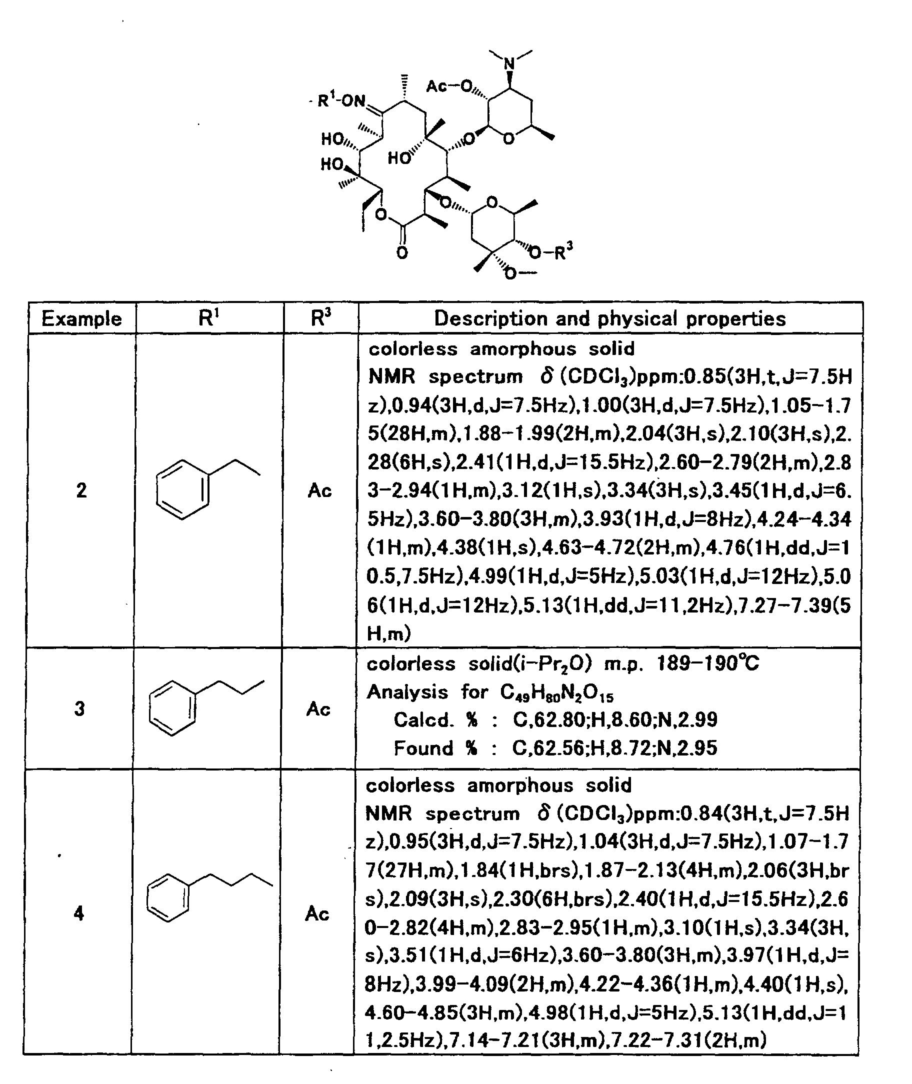

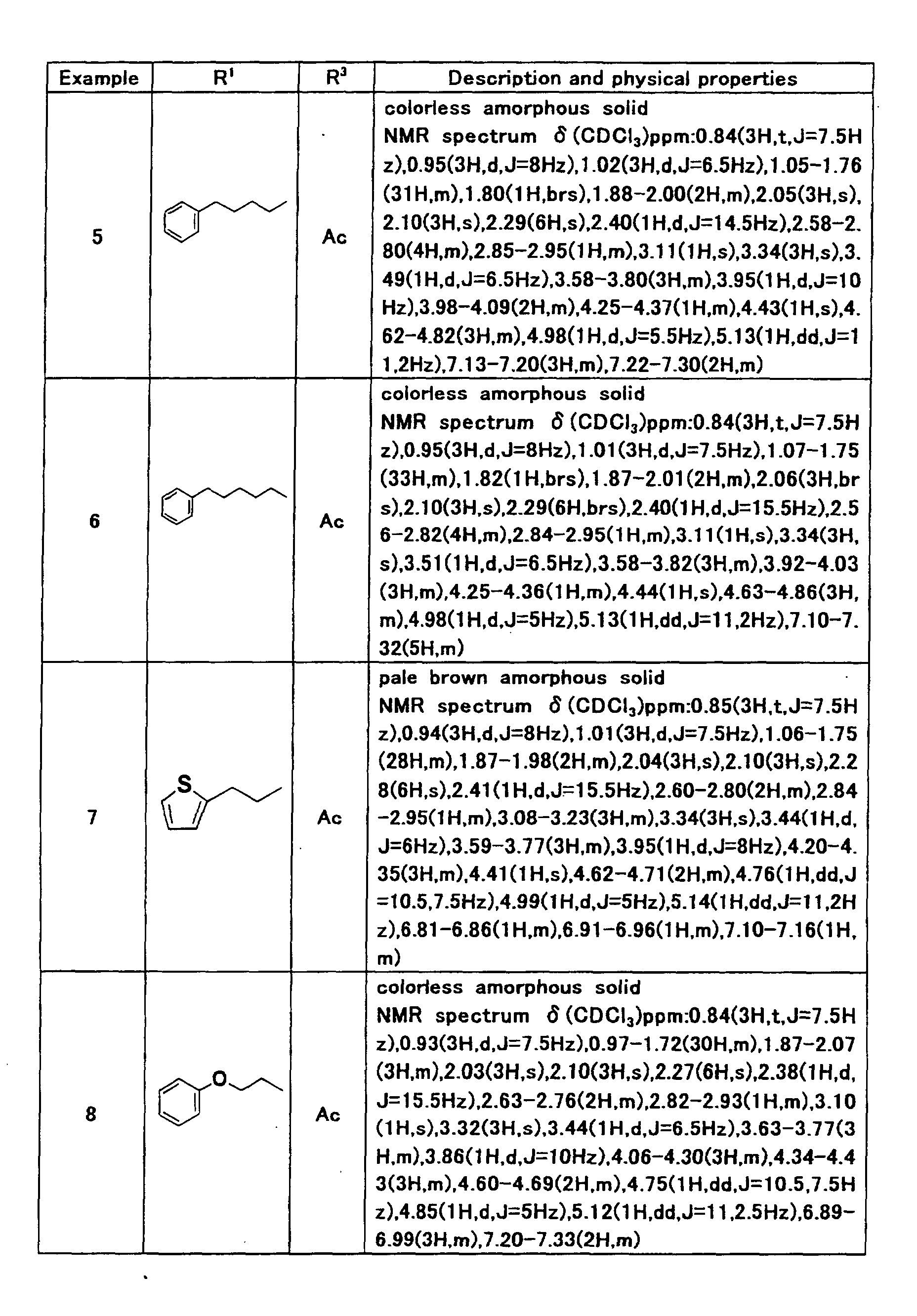

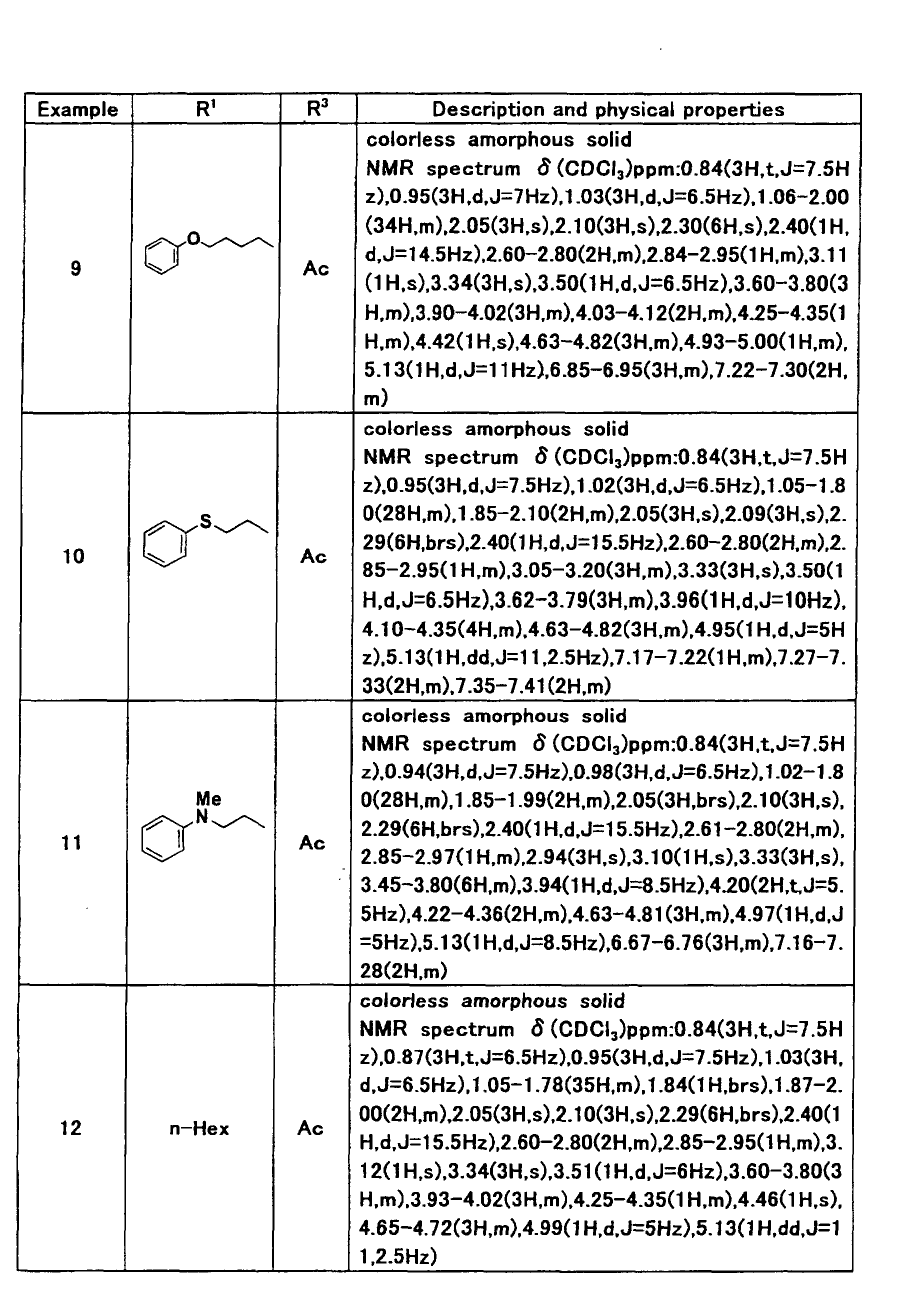

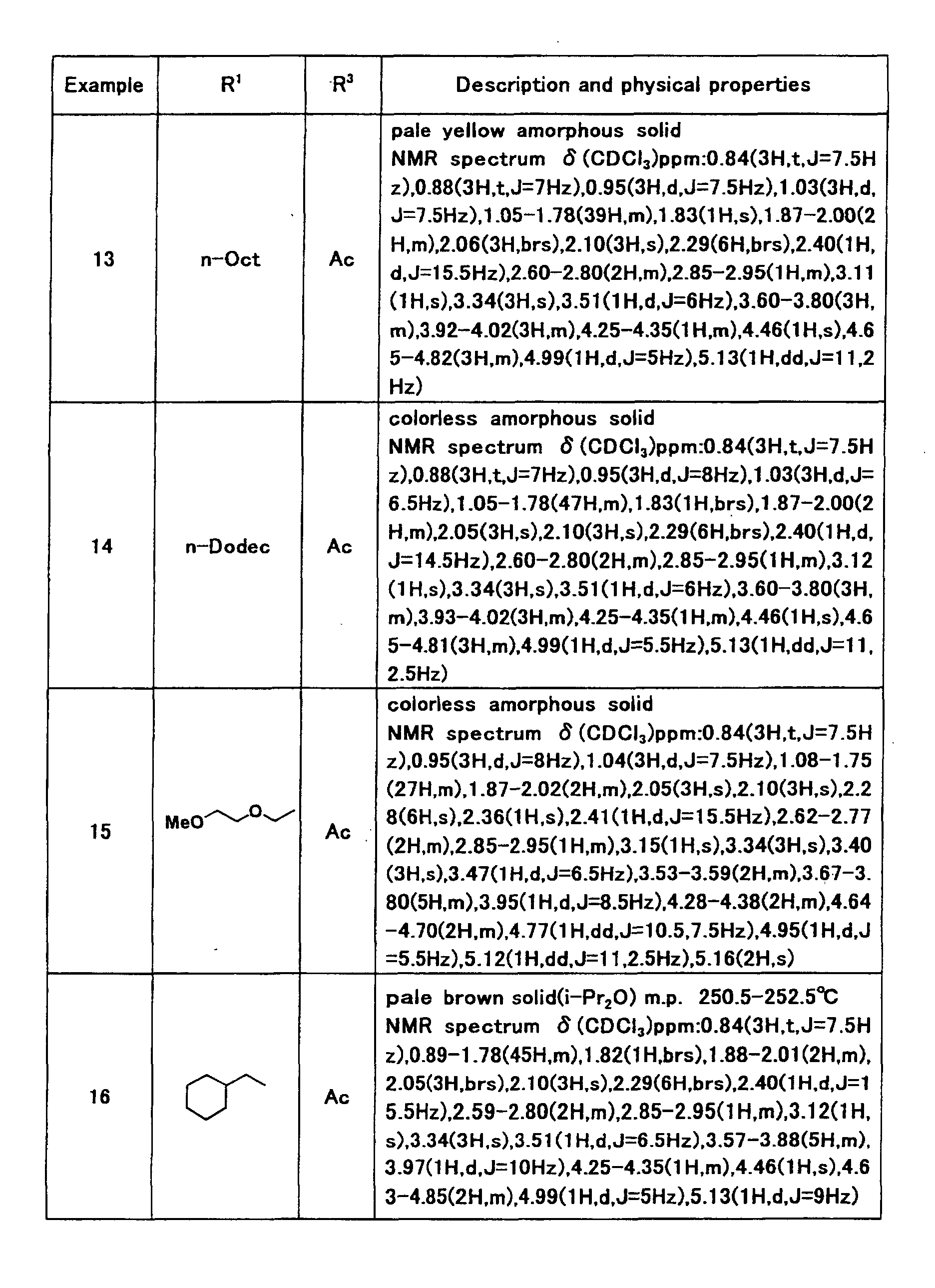

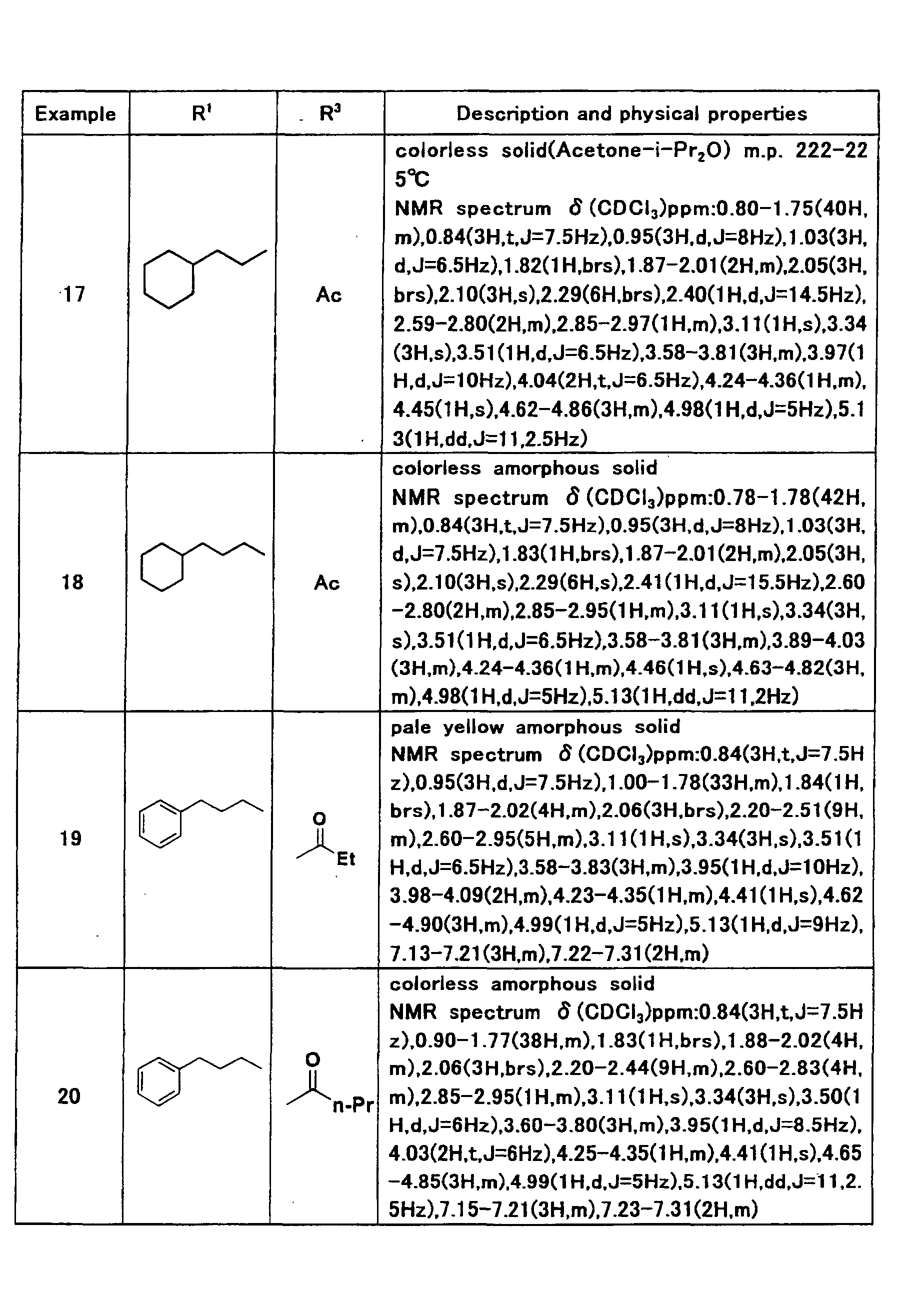

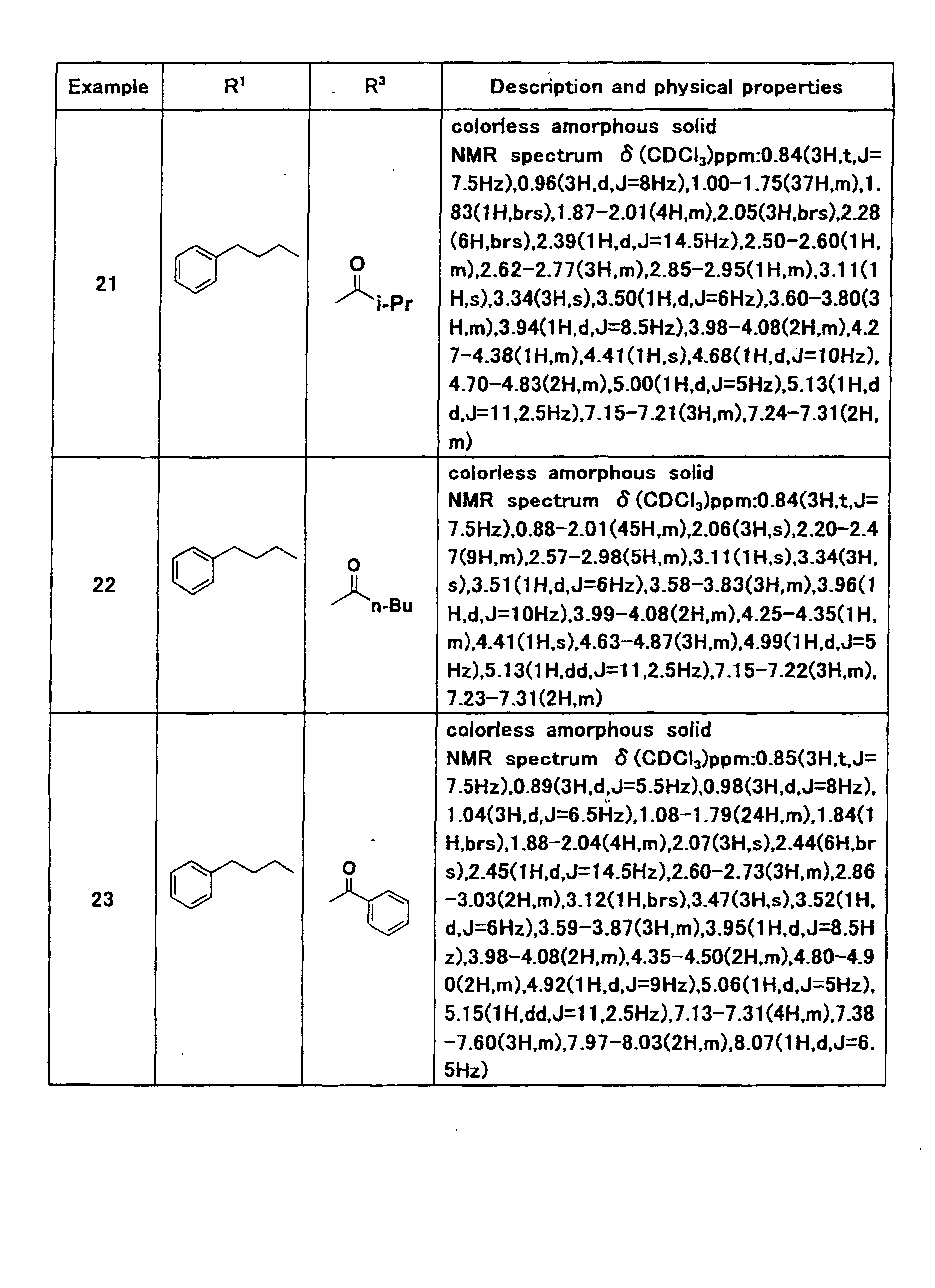

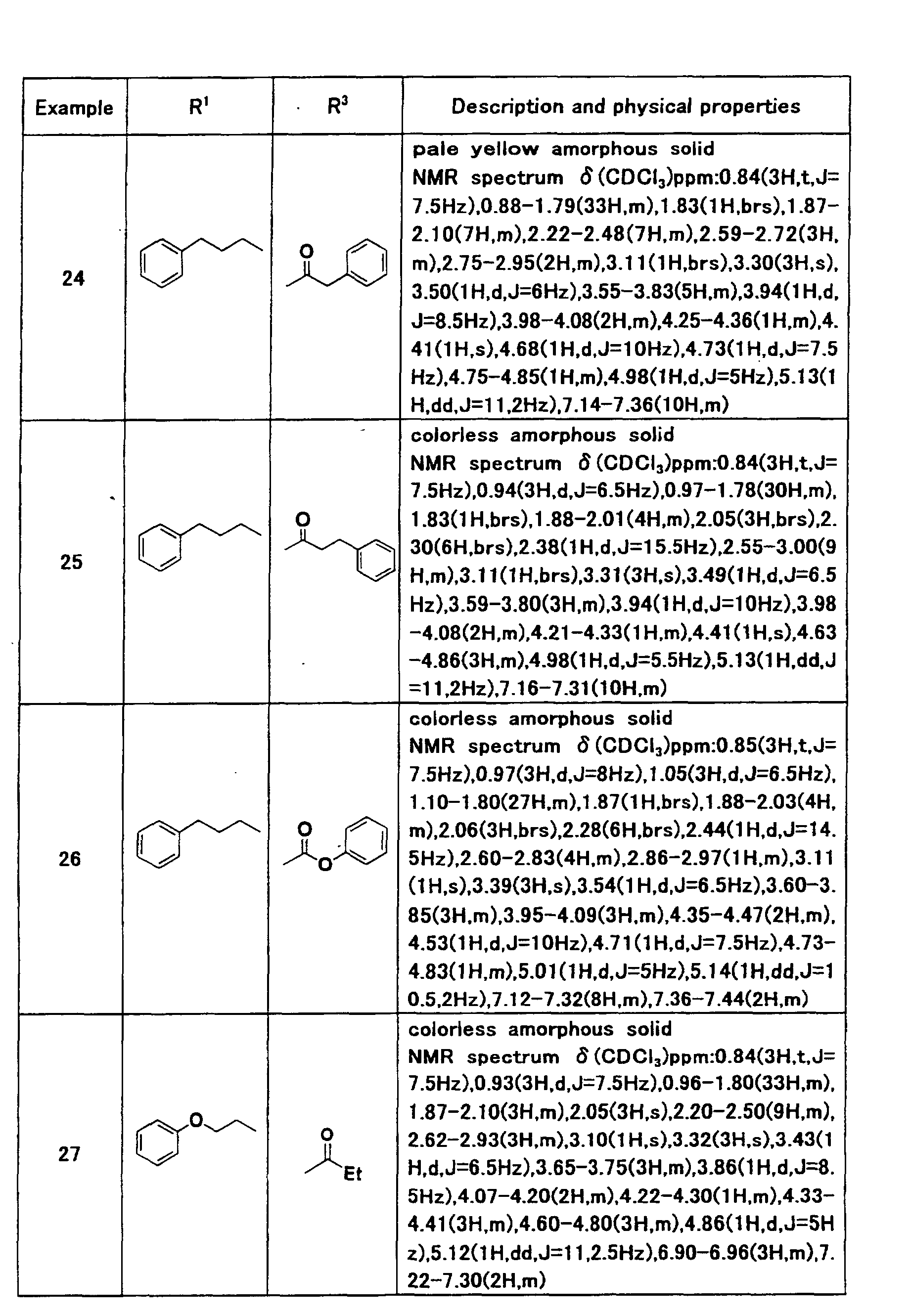

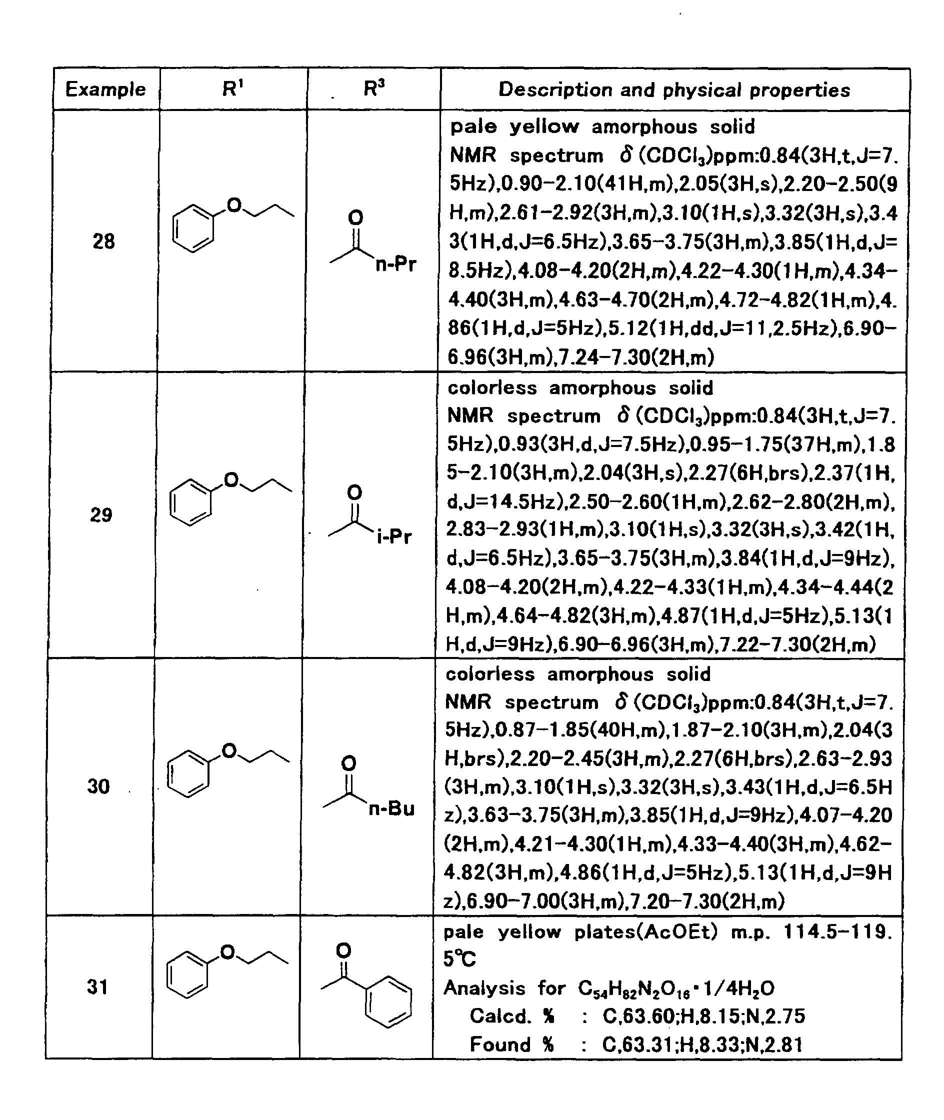

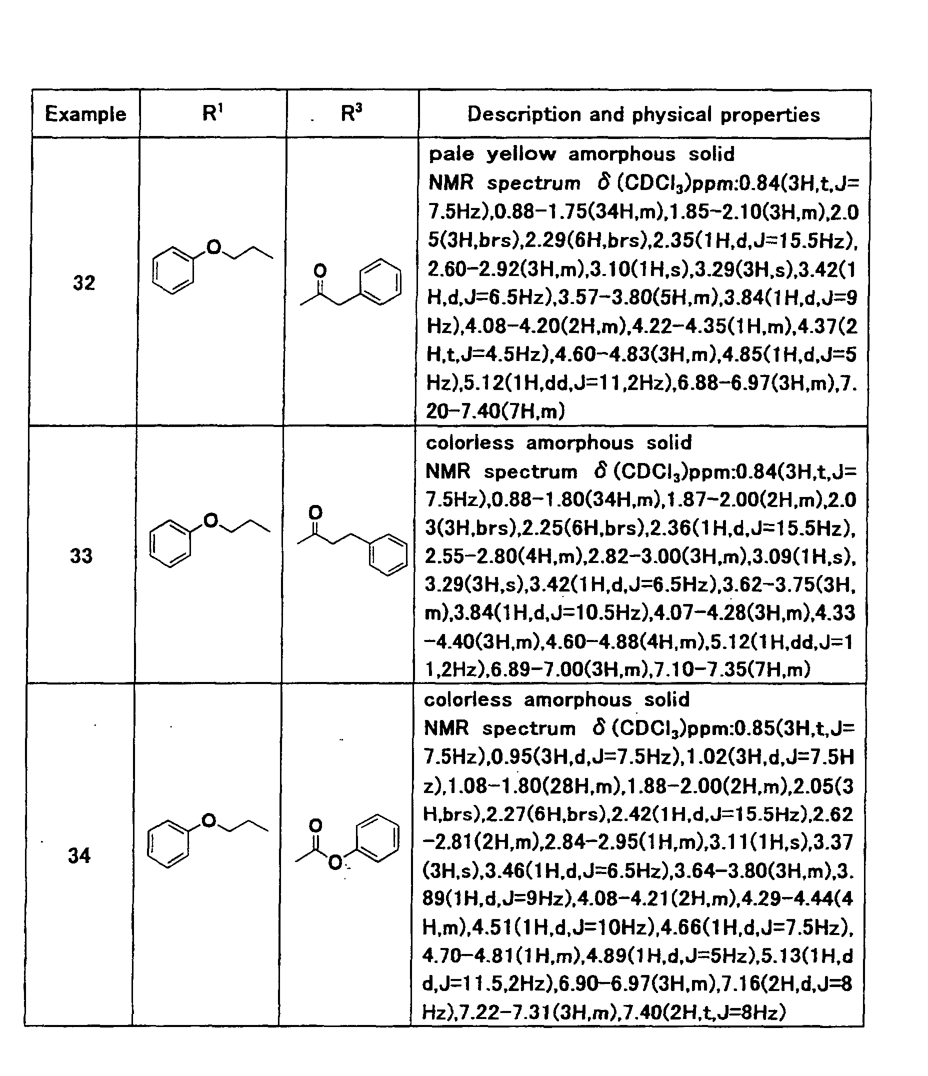

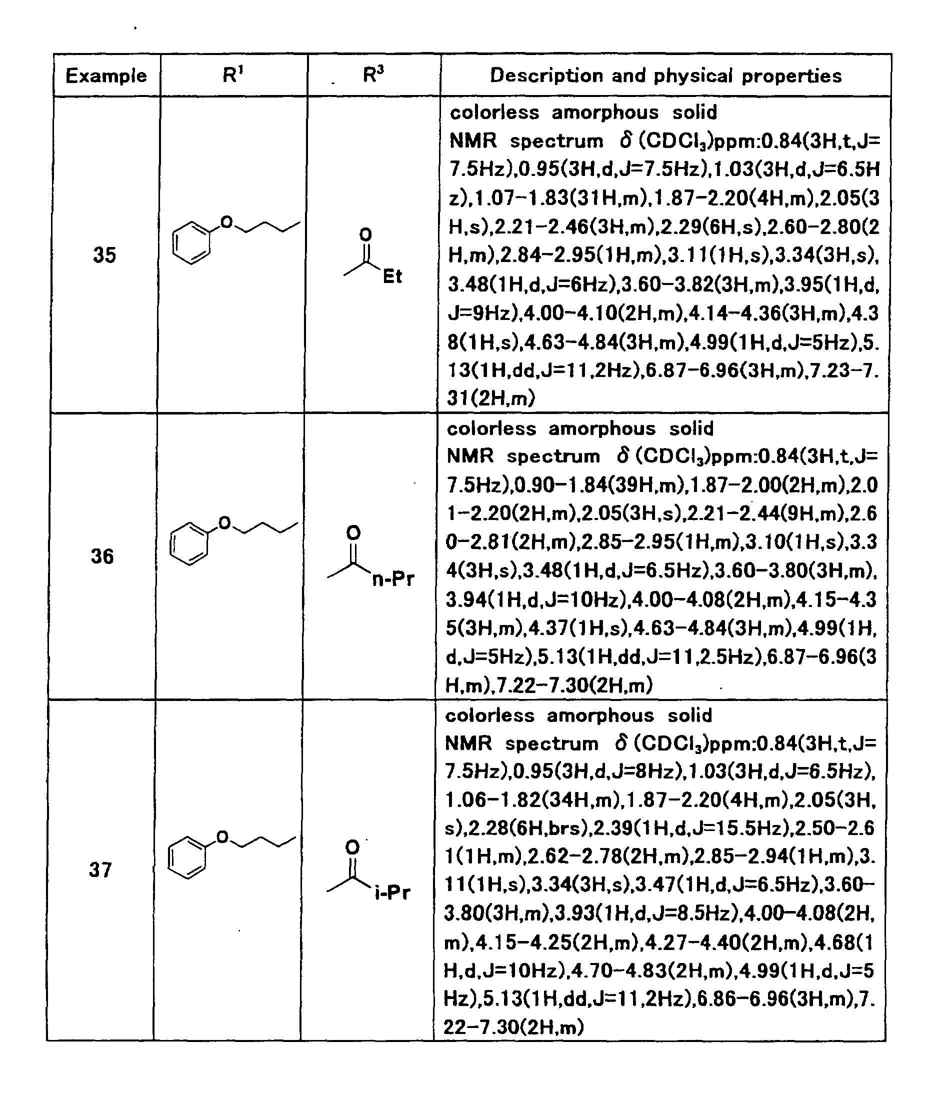

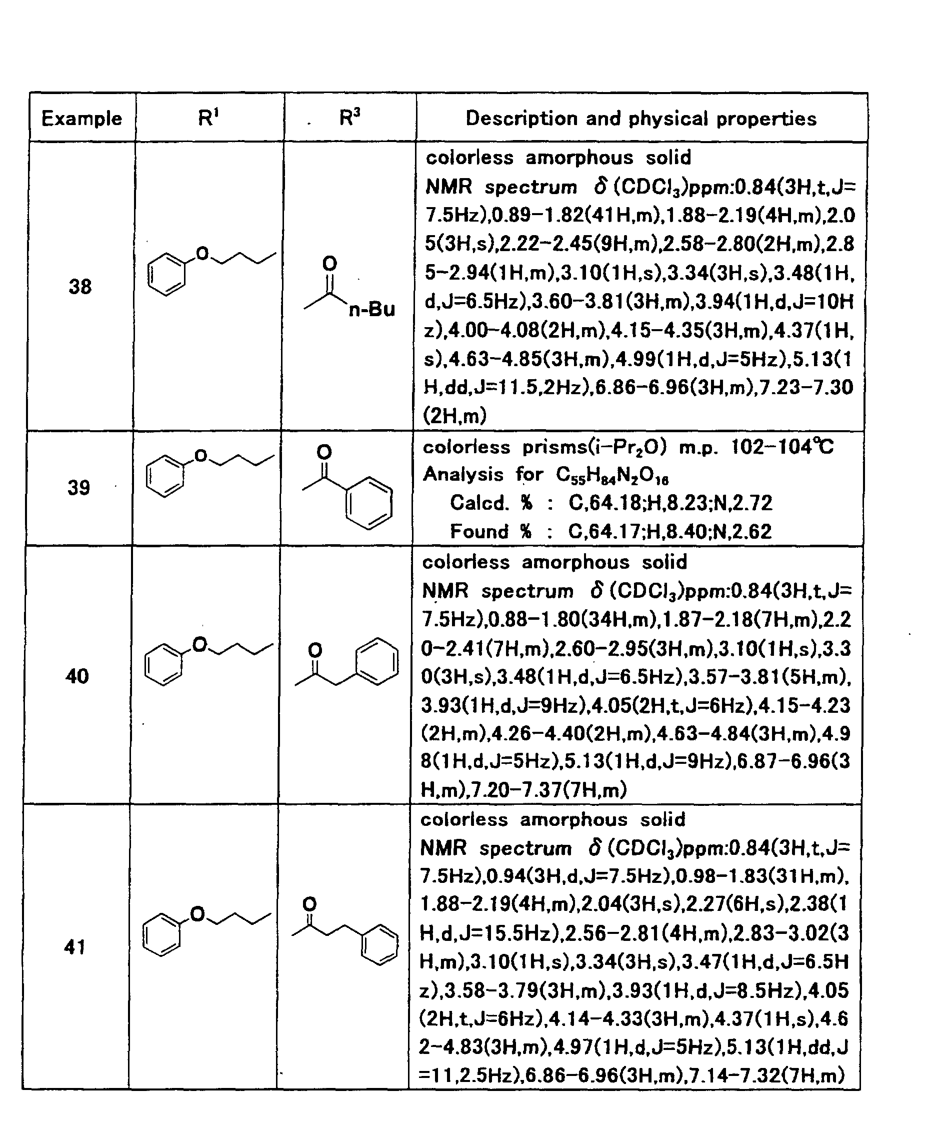

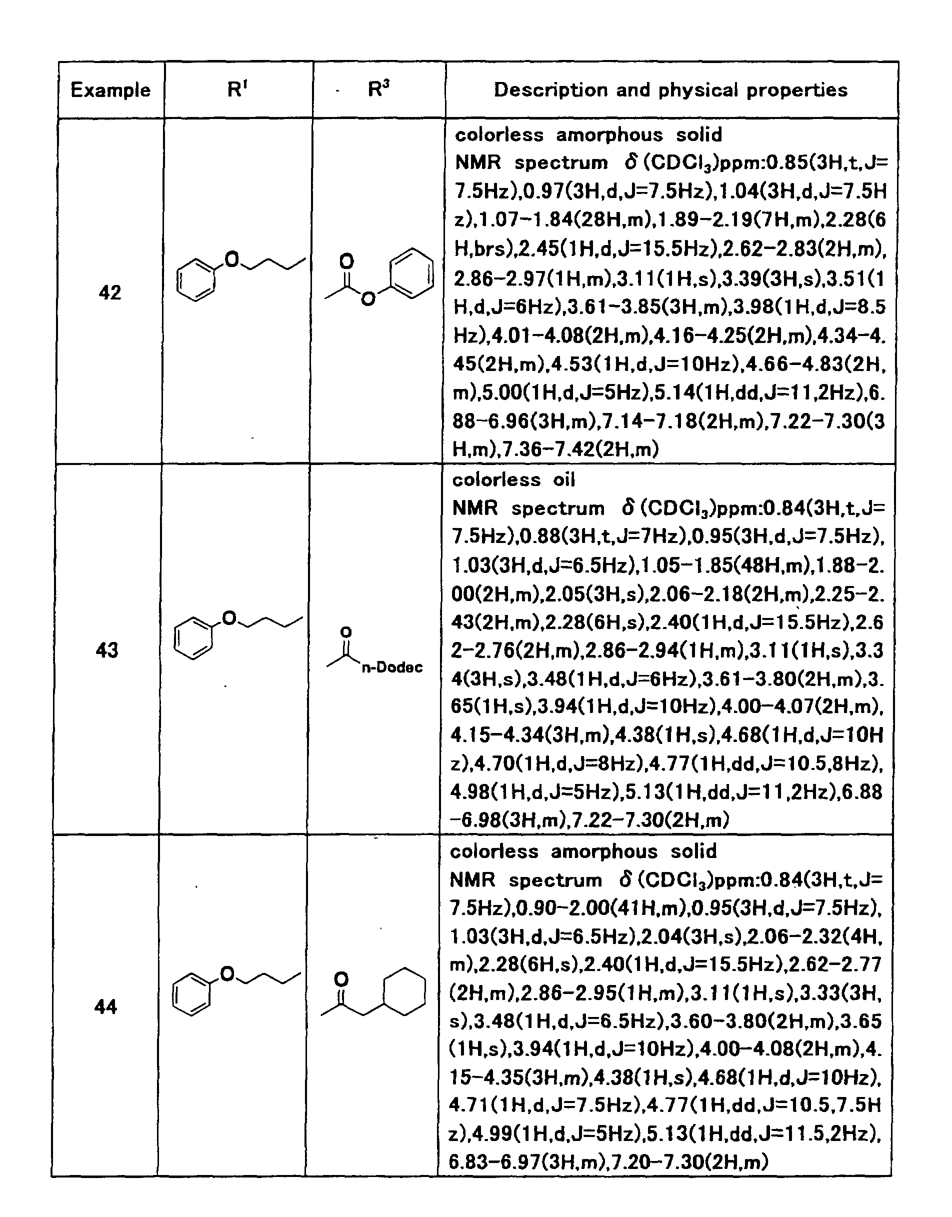

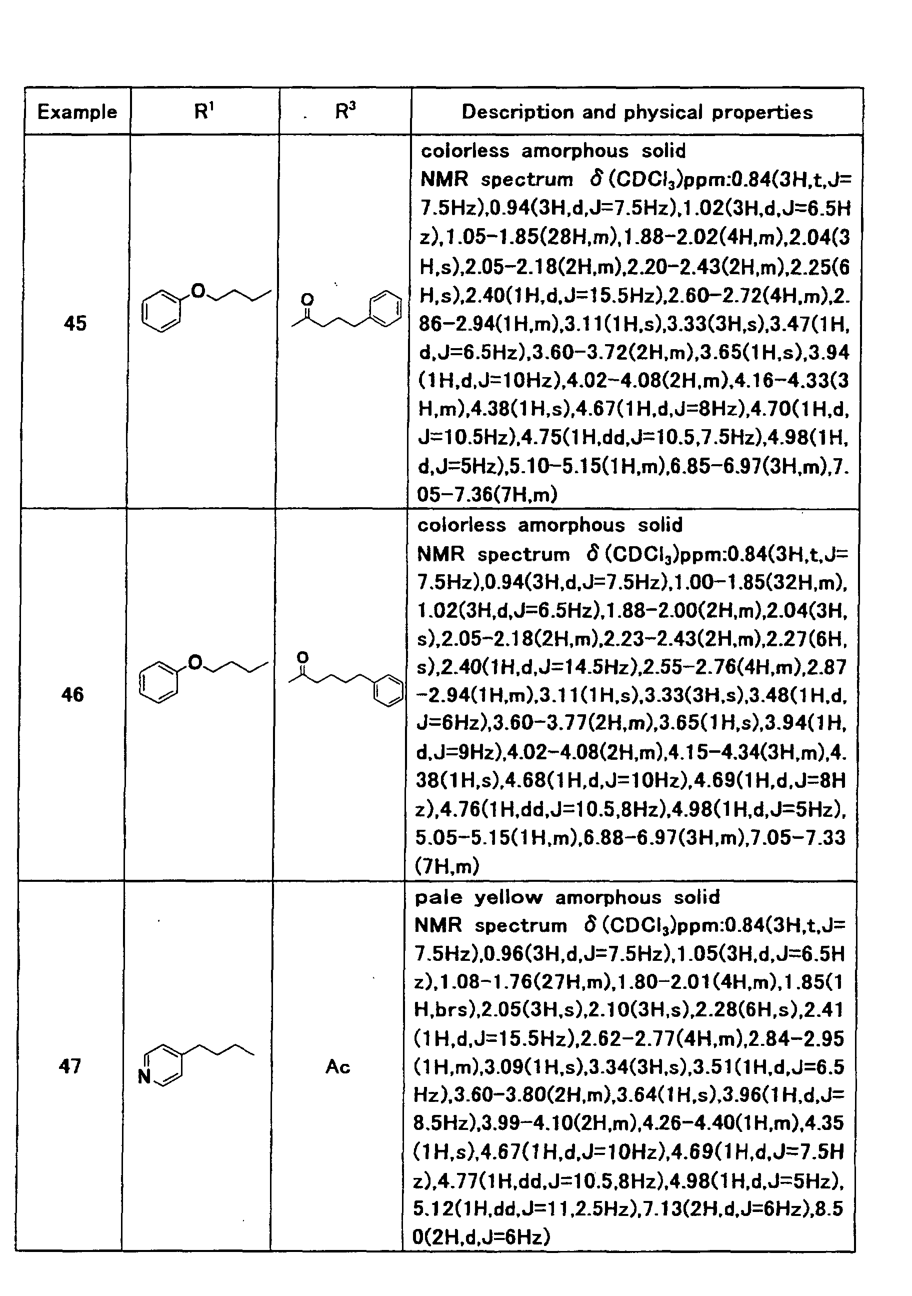

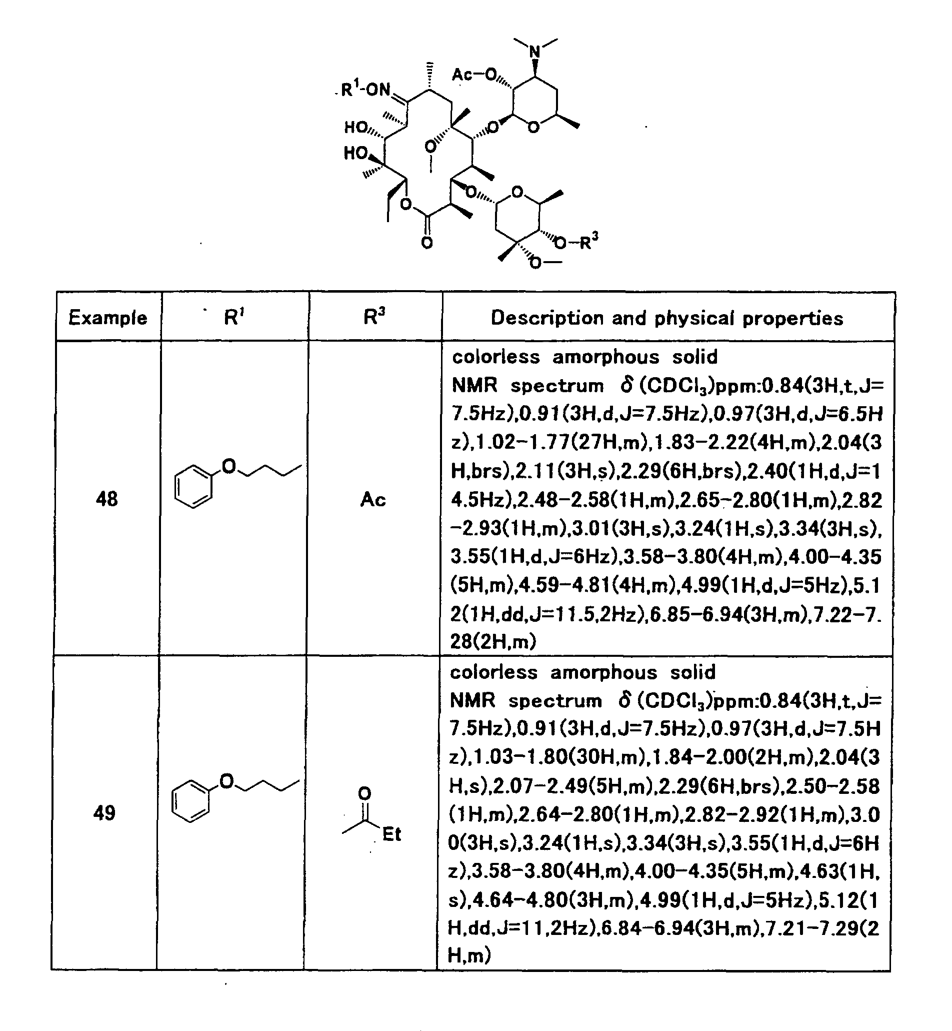

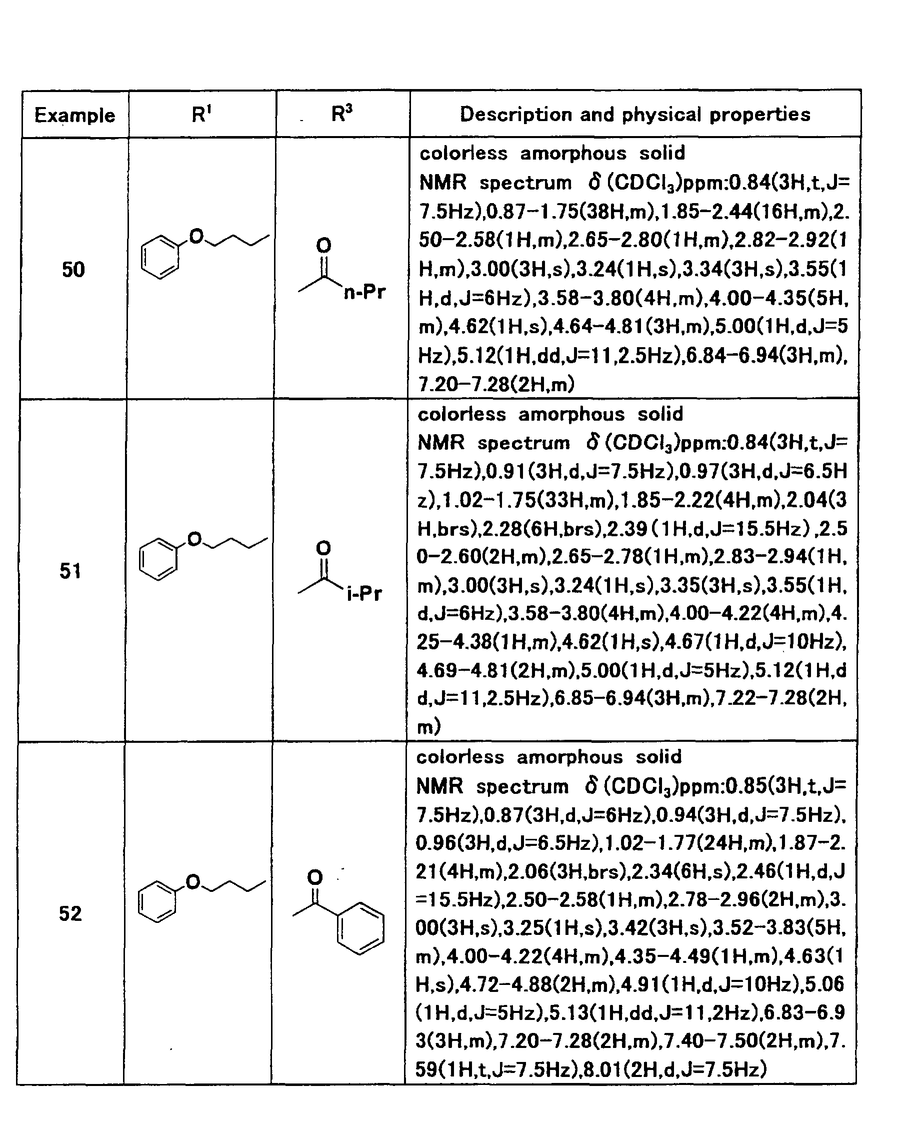

### Example 53:2'-O-Acetyl-4"-O-phenylaminocarbonylerythromycin A 9-[O-(3-phenoxypropyl)oxime]

To a solution of 1.20 g of 2'-O-acetylerythromycin A 9-[O-(3-phenoxypropyl)oxime] in 6 ml of pyridine, 0.63 ml of phenyl isocyanate was added dropwise, and the mixture was stirred at room temperature for 25 hours. The reaction mixture was added with water, and the mixture was made alkaline with saturated aqueous sodium hydrogencarbonate solution and extracted with diethyl ether. The extract was successively washed with water and saturated brine, dried over sodium sulfate, and the solvent was removed under reduced pressure. The residue was purified by column chromatography (silica gel, ethyl acetate) and purified again by column chromatography (silica gel, n-heptane:acetone:triethylamine= 7 : 3 : 0.15) to give 0.95 g of a colorless amorphous solid.
NMR spectrum δ (CDCl₃) ppm: 0.85 (3H, t, J=7.5Hz), 0.95 (3H, d, J=7.5Hz), 1.03 (3H, d, J=6.5Hz), 1.07-1.86 (28H, m), 1.88-2.00 (2H, m), 2.02-2.19 (2H, m), 2.08 (3H, s), 2.34 (6H, s), 2.44 (1H, d, J=15.5Hz), 2.61-2.69 (1H, m), 2.72-2.94 (2H, m), 3.11 (1H, s), 3.37 (3H, s), 3.52 (1H, d, J=6.5Hz), 3.60-3.75 (3H, m), 3.99 (1H, d, J=9Hz), 4.01-4.08 (2H, m), 4.16-4.25 (2H, m), 4.28-4.36 (1H, m), 4.38 (1H, s), 4.59-4.69 (2H, m), 4.73-4.83 (1H, m), 4.97 (1H, d, J=5Hz); 5.13 (1H, dd, J=11, 2Hz), 6.73 (1H, brs), 6.87-6.95 (3H, m), 7.09 (1H, t, J=7.5Hz), 7.23-7.29 (2H, m), 7.32 (2H, t, J=8Hz), 7.40 (2H, d, J=8Hz)

### Example 54: 4"-O-Acetylerythromycin A 9-[O-(3-phenoxypropyl)oxime]

A solution of 0.13 g of 2',4"-O-diacetylerythromycin A 9-[O-(3-phenoxypropyl)-oxime] in 10 ml of methanol was stirred at room temperature for 4 days. The reaction mixture was concentrated under reduced pressure to give 0.12 g of a colorless solid. The solid was recrystallized from a mixture of methanol and water to give colorless prisms having a melting point of 127-130°C.
NMR spectrum δ (CDCl₃) ppm: 0.85 (3H, t, J=7.5Hz), 0.97-1.83 (35H, m), 1.86-2.18 (4H, m), 2.10 (3H, s), 2.20-2.48 (7H, m), 2.50-2.71 (2H, m), 2.87-2.98 (1H, m), 3.09 (1H, s), 3.18-3.34 (1H, m), 3.31 (3H, s), 3.57 (1H, d, J=6.5Hz), 3.62-3.80 (3H, m), 3.97-4.09 (3H, m), 4.16-4.25 (2H, m), 4.27-4.40 (2H, m), 4.57 (1H, d, J=7.5Hz), 4.68 (1H, d, J=10Hz), 4.98 (1H, d, J=5Hz), 5.13 (1H, d, J=8.5Hz), 6.86-6.96 (3H, m), 7.21-7.30 (2H, m)

The compounds of Examples 55 through 108 were obtained in the same manner as that described in Example 54.

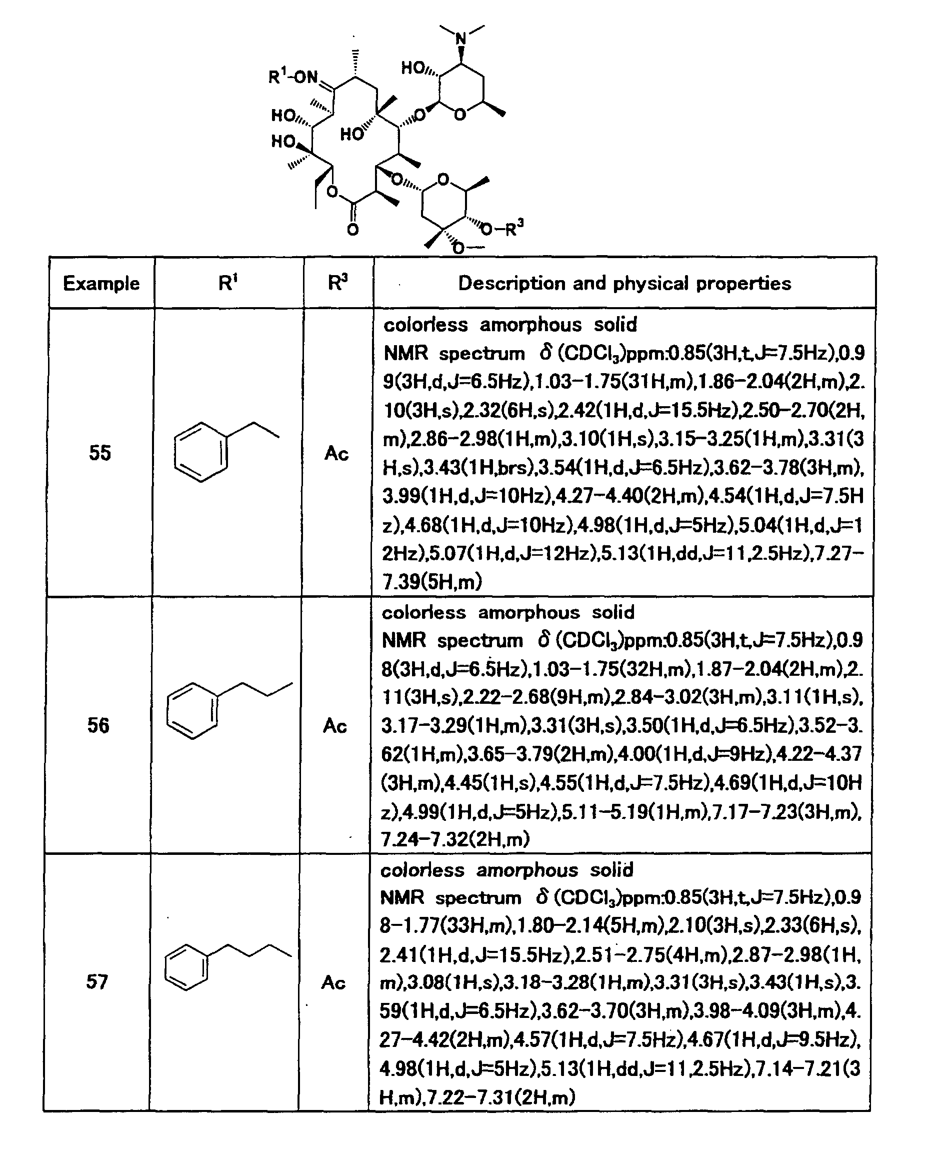

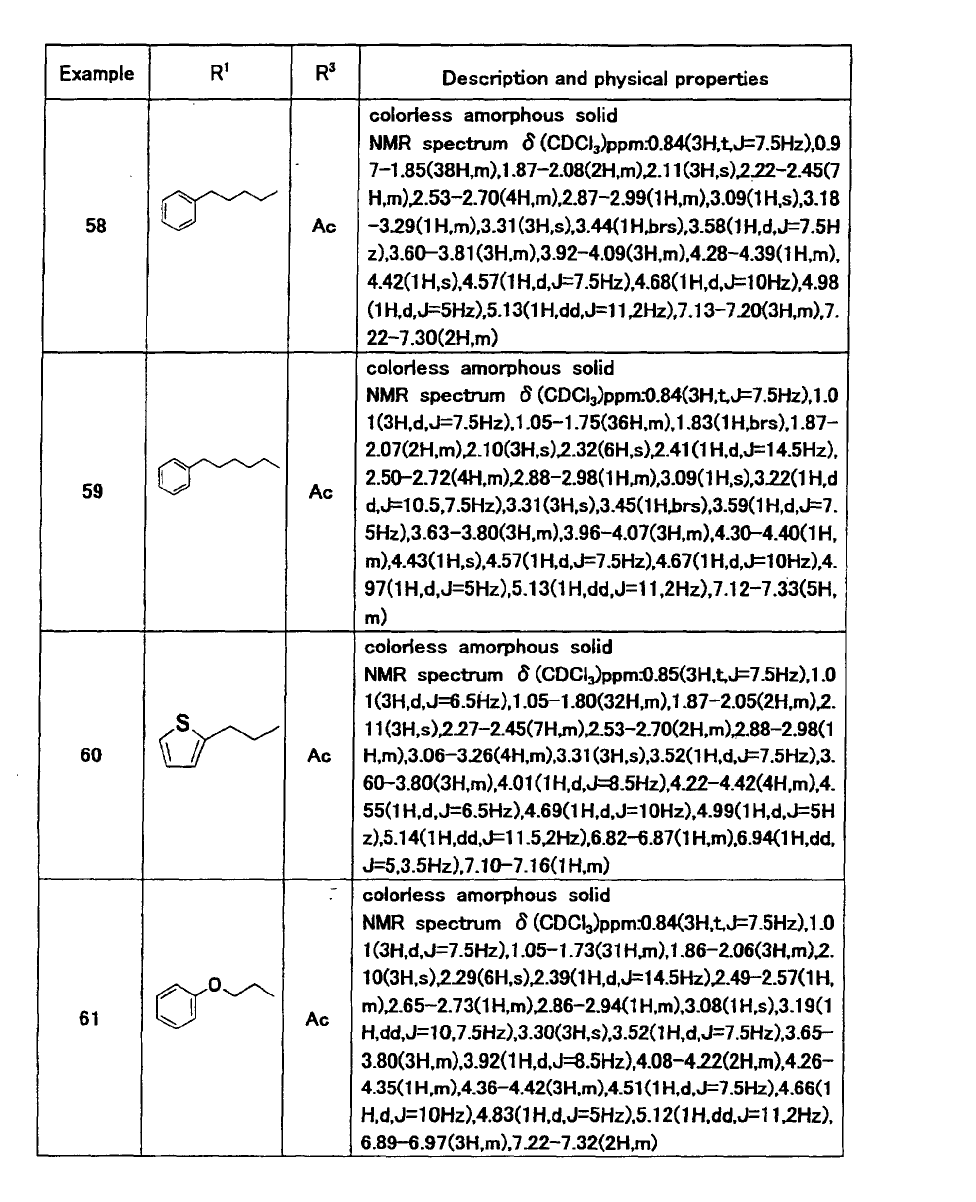

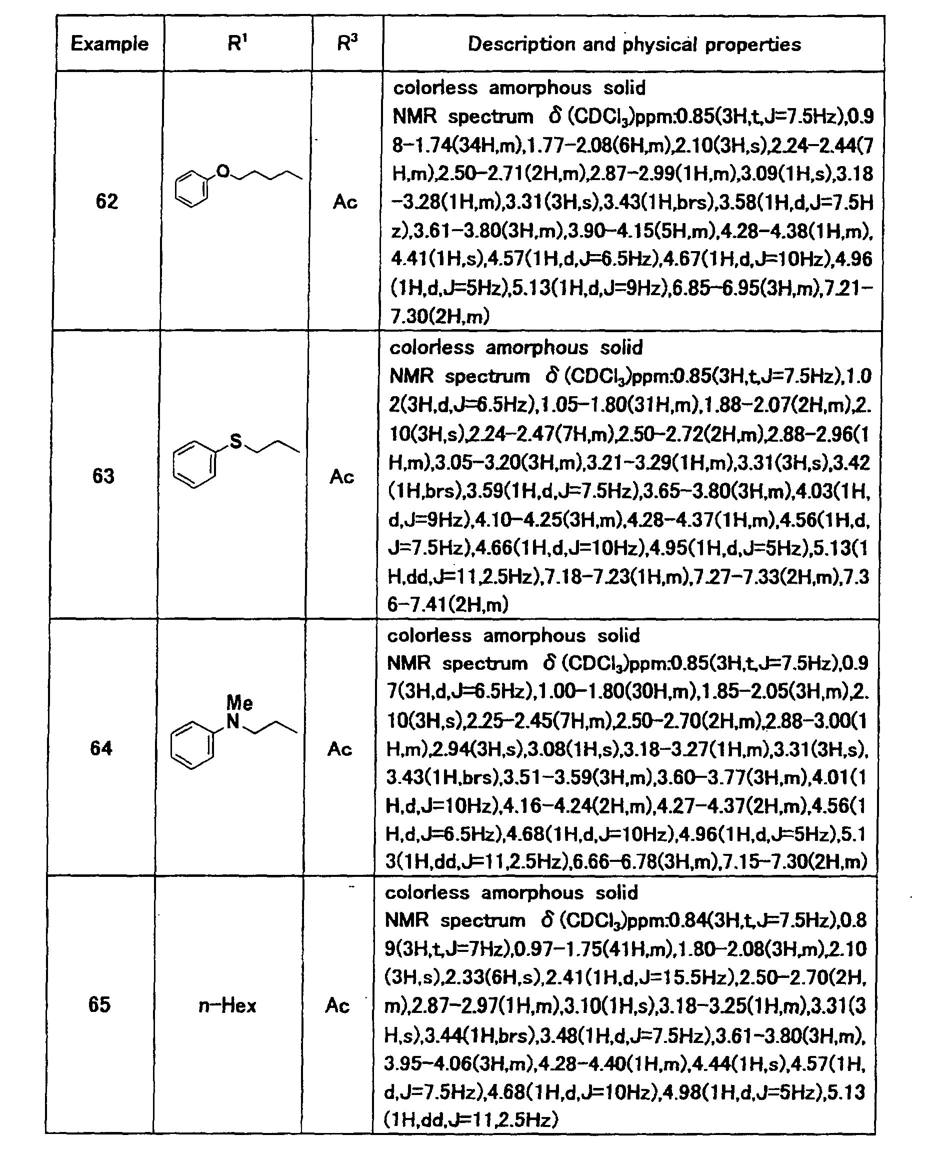

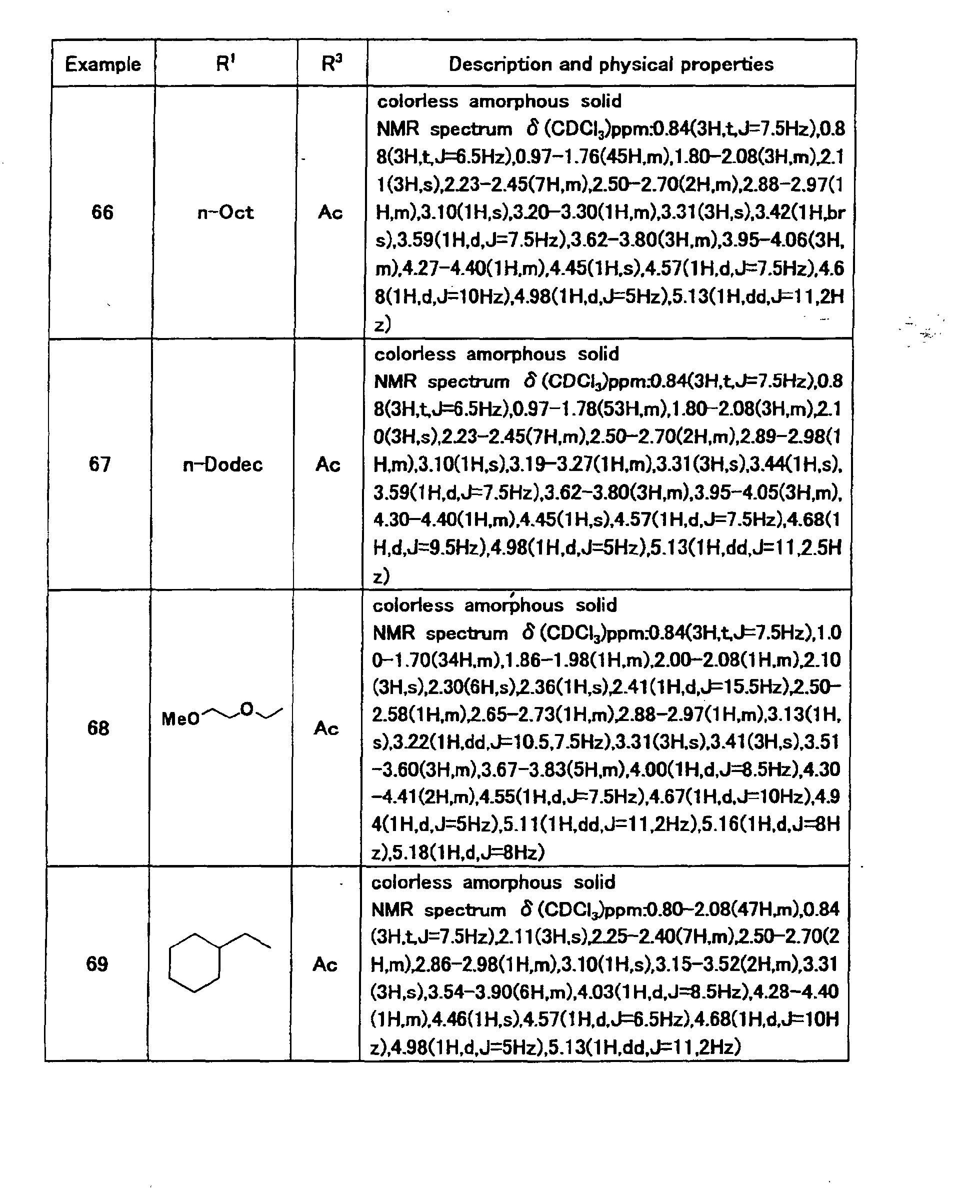

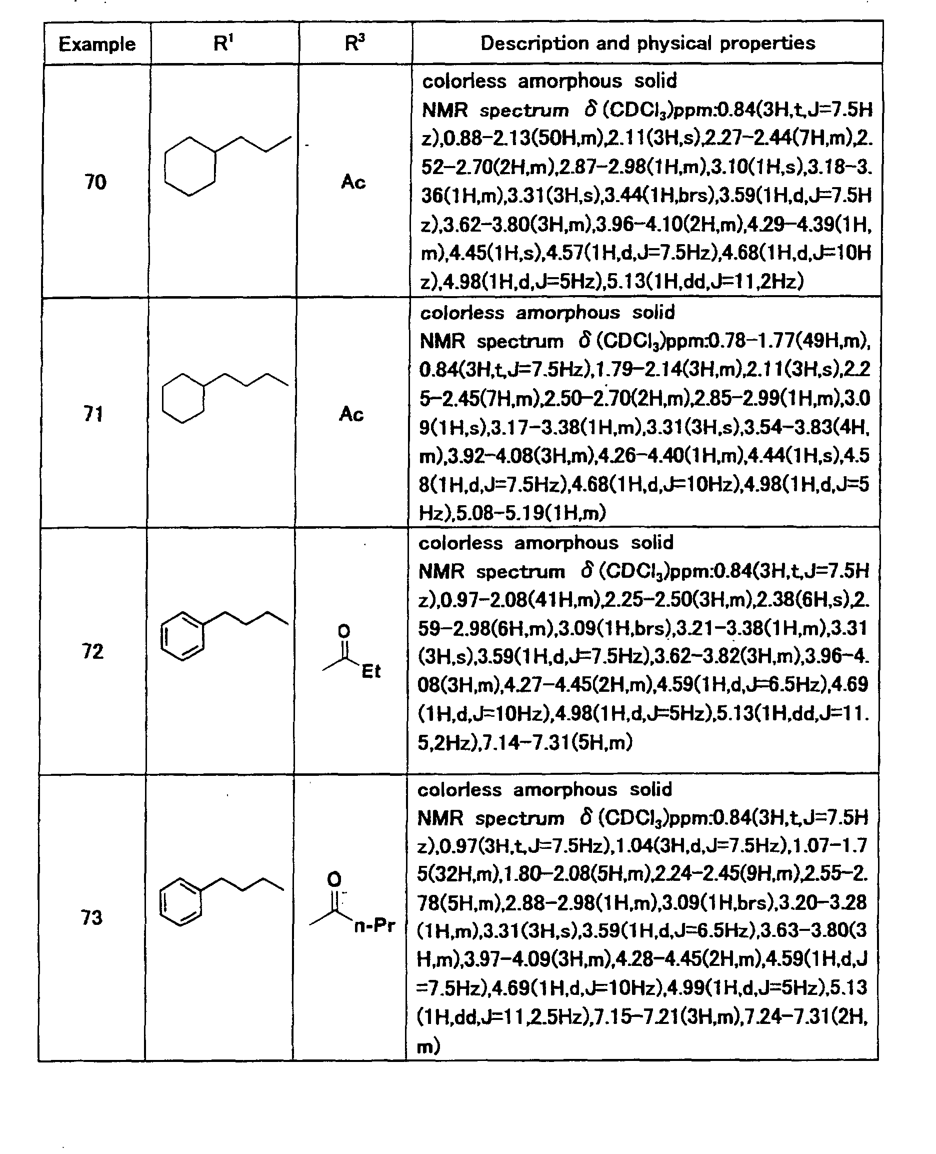

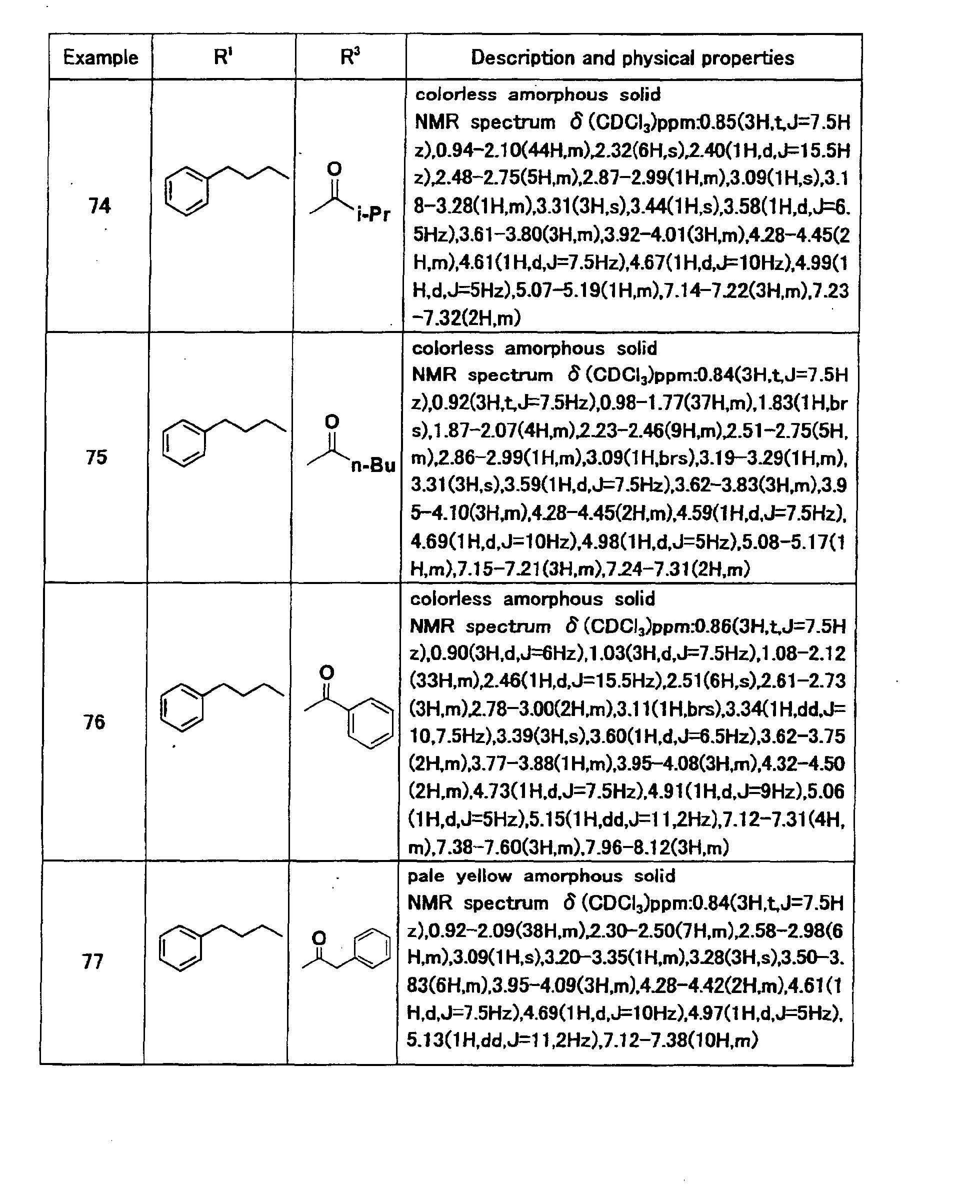

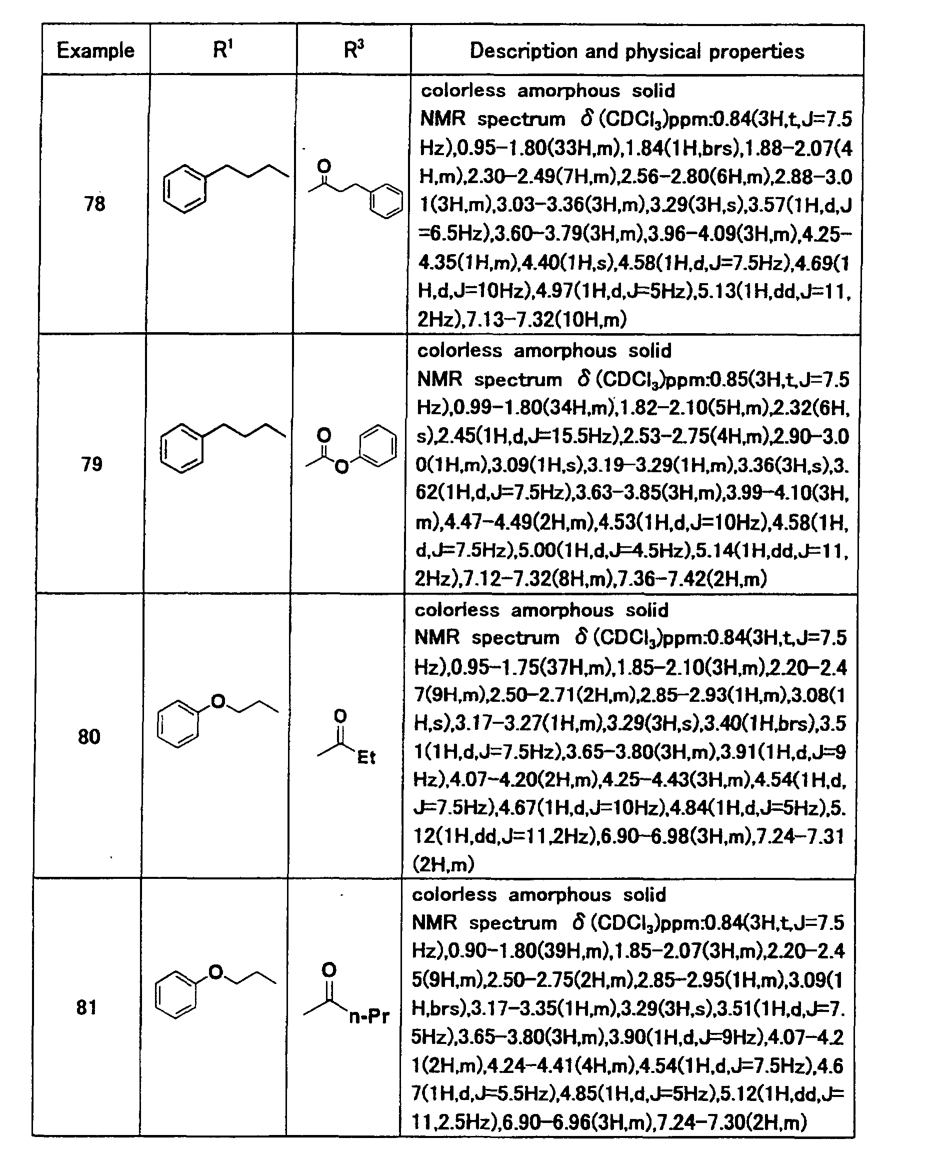

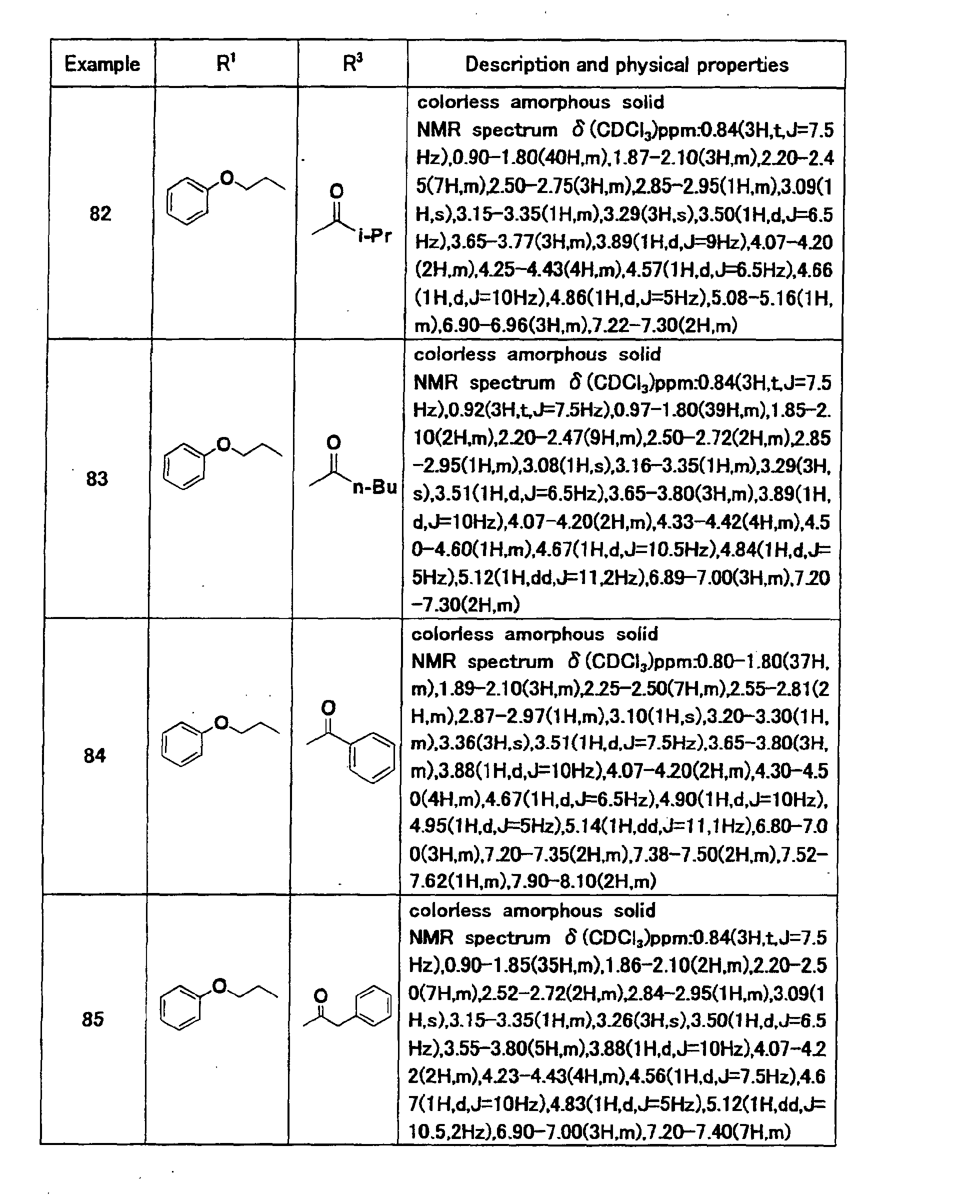

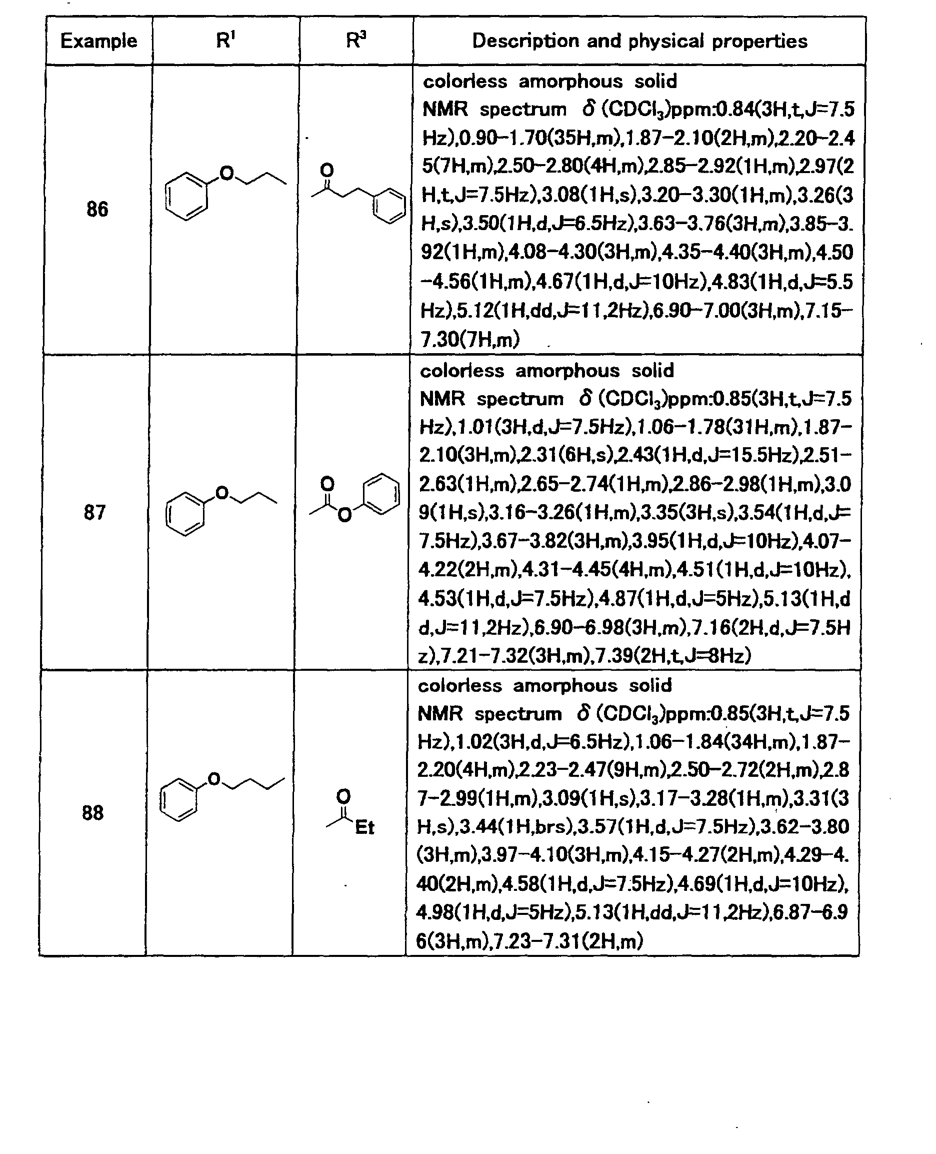

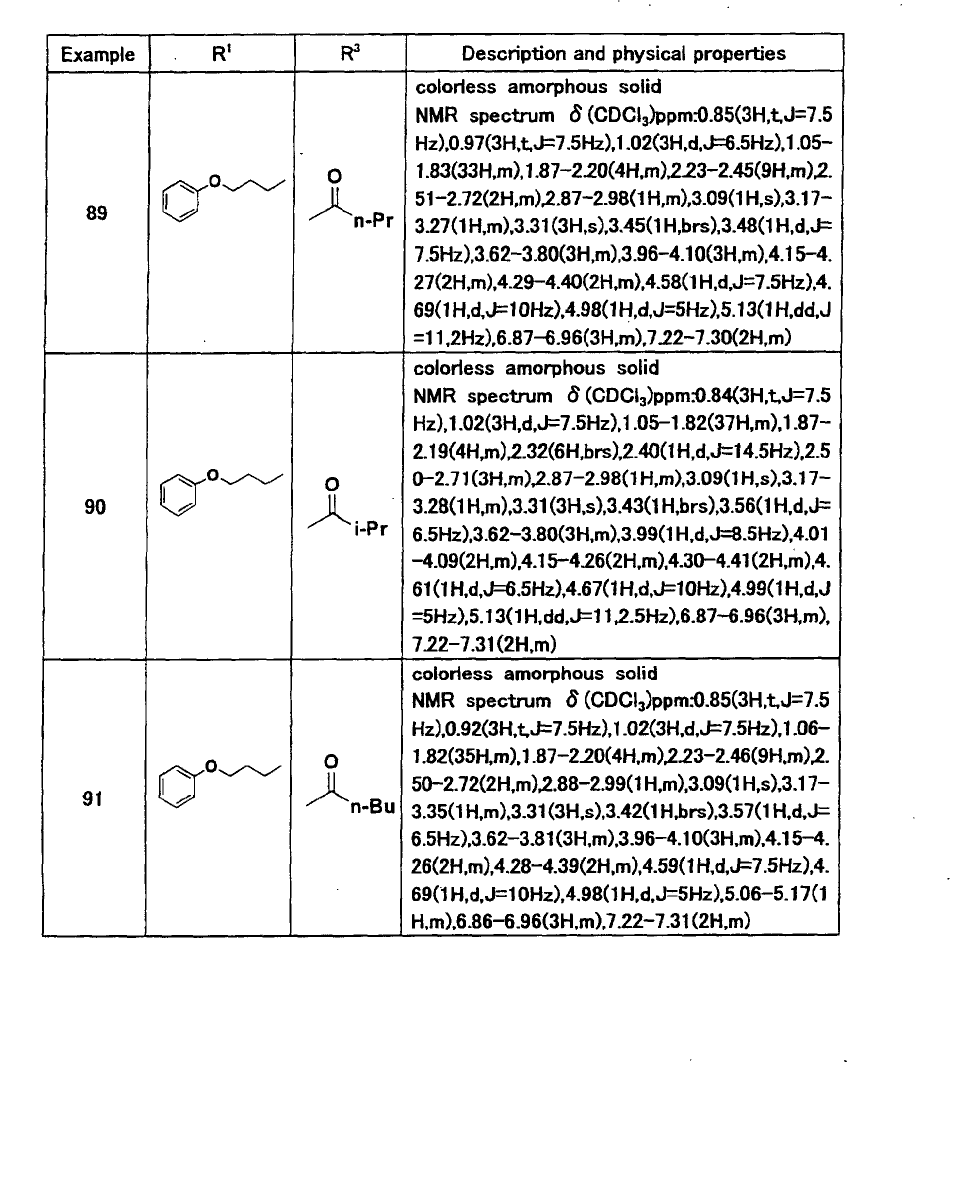

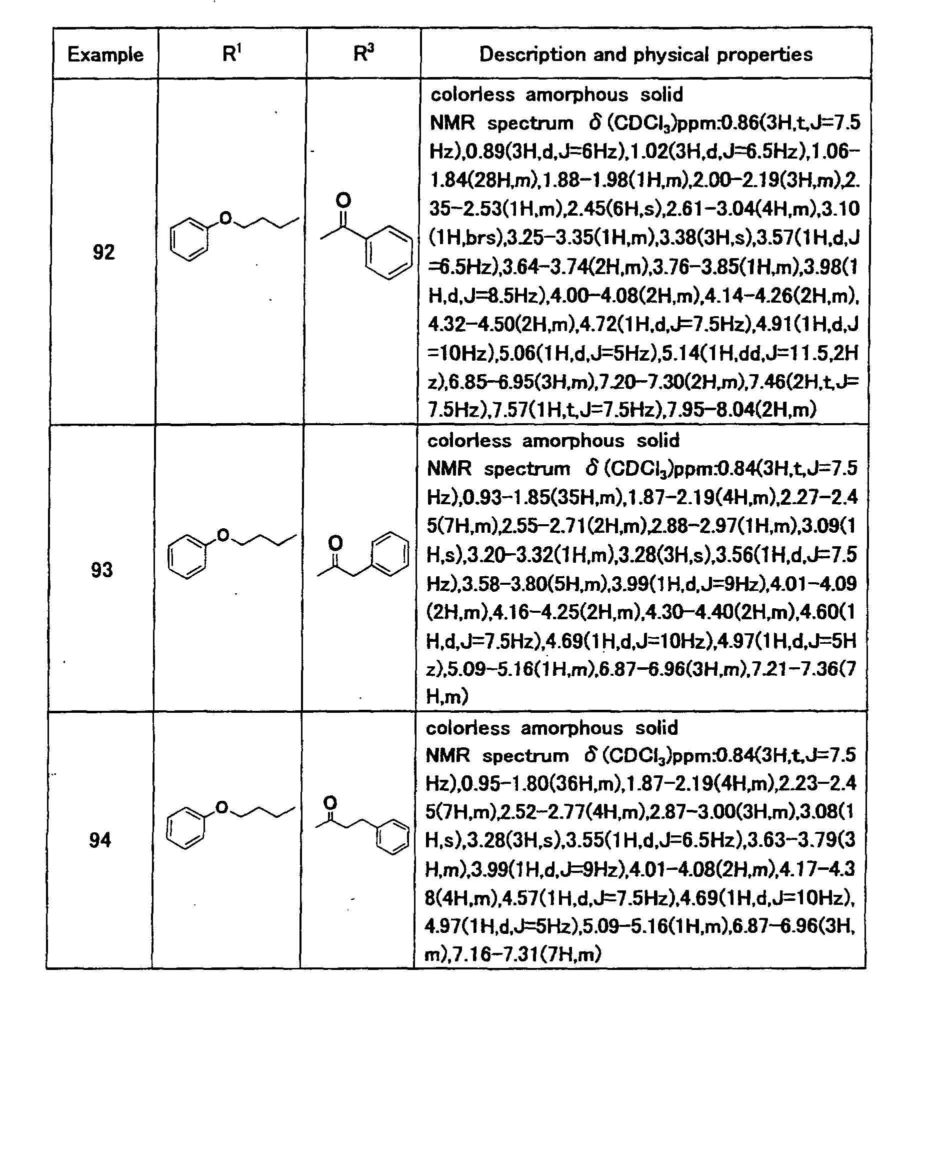

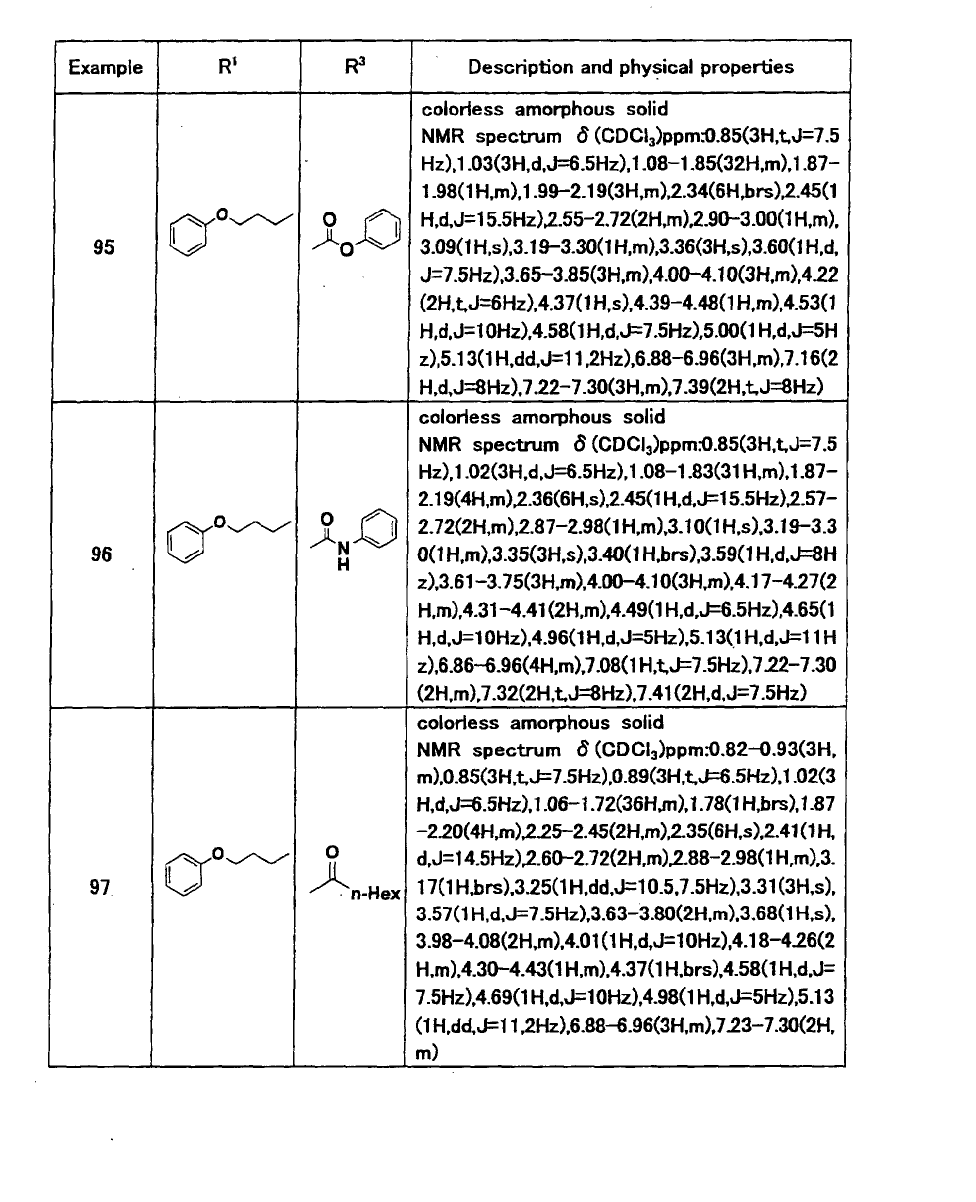

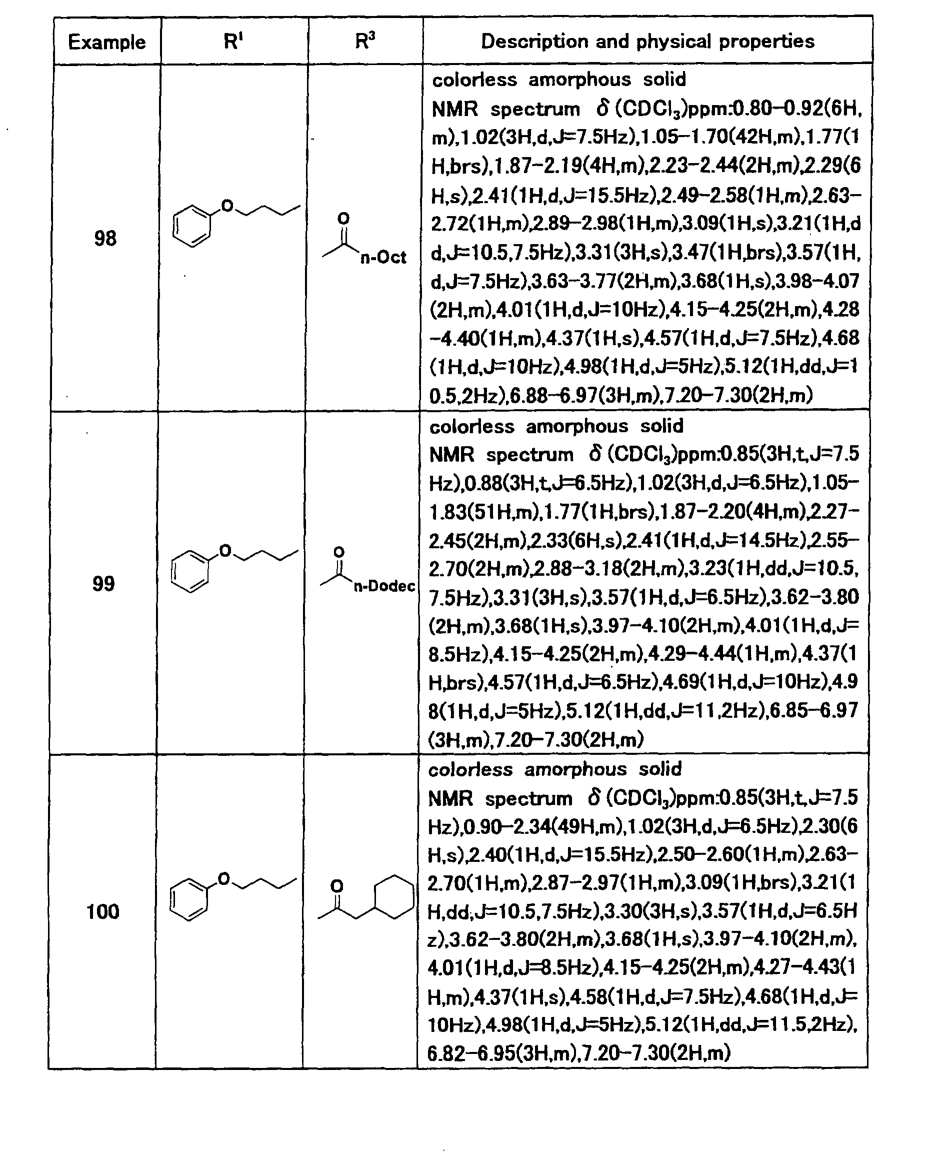

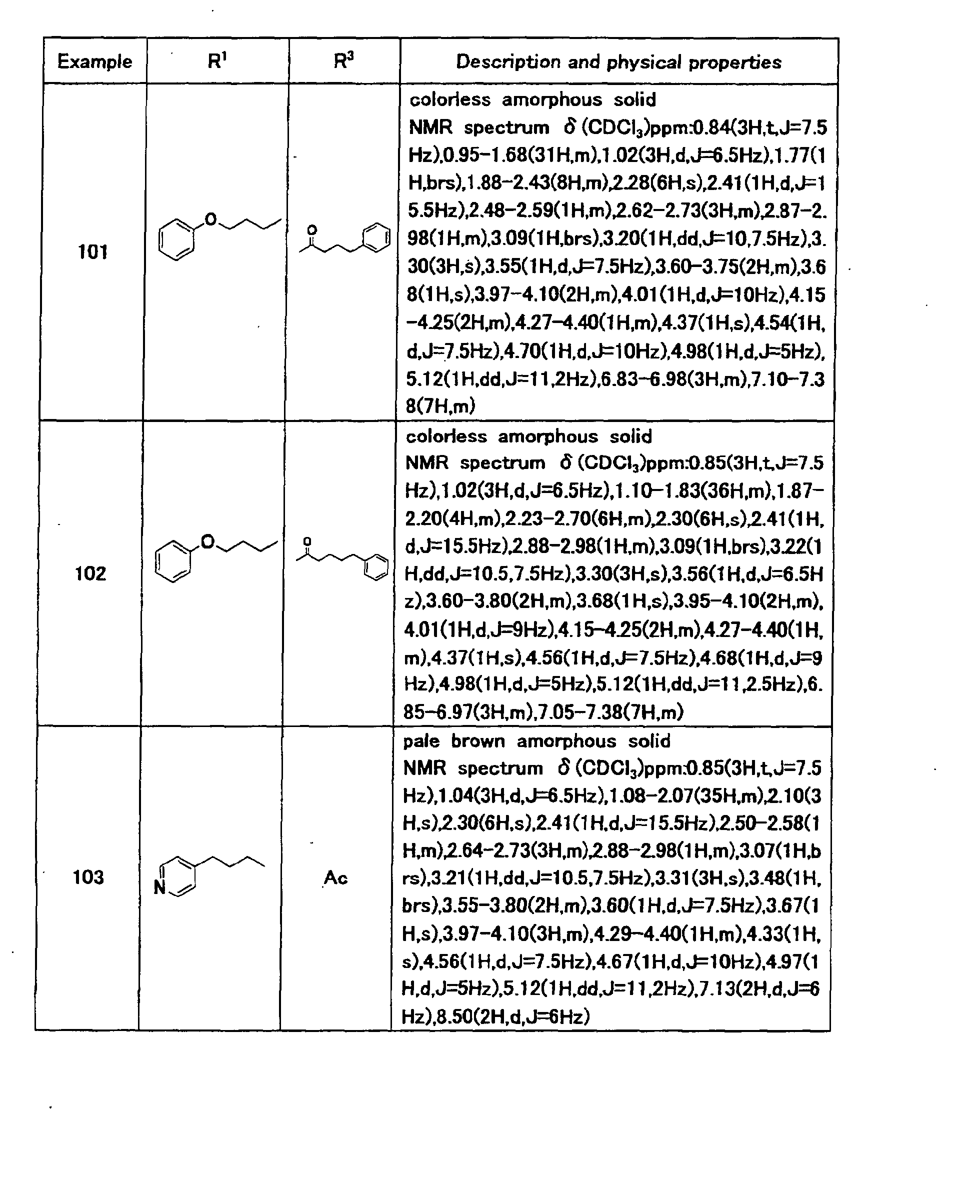

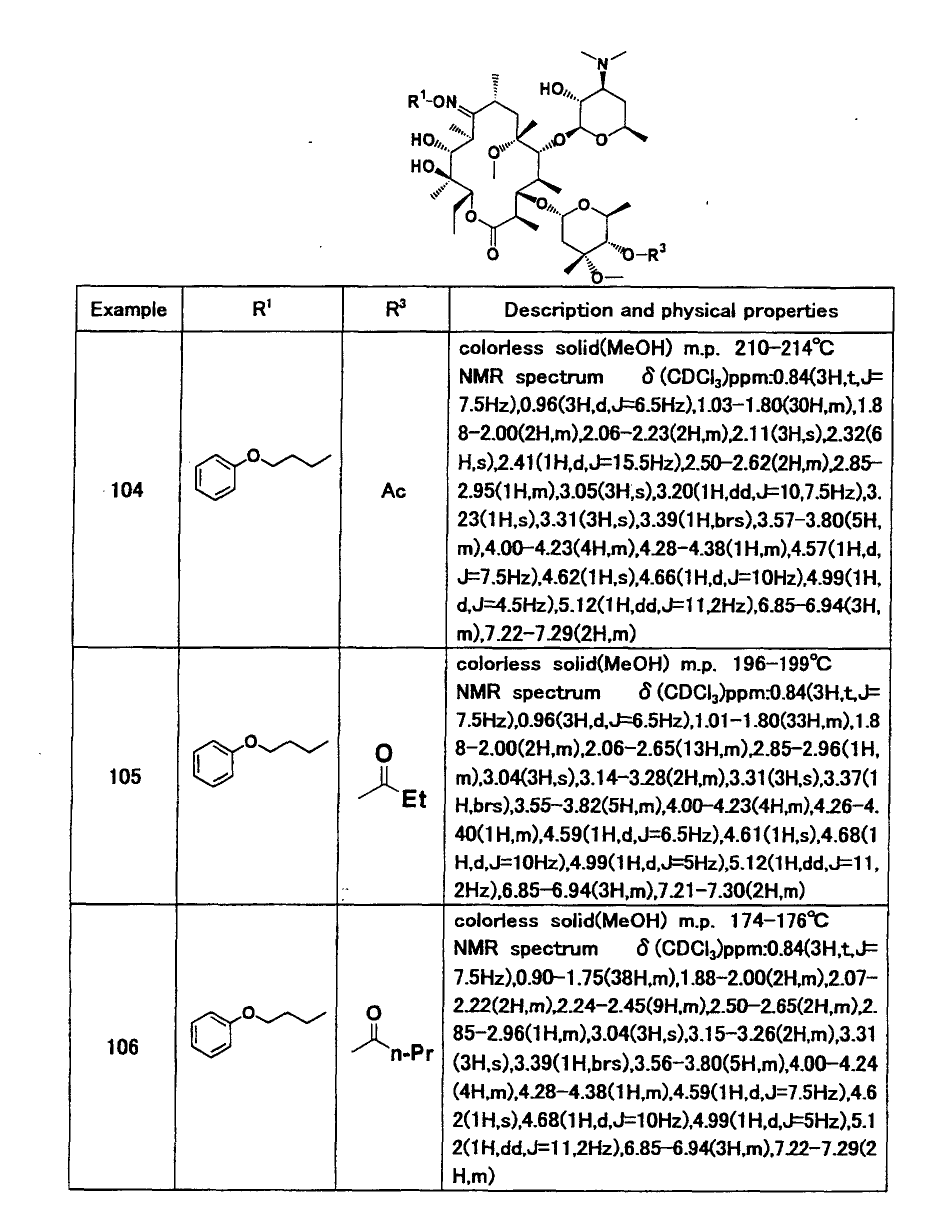

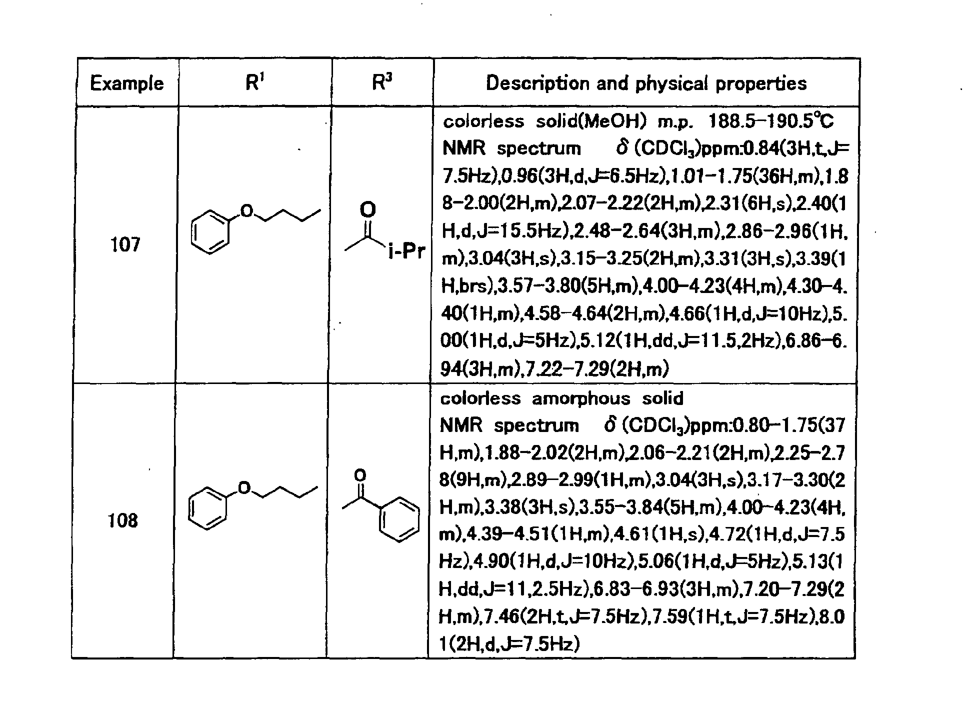

### Example 109: 4"-O-Acetylerythromycin A 9-[O-(n-octyl)oxime]

A 0.50 g portion of the colorless amorphous solid obtained in Example 66 was crystallized from methanol to give 0.35 g of colorless crystals having a melting point of 157.5-160°C. The NMR spectrum of the obtained compound was identical to that of the compound obtained in Example 66.

### Example 110: 4"-O-AcetylerythromycinA 9-[O-(3-cyclohexylpropyl)oxime]

A 0.90 g portion of the colorless amorphous solid obtained in Example 71 was crystallized from n-heptane to give 0.47 g of colorless crystals having a melting point of 130-132.5°C. The NMR spectrum of the obtained compound was identical to that of the compound obtained in Example 71.

In order to evaluate excellent efficacy of the compounds of the present invention, their antibacterial spectra against atypical acid-fast mycobacteria were measured. Clarithromycin, rifampicin, and 4"-O-acetylerythromycin A 9-(O-methyloxime)[a compound disclosed in the Japanese Patent Unexamined Publication (KOKAI) No. 63-107921/1988] were used as reference compounds. Ac in the formula represents acetyl group.

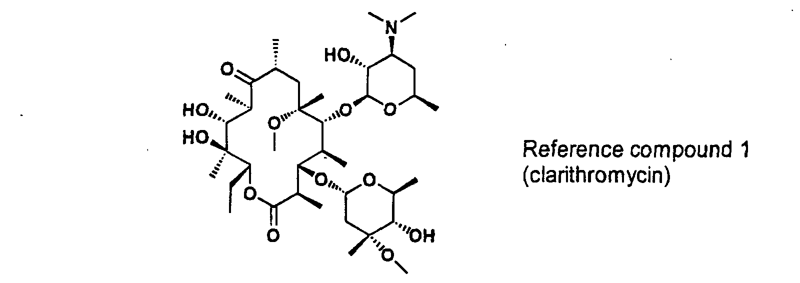

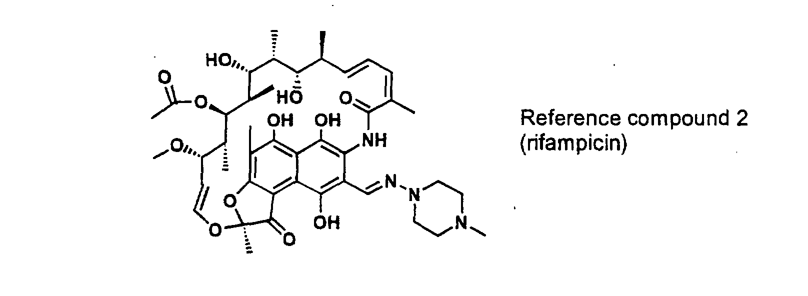

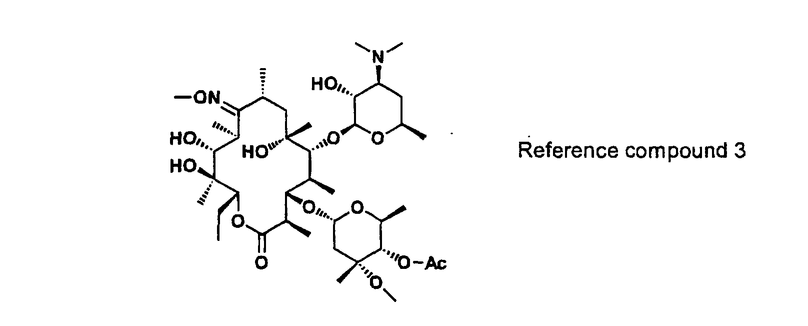

### Antibacterial activity against atypical acid-fast mycobacteria

Antibacterial activities (minimum inhibitory concentrations) against clinical isolates of atypical acid-fast mycobacteria were measured by the agar dilution method according to the standard method of the Japan Society of Chemotherapy. About 5 µl of bacterial suspension (adjusted to 10⁶ CFU/ml) were spotted on the 7H11 agar plates containing the test compounds. The minimum inhibitory concentrations were determined by the growth or no growth of the bacteria after incubation at 37°C for 7 days. The results are shown in the following table. The compounds of the present invention had more excellent antibacterial activity than the reference compounds against atypical acid-fast mycobacteria including clarithromycin-resistant atypical acid-fast mycobacteria (M. avium 20092 and other bacteria).
Names of the bacteria in the table are as follows:
*Mycobacterium avium (M. avium)*
*Mycobacterium intracellulare (M*. *intracellulare)*

| Antibacterial activity (Minimum inhibitory concentration µg/ml) | | | | | | | |
|---|---|---|---|---|---|---|---|
| Strain | Example | | | | Reference compound | | |
| | 1 | 54 | 66 | 71 | 1 | 2 | 3 |
| M.avium 20034 | 3.13 | 3.13 | 1.56 | 1.56 | 3.13 | 12.5 | >50 |
| M.avium 20045 | 1.56 | 1.56 | 1.56 | 3.13 | 1.56 | 3.13 | 12.5 |
| M.avium 20092 | 0.78 | 0.78 | 1.56 | 3.13 | >50 | 50 | >50 |
| M.avium 20096 | 0.78 | 0.78 | 1.56 | 1.56 | >50 | 3.13 | >50 |
| M.intracellulare 20066 | 0.78 | 0.78 | 1.56 | 1.56 | 3.13 | 3.13 | 12.5 |
| M.intracellulare 20067 | 0.39 | 0.78 | 1.56 | 1.56 | 1.56 | 1.56 | 6.25 |
| M.intracellulare 20073 | 0.78 | 0.78 | 1.56 | 1.56 | 1.56 | 3.13 | 12.5 |
| M.intracellulare 20075 | 0.78 | 0.78 | 1.56 | 1.56 | 3.13 | 3.13 | 12.5 |

### Industrial Applicability

The novel erythromycin derivatives and salts thereof according to the present invention have excellent antibacterial activity against atypical acid-fast mycobacteria including multiple drug-resistant bacteria and are useful as antibacterial agents.

## Claims

1. An erythromycin derivative represented by the following general formula or a salt thereof: 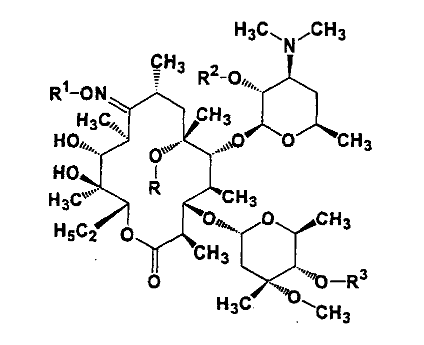 wherein R represents a hydrogen atom or a lower alkyl group; R¹ represents a C₅₋₁₂ alkyl group which may be substituted, a cycloalkyl group which may be substituted, a (cycloalkyl)alkyl group which may be substituted, an aralkyl group which may be substituted, or a group represented by the formula -(CH₂)ₙ-X-R⁴; R² represents a hydrogen atom or an acetyl group; R³ represents an acyl group which may be substituted, or a group represented by the formula -C(=O)-Y-R⁵; R⁴ represents an alkyl group which may be substituted, an alkoxyalkyl group which may be substituted, an alkylthioalkyl group which may be substituted, an alkylaminoalkyl group which may be substituted, an aryl group which may be substituted, or an aralkyl group which may be substituted; R⁵ represents an alkyl group which may be substituted, an aryl group which may be substituted, or an aralkyl group which may be substituted; n represents an integer of from 1 to 6; X represents an oxygen atom, a sulfur atom, or a group represented by -NZ-; Y represents an oxygen atom or a group represented by -NH-; and Z represents a hydrogen atom or an alkyl group which may be substituted.

2. An erythromycin derivative represented by the following general formula or a salt thereof: 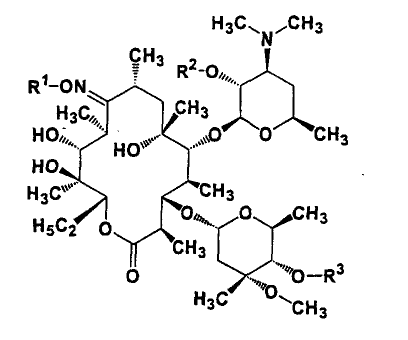 wherein R¹ represents a C₅₋₁₂ alkyl group which may be substituted, a cycloalkyl group which may be substituted, a (cycloalkyl)alkyl group which may be substituted, an aralkyl group which may be substituted, or a group represented by the formula -(CH₂)ₙ-X-R⁴; R² represents a hydrogen atom or an acetyl group; R³ represents an acyl group which may be substituted, or a group represented by the formula -C(=O)-Y-R⁵; R⁴ represents an alkyl group which may be substituted, an alkoxyalkyl group which may be substituted, an alkylthioalkyl group which may be substituted, an alkylaminoalkyl group which may be substituted, an aryl group which may be substituted, or an aralkyl group which may be substituted; R⁵ represents an alkyl group which may be substituted, an aryl group which may be substituted, or an aralkyl group which may be substituted; n represents an integer of from 1 to 6; X represents an oxygen atom, a sulfur atom, or a group represented by -NZ-; Y represents an oxygen atom or a group represented by -NH-; and Z represents a hydrogen atom or an alkyl group which may be substituted.

3. The compound or the salt thereof according to any one of claim 1 or 2, wherein R² is a hydrogen atom.

4. A medicament comprising the compound or the physiologically acceptable salt thereof according to any one of claims 1 to 3 as an active ingredient.

5. An antibacterial agent comprising the compound or the physiologically acceptable salt thereof according to any one of claims 1 to 3 as an active ingredient.

6. A medicament for the treatment of an atypical acid-fast microbacteriosis which comprises the compound or the physiologically acceptable salt thereof according to any one of claims 1 to 3 as an active ingredient.

7. Use of the compound or the physiologically acceptable salt thereof according to any one of claims 1 to 3 for the manufacture of the medicament according to claim 4.

8. A method for treatment of an infectious disease which comprises the step of administering a therapeutically effective amount of the compound or the physiologically acceptable salt thereof according to any one of claims 1 to 3 to a mammal including a human.
